# EUROPEAN PATENT APPLICATION

(11) **EP 3 616 793 A1**
(43) Date of publication of application: **04.03.2020**
(21) Application number: 19193764.8
(22) Date of filing: 27.08.2019
(51) Int. Cl.: B01L 3/00, B01L 7/00, B01F 3/08, B01F 13/00, B01J 19/00, C12Q 1/6844, G01N 35/08, G01N 33/50

(54) **DEVICE AND METHOD FOR DETECTING NUCLEIC ACID**

(30) Priority: 31.08.2018 JP 2018163063
(71) Applicant: SYSMEX CORPORATION, Kobe-shi Hyogo 651-0073 (JP)
(72) Inventor: Miura, Yoshinobu, Hyogo, 651-0073 (JP); Kitagawa, Nobuhiro, Hyogo, 651-0073 (JP); Maekawa, Takanori, Hyogo, 651-0073 (JP)
(74) Representative: Hoffmann Eitle

(57) **Abstract**

Disclosed is a nucleic acid detection device which includes: an installation unit on which a cartridge is installed, the cartridge including a chamber configured to store nucleic acid, a reagent for amplifying the nucleic acid and a dispersion medium for dispersing the nucleic acid and the reagent; a sample processing unit configured to produce, within the chamber, an emulsion in which droplets containing the nucleic acid and the reagent are dispersed in the dispersion medium; a temperature regulating unit configured to regulate a temperature of the cartridge to amplify the nucleic acid contained in the droplet; and a detection unit configured to detect a signal based on the nucleic acid amplified by temperature regulation by the temperature regulating unit.

## Description

### TECHNICAL FIELD

The present invention relates to a nucleic acid detection device which produces an emulsion containing nucleic acid by using a cartridge including a storage space for storing liquid, and which detects the nucleic acid (see, for example, US 9,126,160).

### BACKGROUND

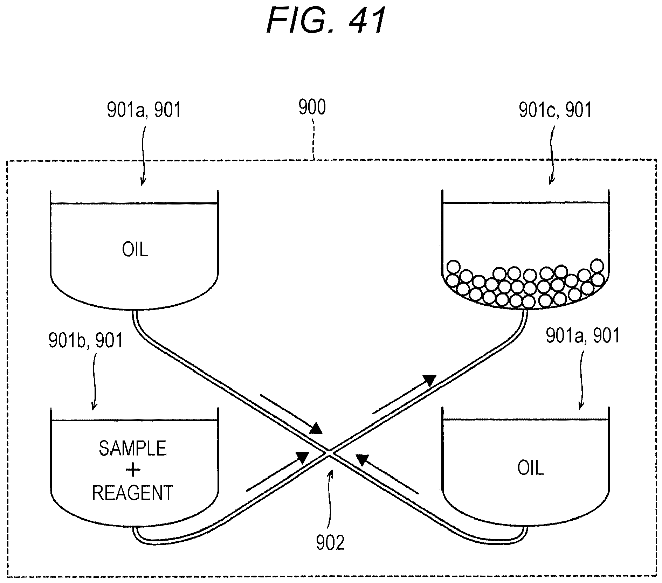

US 9,126,160 discloses a technique for forming an emulsion containing nucleic acid by feeding liquid to a fluid channel 902 in a cartridge 900 in which a plurality of wells 901 for retaining liquid are formed as shown in Fig. 41. In Fig. 41, the cartridge 900 includes four wells 901 of two oil wells 901a, one sample well 901b, and one collection well 901c. Each well 901 is connected via a fluid channel 902 formed in the cartridge 900. Oil is fed from the two oil wells 901a, and a mixed solution of a sample containing nucleic acid and a reagent is fed from the sample well 901b. The oil or mixed solution stored in the well 901 is injected from an opening at the top of the well 901, and the liquid in the well 901 is fed to the fluid channel 902 by applying air pressure from the opening. When the mixed solution and the oil merge at the fluid channel 902, droplets of the mixed solution are sequentially formed one by one in the oil at a merging portion. As a result of the sequentially formed droplets being stored in the collection well 901c through the fluid channel 902, an emulsion in which a large number of droplets are dispersed in oil is stored in the collection well 901c.

US 9,126,160 discloses that the produced emulsion is transferred to a device outside the cartridge to perform nucleic acid amplification in the droplet and to detect the droplet. US 9,126,160 discloses that heat circulation is applied to the cartridge 900 to induce nucleic acid amplification in the cartridge 900, and detection is performed by imaging droplets in the wells of the cartridge 900.

In US 9,126,160, when the emulsion formed by the cartridge is transferred to a device outside the cartridge to perform processing, the sample may be mixed up or may be contaminated with another sample. Therefore, in order to suppress sample mix-up and contamination, it is desirable to perform as many steps as possible in a nucleic acid detection assay inside the cartridge.

However, in that case, since a dedicated device for performing processing outside the cartridge cannot be used, the structure of the cartridge and the processing method tend to be complicated. For example, in US 9,126,160 shown in Fig. 41, since droplets are formed one by one in the oil at the merging portion of the fluid channel in the cartridge, it is necessary to form a fluid channel of an appropriate structure according to viscosity of each liquid and affinity between the liquids, and precisely control liquid feed in the fluid channel. Therefore, in order to suppress sample mix-up and contamination, it is desirable that more processing steps can be performed inside the cartridge and that the processing can be performed more easily.

### SUMMARY OF THE INVENTION

The scope of the present invention is defined solely by the appended claims, and is not affected to any degree by the statements within this summary.

A nucleic acid detection device according to a first aspect of this invention includes an installation unit (210) for installing a cartridge (100) that includes a chamber (11) for producing an emulsion (83) in which a dispersoid containing nucleic acid and a reagent (81) for amplifying the nucleic acid is dispersed in a dispersion medium (82) and that detects a signal based on the nucleic acid amplified in the emulsion (83); a sample processing unit (220) for producing the emulsion (83) in the chamber (11), by forming a droplet (84) of the dispersoid in the dispersion medium (82) in the chamber (11); a temperature regulating unit (260) for regulating a temperature of the cartridge (100) to amplify the nucleic acid contained in the droplet (84); and a detection unit (270) for detecting the signal based on the nucleic acid amplified by temperature regulation by the temperature regulating unit (260).

An emulsion is a dispersion system solution in which a dispersoid is dispersed in a dispersion medium. The dispersion system refers to a state in which the dispersoid is floated or suspended in the dispersion medium. The dispersion medium and the dispersoid are both liquids. However, the dispersion medium and the dispersoid may be a solution containing other than liquid. The dispersoid is not mixed with the dispersion medium. That is, the dispersion medium and the dispersoid do not form a homogeneous phase by mixing. The dispersoids are separated from each other by the dispersion medium and surrounded by the dispersion medium. Therefore, in the emulsion, droplets of the dispersoid are formed in the dispersion medium. Forming an emulsion is called "emulsification". Dissolving an emulsion so that the dispersoid and the dispersion medium are individually separated is called "demulsification."

In the nucleic acid detection device according to the first aspect, since the sample processing unit (220) produces the emulsion (83) in the chamber (11) of the cartridge (100), and the nucleic acid in the droplet (84) contained in the emulsion (83) is amplified by the temperature regulation of the cartridge (100) by the temperature regulating unit (260), it is possible to perform at least the step of producing an emulsion and the nucleic acid amplification step of the nucleic acid detection assay inside the cartridge (100). As a result, it is possible to suppress sample mix-up and contamination, as compared with the case of transferring the emulsion to a device outside the cartridge (100) for performing these steps. Then, since the sample processing unit (220) produces an emulsion (83) in the chamber (11) by forming the droplet (84) of the dispersoid in the dispersion medium (82) in the chamber (11), unlike the case where droplets are formed one by one at a merging portion of the fluid channel, there is no need for precise liquid feed control required for droplet formation in the channel, and the dispersoid and the dispersion medium (82) can be stored in the chamber (11) to form droplets (84), there is also no need to feed the droplets formed at the merging portion to the chamber. As a result, the emulsion (83) can be produced more easily. Also, since nucleic acid are amplified by adjusting the temperature of the cartridge (100), there is no need to transfer the emulsion (83) as compared with the case of transferring the emulsion (83) to another device for nucleic acid amplification. Therefore, it is possible to suppress sample mix-up and contamination. As a result of these, it is possible to suppress sample mix-up and contamination and also perform processing more easily.

In the nucleic acid detection device according to the first aspect, preferably, the sample processing unit (220) forms the droplet (84) in the chamber (11) by stirring the dispersion medium (82) and the dispersoid in the chamber. With this configuration, the dispersoid and the dispersion medium (82) move relative to each other in the chamber (11) by stirring, and the dispersoid is refined in the dispersion medium (82) to be fine droplets (84). Thus, it is possible to collectively form droplets (84) of the number required for detecting the nucleic acid in the chamber (11). In the present specification, the phrase "collectively form droplets" means not sequentially forming droplets one by one but collectively forming a group of a plurality of droplets.

In the nucleic acid detection device according to the first aspect, preferably, the sample processing unit (220) includes a rotation mechanism (221) that rotates the cartridge (100) installed in the installation unit, and the rotation mechanism (221) rotationally moves the cartridge (100) to a detection position for the detection unit (270) to detect the signal based on the nucleic acid. With this configuration, detection by the detection unit (270) can be easily performed simply by rotationally moving the cartridge (100) to the detection position by the rotation mechanism (221) while the produced emulsion (83) is stored inside the cartridge (100).

In this case, preferably, the rotation mechanism (221) moves the chamber (11) to the detection position, and the detection unit (270) detects the signal based on the nucleic acid in the chamber (11) moved to the detection position. With this configuration, the detection step of the nucleic acid can be performed inside the cartridge (100). Therefore, it is possible to suppress sample mix-up and contamination, as compared with the case of transferring the emulsion to a device outside the cartridge (100) for performing detection.

In the nucleic acid detection device according to the first aspect, preferably, the sample processing unit (220) includes the rotation mechanism (221) that rotates the cartridge (100) installed in the installation unit (210), and the rotation mechanism (221) transfers each of a solution containing the nucleic acid to be the droplet (84) and the reagent (81) for amplifying the nucleic acid to the chamber (11) in the cartridge (100) by rotating the cartridge (100). With this configuration, the solution containing the nucleic acid and the reagent (81) for amplifying the nucleic acid can be transferred using centrifugal force accompanying rotation of the cartridge (100) using the rotation mechanism (221) without receiving pressure supply from outside by a pump or the like. Therefore, for example, as compared with a configuration in which a well opened at the top is formed in the cartridge (100) and a pressure is applied to the opening of the well to transfer liquid, a part opened to outside for connecting with a pressure source can be suppressed from being formed in the cartridge (100), so that contamination can be effectively suppressed.

In this case, preferably, the rotation mechanism (221) rotates the cartridge (100) to mix the dispersion medium (82) and the dispersoid in the chamber (11), thereby forming a plurality of droplets (84) in the chamber (11). With this configuration, the emulsion (83) containing the plurality of droplets (84) can be formed in the chamber (11) simply by rotating the cartridge (100) using the rotation mechanism (221). Thus, the emulsion (83) can be efficiently produced in the chamber (11), as compared with the case where droplets are formed one by one at the merging portion of the fluid channel. Further, since liquid transfer and production of the emulsion (83) can be performed by the rotation mechanism (221), it is possible to simplify device configuration, as compared with a case where a mechanism for transferring liquid and a mechanism for producing the emulsion (83) are separately provided.

In the nucleic acid detection device according to the first aspect, preferably, the sample processing unit (220) forms the emulsion (83) containing the droplet (84) of the dispersoid containing one molecule or one nucleic acid in the chamber (11). With this configuration, it is possible to compartmentalize each one of the nucleic acid contained in the sample into the droplet (84) contained in the emulsion (83) to ensure processing. As a result, even nucleic acid which is contained in an extremely small amount in a sample can be amplified with high accuracy, so that it is possible to improve detection accuracy even when detecting the amplified nucleic acid.

In the nucleic acid detection device according to the first aspect, preferably, the sample processing unit (220) extracts the nucleic acid from a sample (80) in a cartridge (700), and the sample processing unit (220) forms the emulsion (83) by the dispersoid containing the nucleic acid extracted. With this configuration, a step of extracting the nucleic acid can be performed in the cartridge (700) as well as the step of producing an emulsion (83) and the nucleic acid amplification step. Thus, it is possible to effectively suppress sample mix-up and contamination, as compared with the case where the step of extracting the nucleic acid is performed outside the cartridge (700).

In the nucleic acid detection device according to the first aspect, preferably, the temperature regulating unit (260) regulates a temperature of the chamber (11) in which the emulsion (83) is produced. With this configuration, after the emulsion (83) is produced in the chamber (11) by the sample processing unit (220), a nucleic acid amplification step by temperature regulation can be performed in the chamber (11) without transferring the emulsion (83) to another place in the cartridge (100). Thus, it is possible to simplify the structure of the cartridge (100) and simplify processing while suppressing contamination.

In the nucleic acid detection device according to the first aspect, preferably, the temperature regulating unit (260) regulates the temperature of the chamber (11) by heating the cartridge (100) from outside of the cartridge (100). With this configuration, for example, unlike the case where the emulsion (83) is transferred from the cartridge (100) to a mechanism for performing temperature regulation, or the case where temperature regulation is performed by directly contacting the emulsion (83), it is possible to amplify the nucleic acid by performing temperature regulation from outside of the cartridge (100) while storing the emulsion (83) in the cartridge (100). As a result, it is possible to further effectively suppress sample mix-up and contamination.

In the nucleic acid detection device according to the first aspect, preferably, a reagent is enclosed in the cartridge (100) in advance, and the nucleic acid detection device further includes an opening mechanism (250) that opens seal of the reagent in the cartridge (100). With this configuration, it is possible to enclose a reagent necessary for nucleic acid detection in advance in the cartridge (100), and use the reagent by opening it by the opening mechanism (250). Therefore, for example, it is possible to effectively suppress contamination, as compared with a configuration in which a reagent to be used is externally injected into the cartridge (100) from outside.

In the nucleic acid detection device according to the first aspect, preferably, the detection unit (270) includes an imaging unit for acquiring an image in the chamber (16) storing the nucleic acid from outside of the cartridge (100). With this configuration, it is possible to perform imaging from outside of the cartridge (100) to detect the nucleic acid, while storing the nucleic acid in the cartridge (100). Therefore, since there is no need to transfer a liquid containing nucleic acid to outside of the cartridge (100) for detection, it is possible to further effectively suppress sample mix-up and contamination.

In this case, preferably, the nucleic acid detection device further includes an analysis unit (233) for outputting whether or not the nucleic acid to be detected is present, based on captured image information of the emulsion (83) in the chamber (16). With this configuration, unlike the case where the presence or absence of the nucleic acid to be detected is analyzed by an external analyzer, for example, the nucleic acid detection device alone can analyze the presence or absence of the nucleic acid to be detected and can output it to a user.

In the configuration including the rotation mechanism, preferably, the rotation mechanism (221) transfers the reagent (81) for amplifying the nucleic acid stored in a first liquid storage unit (38) included in the cartridge (100) to the chamber (11) by rotating the cartridge (100), and the rotation mechanism (221) mixes the nucleic acid and the reagent (81) for amplifying the nucleic acid in the chamber (11) to form the dispersoid in the chamber (11) by rotating the cartridge (100). With this configuration, it is possible to prepare the dispersoid for forming the droplet (84) in the chamber (11) simply by rotating the cartridge (100).

In this case, preferably, the rotation mechanism (221) transfers the dispersion medium (82) stored in a second liquid storage unit (31) included in the cartridge (100) to the chamber (11) by rotating the cartridge (100), and the rotation mechanism (221) repeats an operation of changing rotational speed of the rotated cartridge (100) to mix the dispersoid and the dispersion medium (82) in the chamber (11), thereby forming a plurality of droplets (84) in the chamber (11). With this configuration, it is possible to change an inertial force acting on a liquid in the chamber (11) to efficiently mix the dispersoid and the dispersion medium (82) by repeating an operation of changing a rotational speed of the cartridge (100) rotated by the rotation mechanism (221). As a result, it is possible to collectively form a plurality of droplets (84) in the chamber (11) easily and efficiently by the rotation mechanism (221).

In the configuration in which the operation of changing a rotational speed of the rotated cartridge (100) is repeated, preferably, the rotation mechanism (221) produces the emulsion (83) by repeating an operation of rotationally driving the cartridge (100) in a predetermined direction and then stopping or driving the cartridge (100) in a reverse direction. With this configuration, when the cartridge (100) is stopped or the direction of rotation is reversed, rapid speed change is easily generated, and a large inertial force can be applied, so that the emulsion (83) can be efficiently produced.

In the configuration in which the operation of changing a rotational speed of the rotated cartridge (100) is repeated, preferably, the rotation mechanism (221) rotates the cartridge (100) to transfer a reagent (85) for demulsifying the emulsion (83) from a third liquid storage unit (32) included in the cartridge (100) to the chamber (11) in which the emulsion (83) is formed With this configuration, the nucleic acid amplified in the droplets (84) contained in the emulsion (83) can be taken out by the reagent (85) for demulsifying the emulsion. At this time, unlike a case of feeding liquid by air pressure or the like, the reagent (85) for demulsifying the emulsion can be fed to the chamber (11) simply by rotating the cartridge (100) by the rotation mechanism (221), so that the emulsion (83) can be easily demulsified.

In this case, preferably, the rotation mechanism (221) rotates the cartridge (100) to transfer a reagent containing a labeling substance (LS) that reacts with an amplification product of the nucleic acid from a fourth liquid storage unit (35) included in the cartridge (100) to a second chamber (14) included in the cartridge (100), the temperature regulating unit (260) regulates a temperature of the second chamber (14) to react the amplification product of the nucleic acid taken from the droplets (84) by demulsification with the labeling substance, and the detection unit (270) detects a signal based on the labeling substance (LS) bound to the nucleic acid taken from the droplets (84). With this configuration, an assay for detection of nucleic acid can be performed in the cartridge (100) by performing nucleic acid amplification in the droplet (84) contained in the emulsion (83), and then binding the labeling substance (LS) to the nucleic acid and the amplification product of the nucleic acid taken out from the droplet (84). As a result, it is possible to effectively suppress contamination in each step above.

In the configuration in which the operation of changing a rotational speed of the rotated cartridge (100) is repeated, preferably, the reagent (81) for amplifying the nucleic acid contains a labeling substance (LS) that reacts with an amplification product of the nucleic acid, the temperature regulating unit (260) react the amplification product of the nucleic acid in the droplets (84) with the labeling substance (LS) by regulating a temperature of the chamber (11), and the detection unit (270) detects a signal based on the labeling substance (LS) in the droplets (84) contained in the emulsion (83) in the chamber (11). With this configuration, an assay for amplification of the nucleic acid, binding of the nucleic acid and the labeling substance (LS), and detection of nucleic acid in the droplets (84) unit in the droplet (84) contained in the emulsion (83) can be performed in the cartridge (100), by containing the labeling substance (LS) as a dispersoid. As a result, it is possible to effectively suppress contamination in each step above.

In the configuration in which the reagent for amplifying the nucleic acid is transferred by rotating the cartridge, preferably, the rotation mechanism (221) transfers a reagent (86) for washing the nucleic acid stored in a fifth liquid storage unit (33) included in the cartridge (100) by rotating the cartridge (100), to bring the nucleic acid captured on a carrier into contact with the reagent (86) for washing the nucleic acid in the cartridge (100). With this configuration, unnecessary components contained in the sample (80) can be removed by washing by bringing the nucleic acid into contact with the reagent (86) for washing the nucleic acid. The unnecessary component means a component unnecessary for detection of the nucleic acid to be detected.

In this case, preferably, the nucleic acid detection device further includes a transfer mechanism (240) that applies a magnetic force to the cartridge (100), in which the carrier is a magnetic particle (PM) that binds to the nucleic acid, and the transfer mechanism (240) transfers the magnetic particle (PM) bound to the nucleic acid to a third chamber (12) included in the cartridge (100) by magnetic force, along with rotation of the cartridge (100) by the rotation mechanism (221), thereby bringing the nucleic acid captured on the magnetic particle (PM) into contact with the reagent (86) for washing the nucleic acid. With this configuration, the nucleic acid can be transferred to the third chamber (12) and washed simply by applying a magnetic force to the cartridge (100) from outside by the transfer mechanism (240) without feeding liquid by air pressure or the like, by binding the nucleic acid to the magnetic particle (PM). In addition, in the case of feeding liquid by air pressure or the like, the nucleic acid in the chamber (11) is transferred together with the liquid, but in the transfer by magnetic force, only the nucleic acid bound to the magnetic particle (PM) can be transferred to the third chamber (12) leaving the liquid behind. As a result, a transfer of the unnecessary components to the third chamber (12) can be suppressed, and the washing of the nucleic acid in the third chamber (12) can be efficiently performed.

In the configuration in which the nucleic acid captured on a carrier is brought into contact with the reagent for washing the nucleic acid in the cartridge by rotating the cartridge, preferably, the carrier is a filter unit (821) that captures the nucleic acid, the rotation mechanism (221) transfers a solution containing the nucleic acid to the filter unit (821) included in a cartridge (800) by rotating the cartridge (800), and the rotation mechanism (221) rotates the cartridge (800) to transfer the reagent (86) for washing the nucleic acid from the fifth liquid storage unit (33) to the filter unit (821), thereby bringing the nucleic acid into contact with the reagent (86) for washing the nucleic acid. With this configuration, with a simple configuration in which the cartridge (800) is only rotated by the rotation mechanism (221) without adding a configuration such as applying a magnetic force to the cartridge (800) from outside, the nucleic acid can be washed by bringing the nucleic acid captured in the filter unit (821) into contact with the reagent (86) for washing the nucleic acid.

In the configuration in which the nucleic acid captured on the carrier is brought into contact with the reagent for washing the nucleic acid in the cartridge by rotating the cartridge, preferably, the rotation mechanism (221) rotates the cartridge (700) to transfer a reagent for eluting the nucleic acid stored in a sixth liquid storage unit (731 a) included in the cartridge (700), and the rotation mechanism (221) brings the reagent into contact with the nucleic acid captured on the carrier, thereby storing the nucleic acid desorbed from the carrier in a chamber (711), and separating the carrier from the chamber (711). With this configuration, after the nucleic acid is washed, the nucleic acid can be desorbed from the carrier by the reagent for eluting the nucleic acid to separate the carrier. As a result, unnecessary components and the like nonspecifically adsorbed on the carrier can be separated from the nucleic acid, so that nucleic acid detection with higher accuracy becomes possible.

In the configuration in which the reagent for amplifying the nucleic acid is transferred by rotating the cartridge, preferably, the rotation mechanism (221) rotates the cartridge (700) to mix in the cartridge (700) a sample (80) introduced into a sample introduction unit (20) included in the cartridge (700), and a reagent for extracting the nucleic acid stored in a seventh liquid storage unit (735) included in the cartridge (700), thereby performing processing for extracting the nucleic acid from the sample (80). With this configuration, a step of extracting the nucleic acid can be performed in the cartridge (700) as well as the step of producing an emulsion (83) and the nucleic acid amplification step. Thus, it is possible to effectively suppress sample mix-up and contamination, as compared with the case where the step of extracting the nucleic acid is performed outside the cartridge (700). Then, the step of extracting nucleic acid can be performed by rotation of the cartridge (700) using the rotation mechanism (221) in the same manner as the step of producing an emulsion (83), so that device configuration can be simplified.

In this case, preferably, the rotation mechanism (221) rotates the cartridge (700) to transfer the sample (80) containing the nucleic acid and the reagent for extracting the nucleic acid to a fourth chamber (715) included in the cartridge (700), and the rotation mechanism (221) rotates the cartridge (700) to mix the sample (80) containing the nucleic acid and the reagent for extracting the nucleic acid in the fourth chamber (715), thereby performing processing for extracting the nucleic acid. With this configuration, the step of extracting the nucleic acid can be performed in the fourth chamber (715) different from the chamber (711) for producing the emulsion (83). Therefore, it is possible to suppress adhesion of unnecessary components other than the nucleic acid generated in the step of extracting the nucleic acid to the inside of the chamber (711) for producing the emulsion (83).

In the configuration including the rotation mechanism, preferably, the rotation mechanism (221) rotates the cartridge (100) to move the chamber (11) storing the emulsion (83) to an arrangement position of the temperature regulating unit (260), and the temperature regulating unit (260) amplifies the nucleic acid contained in the dispersoid in the chamber (11), by periodically changing the temperature of the cartridge (100) to a plurality of temperature ranges. With this configuration, the step of amplifying the nucleic acid can be performed simply by rotating the cartridge (100) and positioning the chamber (11) at the arrangement position of the temperature regulating unit (260).

In the configuration including the rotation mechanism, preferably, the temperature regulating unit (260) includes a plurality of temperature zones (TZ) having different temperatures, the temperature zones being plurally arranged in a rotational direction on the cartridge (100) arranged in the installation unit (210), and the rotation mechanism (221) amplifies the nucleic acid contained in the dispersoid in the chamber (11), by rotating the cartridge (100) to sequentially move the chamber (11) to arrangement positions of the plurality of temperature zones (TZ), and periodically changing the temperature of the cartridge (100) to a plurality of temperature ranges. With this configuration, the step of amplifying the nucleic acid can be performed simply by rotating the cartridge (100) and positioning the chamber (11) in each temperature zone (TZ) of the temperature regulating unit (260). In this case, a set temperature of each temperature zone (TZ) may be constant, and regulation for periodically changing the temperature of the temperature regulating unit (260) is unnecessary, so that temperature regulation can be facilitated.

A method for detecting nucleic acid according to a second aspect of this invention is a method for detecting nucleic acid using a cartridge (100) that includes a chamber (11) for producing an emulsion (83) in which a dispersoid containing nucleic acid and a reagent (81) for amplifying the nucleic acid is dispersed in a dispersion medium (82) and that detects a signal based on the nucleic acid amplified in the emulsion (83), the method including: storing the nucleic acid, the reagent (81) for amplifying the nucleic acid and the dispersion medium (82) in the chamber (11) of the cartridge (100); producing the emulsion (83) in the chamber (11) by forming a droplet (84) of the dispersoid in the dispersion medium (82) in the chamber (11); adjusting a temperature of the cartridge (100) for amplifying the nucleic acid contained in the droplet (84); and detecting the signal based on the nucleic acid amplified by temperature regulation.

In the method for detecting nucleic acid according to the second aspect, since the emulsion (83) is produced in the chamber (11), and the nucleic acid in droplet (84) contained in the emulsion (83) is amplified by adjusting the temperature of the cartridge (100), it is possible to perform at least the step of producing the emulsion (83) and the nucleic acid amplification step of the nucleic acid detection assay inside the cartridge (100). As a result, it is possible to suppress sample mix-up and contamination, as compared with the case of transferring the sample to a device outside the cartridge (100) for performing these steps. Then, since an emulsion (83) is produced in the chamber (11) by forming the droplet (84) of the dispersoid in the dispersion medium (82) in the chamber (11), unlike the case where droplets are formed one by one at a merging portion of the fluid channel, there is no need for precise liquid feed control required for droplet formation in the channel, and the dispersoid and the dispersion medium (82) can be stored in the chamber (11) to form droplets (84), there is also no need to feed the droplets formed at the merging portion to the chamber. As a result, the emulsion (83) can be produced more easily. Also, since nucleic acid are amplified by adjusting the temperature of the cartridge (100), there is no need to transfer the emulsion (83) as compared with the case of transferring the emulsion (83) to another device for nucleic acid amplification. Therefore, it is possible to suppress sample mix-up and contamination. As a result of these, it is possible to suppress sample mix-up and contamination and also perform processing more easily.

In the method for detecting nucleic acid according to the second aspect, preferably, the droplet (84) is formed in the chamber (11) by stirring the dispersion medium (82) and the dispersoid in the chamber (11). With this configuration, the dispersoid and the dispersion medium (82) move relative to each other in the chamber (11) by stirring, and the dispersoid is refined in the dispersion medium (82) to be fine droplets (84). Thus, it is possible to collectively form droplets (84) of the number required for detecting the nucleic acid in the chamber (11).

It is possible to suppress sample mix-up and contamination and also perform processing more easily.

### BRIEF DESCRIPTION OF THE DRAWINGS

Fig. 1 is a schematic view showing an outline of a nucleic acid detection device;
Fig. 2 is a schematic view showing formation of an emulsion in a chamber by a sample processing unit;
Fig. 3 is a schematic side view (A) and a schematic plan view (B) for illustrating a first example of emulsion formation by the sample processing unit;
Fig. 4 is a schematic view showing an example (A) of reversing a direction of rotation of a cartridge and an example (B) of rotating the cartridge in the same direction;
Fig. 5 is a schematic view for illustrating a second example of emulsion formation by the sample processing unit;
Fig. 6 is a schematic side view (A) and a schematic plan view (B) of a chamber for illustrating a third example of emulsion formation by the sample processing unit;
Fig. 7 is a schematic view for illustrating a fourth example of emulsion formation by the sample processing unit;
Fig. 8 is a flow chart for illustrating a method for detecting nucleic acid;
Fig. 9 is a schematic view for illustrating a program;
Fig. 10 is a view showing an example in which a plurality of chambers are provided in a cartridge;
Fig. 11 is a view showing an example in which a liquid storage unit is provided in the cartridge;
Fig. 12 is a view showing a specific configuration example of the cartridge;
Fig. 13 is a flow chart for illustrating an emulsion PCR assay;
Fig. 14 is a view for illustrating function of each part of the cartridge in the emulsion PCR assay;
Fig. 15 is a diagram for illustrating progress of the reaction in the emulsion PCR assay;
Fig. 16 is a schematic plan view showing a first example of a temperature regulating unit;
Fig. 17 is a schematic plan view showing a second example of the temperature regulating unit;
Fig. 18 is a perspective view showing a state in which a lid of a nucleic acid detection device is opened;
Fig. 19 is a perspective view showing a state in which the lid of the nucleic acid detection device is closed;
Fig. 20 is a schematic cross-sectional view showing an internal structure of the nucleic acid detection device;
Fig. 21 is a block diagram showing a control configuration of the nucleic acid detection device;
Fig. 22 is a schematic view showing an example of usage of the nucleic acid detection device;
Fig. 23 is a schematic diagram of a scattergram showing results of nucleic acid detection according to a comparative example;
Fig. 24 shows diagrams showing rotational speed change patterns (A) to (D) of the cartridge in Example 1;
Fig. 25 shows microscope images (A) to (D) of an emulsion in Example 1;
Fig. 26 shows schematic diagrams (A) to (D) of a scattergram obtained in Example 1;

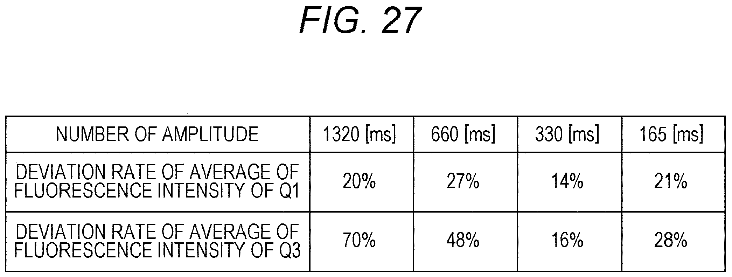

Fig. 27 is a table showing a result of comparison with the comparative example of results of nucleic acid detection according to Example 1;
Fig. 28 shows schematic diagrams (A) to (D) showing chambers at the time of emulsion formation in respective volume proportions in Example 2;
Fig. 29 is a table showing a result of comparison with the comparative example of results of nucleic acid detection according to Example 2;
Fig. 30 is a diagram showing a rotational speed change pattern of the cartridge in Example 3;
Fig. 31 shows schematic diagrams showing a chamber at the time of emulsion formation in Example 3;
Fig. 32 shows schematic diagrams of a scattergram obtained in Example 3;
Fig. 33 is shows diagrams showing rotational speed change patterns (A) to (D) of the cartridge in Example 4;
Fig. 34 shows schematic diagrams (A) to (D) of scattergrams obtained in Example 4;
Fig. 35 is a table showing a result of comparison with the comparative example of results of nucleic acid detection according to Example 4;
Fig. 36 is a view for illustrating a cartridge of a second embodiment;
Fig. 37 is a diagram showing a processing flow using the cartridge of the second embodiment;
Fig. 38 is a view for illustrating a cartridge of a third embodiment;
Fig. 39 is a diagram showing a processing flow using the cartridge of the third embodiment;
Fig. 40 is a view showing a modification example of the nucleic acid detection device; and
Fig. 41 is a view for illustrating the prior art.

### DETAILED DESCRIPTION OF THE PREFERRED EMBODIMENTS

Hereinafter, embodiments will be described with reference to the drawings.

### [First Embodiment]

### (Outline of Nucleic Acid Detection Device)

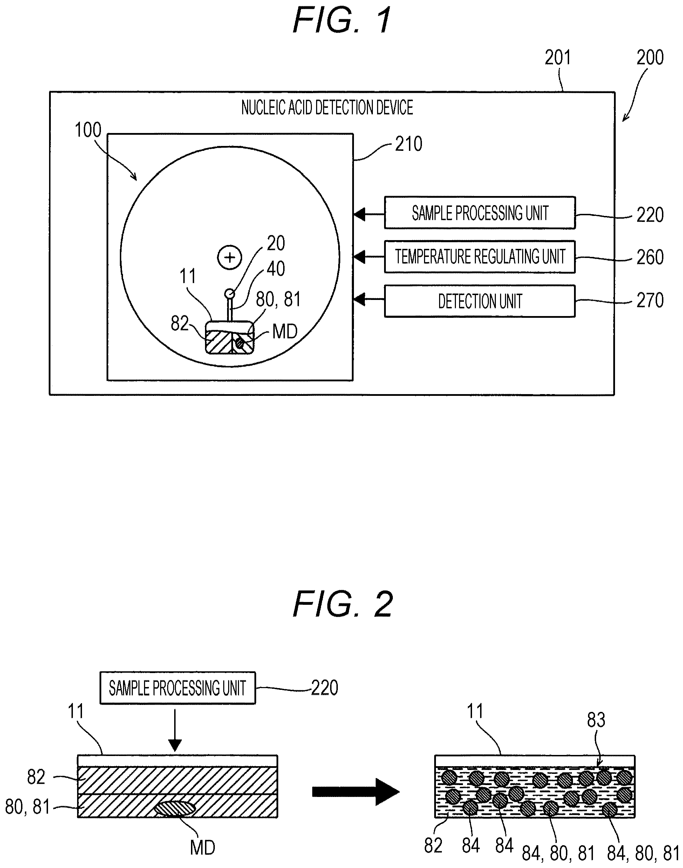

Next, an outline of a nucleic acid detection device 200 according to a first embodiment will be described with reference to Fig. 1.

The nucleic acid detection device 200 according to the first embodiment is a nucleic acid detection device that performs nucleic acid detection using a cartridge 100 including a chamber 11 for storing liquid. The nucleic acid detection device 200 performs a processing for detecting nucleic acid MD using the cartridge 100 into which the nucleic acid MD is injected.

Into the cartridge 100, a sample 80 containing the nucleic acid MD can be injected. The sample is, for example, a biological sample collected from human who is a subject. The sample is, for example, a liquid such as body fluid or blood (whole blood, serum or plasma) collected from a patient, a liquid obtained by subjecting the collected body fluid or blood to a predetermined preprocessing, or a cell or tissue piece collected from a patient. The sample contains, for example, nucleic acid MD such as DNA (deoxyribonucleic acids). The sample may be, for example, an extract obtained by extracting nucleic acid MD from blood or the like by a predetermined preprocessing.

In the cartridge 100, a reagent 81 for amplifying the nucleic acid in the sample 80 and a dispersion medium 82 are stored. The reagent 81 for amplifying the nucleic acid may contain a labeling substance such as a polymerase, a primer and a probe or an intercalator. When the reagent 81 for amplifying the nucleic acid is subjected to a thermal cycle processing of repeating one cycle of changing to a plurality of different temperatures in a state mixed with the nucleic acid MD, a polymerase chain reaction (PCR) occurs to amplify the nucleic acid MD. The sample 80, the reagent 81 for amplifying the nucleic acid, and the dispersion medium 82 are liquids. However, these liquids may contain other than liquids. The dispersion medium 82 is immiscible with the sample 80 and the reagent 81. That is, a mixed solution of the sample 80 and the reagent 81 and the dispersion medium 82 have a property of separating into separate phases. For example, the mixed solution is water-based, and the dispersion medium 82 is oil-based. The mixed solution of the sample 80 and the reagent 81 and the dispersion medium 82 form an emulsion 83 by being stirred in the chamber 11. That is, with the mixed solution containing the sample 80 and the reagent 81 as a dispersoid and an oil as the dispersion medium 82, the mixed solution of the sample 80 and the reagent 81 and the dispersion medium 82 form a dispersed solution in which the dispersoid as droplets 84 is dispersed in the dispersion medium 82.

The cartridge 100 is a replaceable consumable. That is, the cartridge 100 is discarded when it is used for measurement for a preset number of times. The usable number of times of the cartridge 100 is one or several times. The cartridge is a replaceable part that summarizes functions required to process the sample 80.

The cartridge 100 is, for example, a flat plate-shaped member in which a space is formed inside. The cartridge 100 includes a sample introduction unit 20 for introducing a sample 80 collected from a living body. The sample introduction unit 20 is, for example, an opening formed on the outer surface of the cartridge 100. The opening to be the sample introduction unit 20 may be closed in advance. In this case, the opening is opened by an operator when the cartridge 100 is used. The sample introduction unit 20 receives at least the sample 80. The sample introduction unit 20 can receive the mixed solution of the sample 80 and the reagent 81 for amplifying the nucleic acid.

The cartridge 100 includes a chamber capable of storing liquid therein. One or more chambers are provided in the cartridge 100. The chamber is a liquid storage space formed inside the cartridge 100. The chamber can store liquid therein. The chamber 11 may be a substantially closed space so that liquid does not leak inside the cartridge 100. The substantially closed space allows communication with outside of the cartridge 100 through the sample introduction unit 20. The chamber is configured such that, for example, when liquid is received from a passage connected to the chamber, the liquid can be stored substantially without flowing out the liquid in the passage. The chamber 11 can be configured such that the liquid in the chamber 11 does not flow back to the sample introduction unit 20 under normal use conditions.

In the first embodiment, the cartridge 100 is a cartridge for detecting a signal based on the nucleic acid MD amplified in the emulsion 83, including the chamber 11 for producing the emulsion 83 in which the dispersoid containing the nucleic acid MD and the reagent 81 for amplifying the nucleic acid is dispersed in the dispersion medium 82. The chamber 11 is configured as an internal space of a volume capable of storing the sample 80, the reagent 81 and the dispersion medium 82. The chamber 11 stores the sample 80, the reagent 81 and the dispersion medium 82. The cartridge 100 can include the sample introduction unit 20 for introducing a liquid into the chamber 11, a passage 40, a space that functions as a liquid storage unit, and the like. The cartridge 100 can be one in which an internal space such as the chamber 11 or the passage is formed by, for example, laminating a transparent film to a surface of a member in which a hole constituting the chamber 11, the passage 40 or the like is formed to close the opening portion.

The chamber 11 may store the reagent 81 and the dispersion medium 82 in advance, or may not store the reagent. The reagent 81 and the dispersion medium 82 can be injected into the chamber 11 not storing the reagent 81 and the dispersion medium 82 from another place in the cartridge 100 or from outside of the cartridge 100.

By the nucleic acid detection device 200, mixing of the dispersoid containing the sample 80 and the reagent 81 with the dispersion medium 82, stirring, warming or cooling, moving of the solid or liquid used for processing, and various other operations can be performed inside the cartridge 100.

As shown in Fig. 1, the nucleic acid detection device 200 includes an installation unit 210, a sample processing unit 220, a temperature regulating unit 260, and a detection unit 270. The installation unit 210, the sample processing unit 220, the temperature regulating unit 260, and the detection unit 270 are stored, for example, in a housing 201.

The housing 201 is configured by a box-like member having an internal space of a predetermined volume, a combination of a frame and an exterior plate, and the like. For example, the housing 201 of the nucleic acid detection device 200 has a small box-like shape that can be installed on a table.

In the installation unit 210, it is possible to install the cartridge 100 for detecting a signal based on the nucleic acid MD amplified in the emulsion 83, including the chamber 11 for producing the emulsion 83 in which the dispersoid containing the nucleic acid MD and the reagent 81 for amplifying the nucleic acid is dispersed in the dispersion medium 82. The installation unit 210 may hold the cartridge 100 in any posture. The installation unit 210 has, for example, an upper surface that constitutes an installation surface of the cartridge 100. The installation unit 210 supports a lower surface of the cartridge 100.

The sample processing unit 220 is configured to form an emulsion 83 in the cartridge 100 using the cartridge 100 installed in the installation unit 210. The sample processing unit 220 performs a predetermined operation on the cartridge 100 installed in the installation unit 210. The predetermined operation may include application of pressure to the cartridge 100, stirring of liquid in the chamber 11 by application of a centrifugal force, an inertial force, or the like.

In the first embodiment, the sample processing unit 220 is configured to produce the emulsion 83 in the chamber 11, by forming droplets 84 of the dispersoid in the dispersion medium 82 in the chamber 11, as shown in Fig. 2. That is, the sample processing unit 220 does not sequentially form droplets 84 one by one and store them in the chamber 11, but forms droplets 84 of the number necessary for detecting the nucleic acid MD in the chamber 11. The plurality of droplets 84 can be formed collectively in the chamber 11. The phrase "formed collectively" does not require all droplets 84 to be formed simultaneously, but it refers to, for example, form an emulsion 83 containing a desired number of droplets 84 in the chamber 11 by performing one unit operation on the cartridge 100, such as stirring the liquid in the chamber 11 for a predetermined time.

In the emulsion 83 produced in the chamber 11 by the sample processing unit 220, the nucleic acid MD and the reagent 81 for amplifying the nucleic acid are included in the droplet 84. That is, a dispersoid composed of a mixed solution containing at least the nucleic acid MD and the reagent 81 for amplifying the nucleic acid form an interface with the dispersion medium 82 to be a droplet 84. The specific configuration of the sample processing unit 220 will be described later.

Returning to Fig. 1, the temperature regulating unit 260 regulates the temperature of the cartridge 100 for amplifying the nucleic acid MD contained in the droplets 84. The temperature regulating unit 260 is configured at least by an apparatus that changes the temperature of the cartridge 100 by heating. The temperature regulating unit 260 can be configured by, for example, a heating apparatus such as a heater or an apparatus capable of heating and cooling such as a Peltier element. The temperature of the cartridge 100 is changed by heating the temperature regulating unit 260 in direct contact with the cartridge 100 or by heating the cartridge 100 in a noncontact manner.

The temperature regulating unit 260 performs a thermal cycle processing of repeating one cycle of changing the temperature of the cartridge 100 to a plurality of different temperatures a plurality of times. Thus, in the individual droplets 84 contained in the emulsion 83, the primer contained in the reagent 81 for amplifying the nucleic acid is bound to the nucleic acid MD and amplified.

The detection unit 270 detects the signal based on the nucleic acid MD amplified by temperature regulation by the temperature regulating unit 260. The detection unit 270 detects the nucleic acid MD, for example, via a labeling substance specifically bound to the nucleic acid MD to be detected.

The labeling substance is a substance that can be measured by the detection unit 270. As the label, for example, a chemiluminescent substance, a fluorescent substance, a radioactive isotope or the like is used. The labeling substance is, for example, a chemiluminescent substance, a fluorescent substance, a radioactive isotope or the like, and bound to a probe consisting of a nucleic acid complementary to the nucleic acid MD to be detected. The detection unit 270 optically detects an optical signal caused by, for example, a labeling substance stored in the cartridge 100. When performing detection of light emission, the detection unit 270 includes, for example, an imaging unit including an image sensor, or a photodetector such as a photomultiplier tube, a phototube or a photodiode. When performing fluorescence detection, the detection unit 270 includes a light source and an imaging unit or a photodetector. When performing detection of radiation, the detection unit 270 includes a radiation detector such as, for example, a scintillation counter.

The detection unit 270 detects, for example, the nucleic acid MD stored in the chamber 11. The detection unit 270 in this case includes, for example, an imaging unit including an image sensor. For example, the detection unit 270 receives a sample containing the nucleic acid MD extracted from the cartridge 100, and the detection unit 270 detects the nucleic acid MD in a state separated from the cartridge 100. By performing image processing on a captured image in the chamber 11 acquired by the imaging unit, the number of labeling substances that emit fluorescence is counted. The detection unit 270 in this case includes, for example, a flow cytometer. In the flow cytometer, the nucleic acid MD bound to the labeling substance flows through a flow cell, and the nucleic acid MD is irradiated with laser light. The fluorescence of the labeling substance emitted by the irradiated laser light is detected.

In the nucleic acid detection device 200 having such a configuration, since the sample processing unit 220 produces an emulsion 83 in the chamber 11 of the cartridge 100, and the nucleic acid MD in droplet 84 contained in the emulsion 83 is amplified by the temperature regulation of the cartridge 100 by the temperature regulating unit 260, it is possible to perform at least the step of producing an emulsion and the nucleic acid amplification step of the nucleic acid detection assay inside the cartridge 100. As a result, it is possible to suppress sample mix-up and contamination, as compared with the case of transferring the emulsion 83 to a device outside the cartridge 100 for performing these steps. Then, since the sample processing unit 220 produces an emulsion 83 in the chamber 11 by forming the droplets 84 of the dispersoid in the dispersion medium 82 in the chamber 11, unlike the case where droplets are formed one by one at a merging portion of the fluid channel, there is no need for precise liquid feed control required for droplet formation in the channel, and the dispersoid and the dispersion medium 82 can be stored in the chamber 11 to form droplets 84, there is also no need to feed the droplets formed at the merging portion to the chamber. As a result, the emulsion 83 can be produced more easily. Also, since the nucleic acid MD is amplified by adjusting the temperature of the cartridge 100, there is no need to transfer the emulsion 83 as compared with the case of transferring the emulsion 83 to another device for nucleic acid amplification. Therefore, it is possible to suppress sample mix-up and contamination. As a result of these, it is possible to suppress sample mix-up and contamination and also perform processing more easily.

The temperature regulating unit 260 can be configured to regulate the temperature of the chamber 11 in which the emulsion 83 is produced. In this case, the temperature regulating unit 260 performs a thermal cycle processing on the chamber 11 in which the emulsion 83 is produced by the sample processing unit 220, without transferring the emulsion 83 to another chamber in the cartridge 100 or outside of the cartridge 100. Thus, it is possible to simplify the structure of the cartridge 100 and simplify processing while suppressing contamination.

### (Configuration Example of Sample Processing Unit)

In the configuration example of Fig. 2, the sample processing unit 220 forms droplets 84 in the chamber 11 by stirring the dispersion medium 82 and the dispersoid in the chamber. With this configuration, the dispersoid and the dispersion medium 82 move relative to each other in the chamber 11 by the stirring, and the dispersoid is refined in the dispersion medium 82 to be fine droplets 84. Thus, it is possible to collectively form droplets 84 of the number required for detecting nucleic acid in the chamber 11.

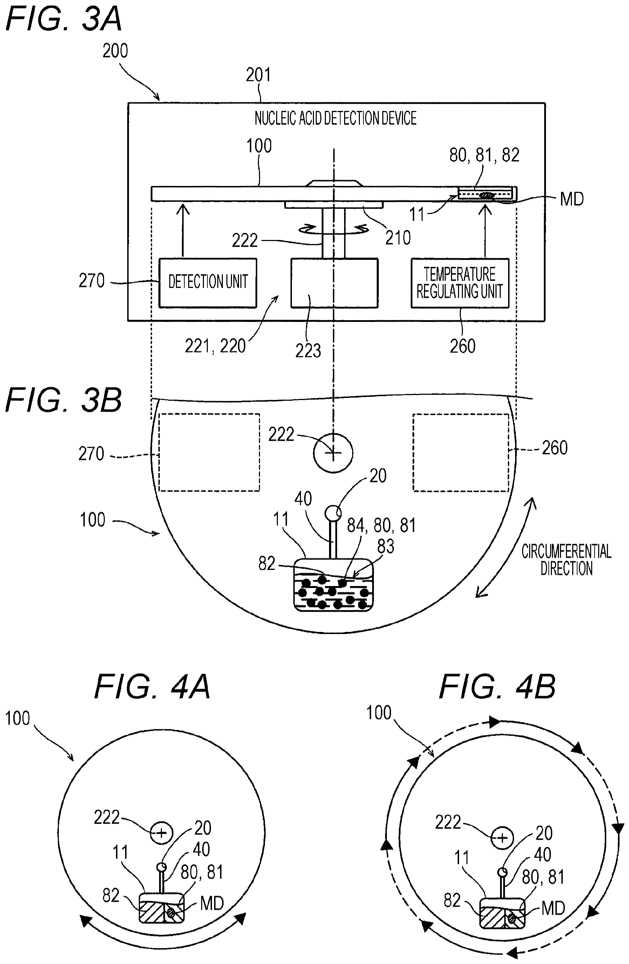

In the example of Fig. 3, the sample processing unit 220 includes a rotation mechanism 221 that rotates the cartridge 100 installed in the installation unit 210.

The rotation mechanism 221 includes a rotation axis 222 and a drive unit 223 that rotates the rotation axis 222. The rotation mechanism 221 can rotate the cartridge 100 installed in the installation unit 210 around the rotation axis 222. The rotation axis 222 extends, for example, in a vertical direction. In the example of Fig. 3A, the rotation mechanism 221 supports the installation unit 210 fixed to one end of the rotation axis 222, and the rotation mechanism 221 integrally rotates the rotation axis 222 and the installation unit 210 by the drive unit 223 connected to the other end of the rotation axis 222. The drive unit 223 is, for example, an electric motor. Thus, the rotation mechanism 221 rotates the cartridge 100 arranged on the installation unit 210 in a horizontal plane around the rotation axis 222.

The rotation mechanism 221 transfers the solution containing the nucleic acid MD to be droplet 84 and the reagent 81 for amplifying the nucleic acid to the chamber 11 respectively in the cartridge 100 by rotating the cartridge 100. The centrifugal force generated by the rotation of the cartridge 100 transfers the solution containing the nucleic acid MD and the reagent 81 for amplifying the nucleic acid in the cartridge 100. Thus, the solution containing the nucleic acid and the reagent 81 for amplifying the nucleic acid can be transferred using centrifugal force accompanying rotation of the cartridge 100 using the rotation mechanism 221 without receiving pressure supply from outside by a pump or the like. Therefore, for example, as compared with a configuration in which a well opened at the top is formed in the cartridge 100 and a pressure is applied to the opening of the well to transfer liquid, a part opened to outside for connecting with a pressure source can be suppressed from being formed in the cartridge 100, so that contamination can be effectively suppressed.

The rotation mechanism 221 moves the chamber 11 to a predetermined position where the temperature regulating unit 260 is arranged by rotating the cartridge 100. Thus, the chamber 11 can be moved to the arrangement position of the temperature regulating unit 260 using the rotation mechanism 221 that forms the emulsion 83 in the chamber 11.

The rotation mechanism 221 rotationally moves the cartridge 100 to a detection position for the detection unit 270 to detect the signal based on the nucleic acid MD. Thus, detection by the detection unit 270 can be easily performed simply by rotationally moving the cartridge 100 to the detection position by the rotation mechanism 221 while the produced emulsion 83 is stored inside the cartridge 100. The detection position is, for example, a position facing the detection unit 270. In the example of Fig. 3, the detection position is a position immediately above the detection unit 270. The rotation mechanism 221 moves the chamber 11 to the detection position, and the detection unit 270 detects the signal based on the nucleic acid MD in the chamber 11 moved to the detection position. Thus, the detection step of the nucleic acid can be performed inside the cartridge 100. Therefore, it is possible to suppress sample mix-up and contamination, as compared with the case of transferring the emulsion to a device outside the cartridge 100 for performing detection. The chamber 11 in which the emulsion 83 is produced and the chamber in which the signal based on the nucleic acid MD is detected may be different chambers. The detection unit 270 detects the signal based on the nucleic acid MD stored in the chamber 11 in which the emulsion 83 is produced, or in another chamber in which the emulsion 83 is transferred from the chamber 11 in the cartridge 100. In the case of Fig. 3, the temperature regulating unit 260 and the detection unit 270 can be fixedly provided in the nucleic acid detection device 200, and in that case, device configuration can be simplified.

The rotation mechanism 221 rotates the cartridge 100 to mix the dispersion medium 82 and the dispersoid in the chamber 11, thereby collectively forming a plurality of droplets 84 in the chamber 11. In this case, the emulsion 83 containing a plurality of droplets 84 can be formed in the chamber 11 by mixing simply by rotating the cartridge 100 using the rotation mechanism 221. Thus, the emulsion 83 can be efficiently produced in the chamber 11, as compared with the case where droplets are formed one by one at the merging portion of the fluid channel. Further, since liquid transfer and production of the emulsion 83 can be performed by the rotation mechanism 221, it is possible to simplify device configuration, as compared with a case where a mechanism for transferring liquid and a mechanism for producing the emulsion 83 are separately provided.

Specifically, the rotation mechanism 221 repeats an operation of changing rotational speed of the rotated cartridge 100 to mix the dispersoid and the dispersion medium 82 in the chamber 11, thereby forming a plurality of the droplets 84 in the chamber 11. The rotation mechanism 221 can change the rotational speed of the rotation axis 222 in at least two stages. The at least two stages may be, for example, two stages of stop and rotation, with the stop at zero rotational speed. In the chamber 11, as a result of repeated changes in rotational speed, stirring proceeds and the mixed solution is finely sheared by the dispersion medium 82 by stirring. As a result, the mixed solution is refined in the dispersion medium 82, and a plurality of droplets 84 of the mixed solution partitioned by an interface between the dispersoid and the dispersion medium 82 are formed in the chamber 11. That is, by repeating the operation of changing the rotational speed of the cartridge 100, a plurality of droplets 84 are collectively produced in the chamber 11.

### (Operation of Changing Rotational Speed of Cartridge)

As shown in Figs. 4A and 4B, the operation of changing the rotational speed of the cartridge 100 includes an operation of reversing a direction of rotation of the cartridge 100 or an operation of accelerating and decelerating in the same direction. Changing the rotational speed includes accelerating or decelerating the cartridge 100 rotating in a fixed rotational direction, and performing stopping by deceleration and acceleration in an opposite direction in order to reverse the direction of rotation of the cartridge 100 and rotate the cartridge 100 in a reverse direction.

Fig. 4A shows an example of the operation of reversing the direction of rotation of the cartridge 100. For example, the cartridge 100 is alternately rotated around the rotation axis 222 to one side and the other side around the rotation axis 222. In this case, assuming that one side around the rotation axis 222 is positive (+) and the other side is negative (-), the rotational speed is positive, zero, negative, zero, positive, ..., thus acceleration from a stop state and deceleration to the stop state are repeated each time the rotational direction reverses. Therefore, it is possible to apply a large inertial force to the liquid in the chamber 11. As a result, the dispersoid and the dispersion medium 82 are efficiently stirred. As described above, when the direction of rotation of the cartridge 100 is reversed, a large inertial force can be applied when the rotational direction is reversed, so that the emulsion 83 can be efficiently produced.

Fig. 4B shows an example of the operation of accelerating and decelerating in the same direction. In Fig. 4B, for convenience, acceleration is indicated by a solid arrow in a circumferential direction of the cartridge 100, and deceleration is indicated by a dashed arrow. In Fig. 4B, the cartridge 100 is rotated in a clockwise direction, but may be in a counterclockwise direction. The cartridge 100 is rotated around the rotation axis 222 in a fixed direction around the rotation axis 222 so as to alternately repeat acceleration and deceleration. The deceleration may be performed until the rotation of the cartridge 100 is stopped, or acceleration and deceleration may be repeated between a predetermined high speed and a predetermined low speed. In Fig. 4B, although acceleration and deceleration are alternately repeated, the cartridge 100 also may be rotated in a pattern that does not repeat alternately, for example, like acceleration, acceleration, deceleration, acceleration, acceleration, deceleration, .... When the cartridge 100 is accelerated and decelerated in the same direction, it is not necessary to reversely rotate the rotation mechanism for rotating the cartridge 100, so that the emulsion 83 can be easily produced.

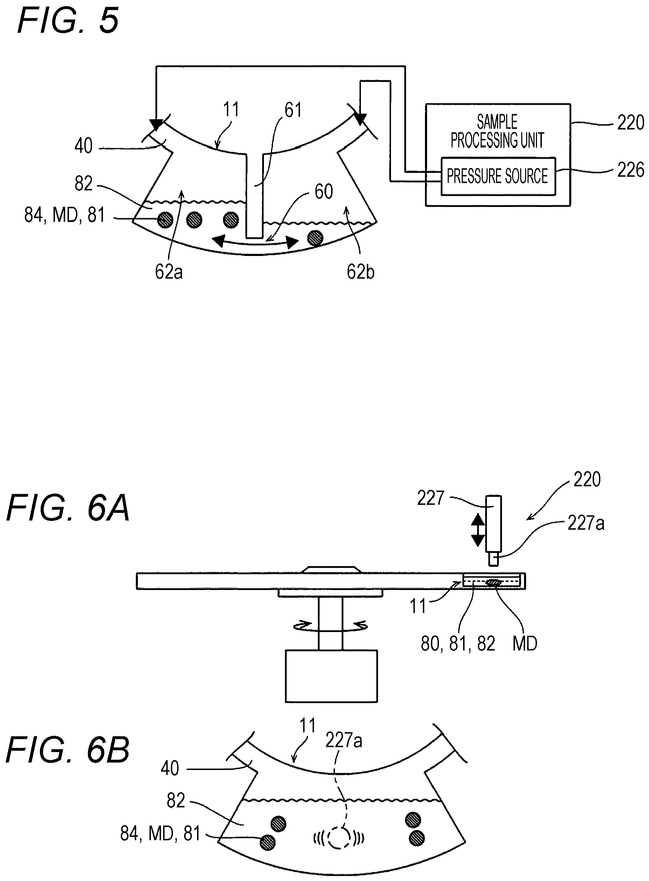

In the example of Fig. 5, the sample processing unit 220 includes a pressure source 226 for supplying pressure to the chamber 11 of the cartridge 100 installed in the installation unit 210. The pressure source 226 is, for example, a compressor or a pump.

In the example of Fig. 5, the chamber 11 is provided with a narrowed portion 60 in which a partial region in the chamber is narrower than the other portions. When the liquid in the chamber 11 flows, the narrowed portion 60 obstructs a flow of the liquid to change a flow rate of the liquid in the chamber 11 and promote stirring of the liquid. The narrowed portion 60 can be formed, for example, by the shape of the chamber itself, or a wall, a protrusion, a pillar or the like provided in the chamber. For example, in Fig. 5, the narrowed portion 60 is provided such that a radial width of the chamber 11 is narrower than that of other portions, by a wall 61 formed at a central portion in the chamber 11. The narrowed portion 60 divides the inside of the chamber 11 into a region 62a on one side and a region 62b on the other side across the narrowed portion 60, in the circumferential direction which is the direction of rotation of the cartridge 100.

The pressure source 226 is connected to, for example, the region 62a on one side and the region 62b on the other side, respectively. The pressure source 226 alternately supplies pressure to the region 62a on one side and the region 62b on the other side. By pressure supply, the liquid passes through the narrowed portion 60 to move to the region 62a on one side and the region 62b on the other side. When the liquid passes through the narrowed portion 60, the flow rate changes, and the dispersoid and the dispersion medium 82 in the chamber 11 are stirred. As a result, an emulsion 83 containing a plurality of droplets 84 in the chamber 11 is produced. The pressure source 226 may be connected to only one of the region 62a and the region 62b to alternately supply positive pressure and negative pressure.

In the example of Fig. 6, the sample processing unit 220 includes a vibratory apparatus 227 for applying vibration from outside to the chamber 11 of the cartridge 100 installed in the installation unit 210. The vibratory apparatus 227 is, for example, an ultrasonic probe.

The vibratory apparatus 227 is movable so as to approach or separate the cartridge 100 installed in the installation unit 210 by a moving mechanism (not shown). The vibratory apparatus 227 brings a vibrator 227a into contact with a position (refer to a broken line part in Fig. 6B) overlapping the chamber 11 storing the dispersoid and the dispersion medium 82 on the upper or lower surface of the cartridge 100 to vibrate the vibrator 227a. Vibration generated from the vibrator 227a is transmitted from the surface (i.e., the upper or lower surface) of the cartridge 100 to the liquid in the chamber 11. The vibration transmitted to the liquid in the chamber 11 stirs the dispersoid and the dispersion medium 82. As a result, the dispersoid is refined in the dispersion medium 82, and a plurality of droplets 84 is collectively produced in the chamber 11.

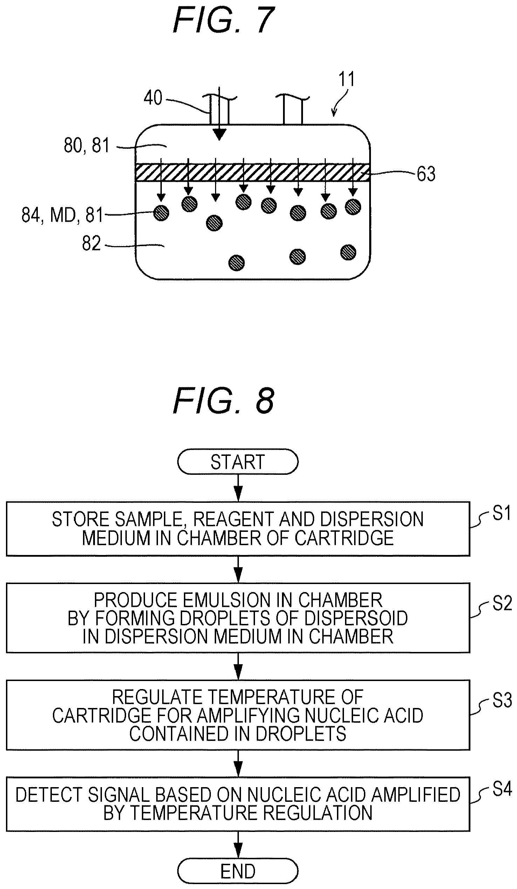

In the example of Fig. 7, an example in which a plurality of droplets 84 is collectively formed in the chamber 11 by passing the dispersoid through a porous filter 63 provided in the chamber 11 is shown. The dispersoid is stored on one side with respect to the filter 63, and the dispersion medium 82 is stored on the other side with respect to the filter 63. A pressure directed to the filter 63 is applied to the dispersoid in the chamber 11 by pressure supply to the chamber 11 or centrifugal force generated by the rotation of the cartridge 100. When the dispersoid passes through the filter 63 by the pressure, the dispersoid is refined, and the dispersoid refined by passing through the filter 63 flows into the dispersion medium 82. As a result, a plurality of droplets 84 is collectively produced in the chamber 11.

In the example of Fig. 7, the sample processing unit 220 includes the rotation mechanism 221 or the pressure source 226 described above. The rotation mechanism 221 applies pressure that passes through the filter 63 to the dispersoid in the chamber 11, by the centrifugal force generated by rotating the cartridge 100. The pressure source 226 supplies pressure that passes through the filter 63 to the dispersoid in the chamber 11, by supplying pressure into the chamber 11.

### (Outline of Method for Detecting Nucleic Acid)

A method for detecting nucleic acid according to the first embodiment will be described with reference to Fig. 8. The method for detecting nucleic acid of the first embodiment is a method for detecting nucleic acid using the cartridge 100 for detecting the signal based on the nucleic acid MD amplified in the emulsion 83, including the chamber 11 for producing the emulsion 83 in which the dispersoid containing the nucleic acid MD and the reagent 81 for amplifying the nucleic acid are dispersed in the dispersion medium 82. The method for detecting nucleic acid can be performed by the nucleic acid detection device 200 using the cartridge 100 into which the nucleic acid MD is injected.

As shown in Fig. 8, the method for detecting nucleic acid of the first embodiment includes the following steps S1 to S4. (S1) Store the nucleic acid MD, the reagent 81 for amplifying the nucleic acid, and the dispersion medium 82 in the chamber 11 of the cartridge 100. (S2) Produce an emulsion 83 in the chamber 11 by forming droplets 84 of the dispersoid in the dispersion medium 82 in the chamber 11. (S3) Regulate the temperature of the cartridge 100 for amplifying the nucleic acid MD contained in the droplets 84. (S4) Detect the signal based on the nucleic acid MD amplified by temperature regulation.

In step S1, the nucleic acid MD, the reagent 81 for amplifying nucleic acid, and the dispersion medium 82 can be directly injected into the chamber 11. When the nucleic acid MD, the reagent 81 for amplifying the nucleic acid and the dispersion medium 82 are stored in a site other than the chamber 11 of the cartridge 100, they are transferred to the chamber 11 via the passage 40 (see Fig. 1). As a result of step S1, the sample 80, the reagent 81 for amplifying nucleic acid and the dispersion medium 82 are stored inside the same chamber 11.

In step S2, dispersoid droplets 84 are formed in the dispersion medium 82 in the chamber 11 (see Fig. 2). As described above, the method for producing the emulsion 83 may employ any of a method of rotating the cartridge 100 (see Figs. 3 and 4), a method of stirring liquid by pressure supply (see Fig. 5), a method of applying vibration (see Fig. 6), a method of passing through a filter (see Fig. 7), and the like. In this case, the droplets 84 in the chamber 11 are formed by stirring the dispersion medium 82 and the dispersoid in the chamber 11. Thus, the emulsion 83 in which the droplets 84 of dispersoid containing the nucleic acid MD and the reagent 81 for amplifying nucleic acid are dispersed in the dispersion medium 82 is formed. The droplet 84 contained in the emulsion 83 contains the nucleic acid MD and the reagent 81 for amplifying the nucleic acid. This makes it possible to reliably amplify the nucleic acid MD by the reagent 81 in the finely compartmentalized individual droplets 84.

In step S3, the temperature of the cartridge 100 storing the emulsion 83 is regulated to change periodically. That is, a thermal cycle processing is performed by the temperature regulating unit 260. As a result, in individual droplets 84 contained in the emulsion 83, the nucleic acid MD is amplified by the reagent 81 for amplifying the nucleic acid. Temperature regulation may be performed on the chamber 11 in which the emulsion 83 is produced. That is, after the emulsion 83 is produced in the chamber 11, the thermal cycle processing can be performed on the emulsion 83 in the same chamber 11 by the temperature regulating unit 260.

In step S4, the signal based on the nucleic acid MD amplified by a temperature regulation is detected by the detection unit 270. When performing detection, the nucleic acid MD may remain stored in the droplet 84 or may be removed from the droplet 84 by demulsifying the emulsion 83.

As described above, in the method for detecting nucleic acid according to the first embodiment, since the emulsion 83 is produced in the chamber 11, and the nucleic acid in droplet 84 contained in the emulsion 83 is amplified by adjusting the temperature of the cartridge 100, it is possible to perform at least the step of producing the emulsion 83 and the nucleic acid amplification step of the nucleic acid detection assay inside the cartridge 100. As a result, it is possible to suppress sample mix-up and contamination, as compared with the case of transferring the emulsion 83 to a device outside the cartridge 100 for performing these steps. Then, since the emulsion 83 is produced in the chamber 11 by forming the droplets 84 of the dispersoid in the dispersion medium 82 in the chamber 11, unlike the case where droplets are formed one by one at a merging portion of the fluid channel, there is no need for precise liquid feed control required for droplet formation in the channel, and the dispersoid and the dispersion medium 82 can be stored in the chamber 11 to form droplets 84, there is also no need to feed the droplets formed at the merging portion to the chamber. As a result, the emulsion 83 can be produced more easily. Also, since the nucleic acid MD is amplified by adjusting the temperature of the cartridge 100, there is no need to transfer the emulsion 83 as compared with the case of transferring the emulsion 83 to another device for nucleic acid amplification. Therefore, it is possible to suppress sample mix-up and contamination. As a result of these, it is possible to suppress sample mix-up and contamination and also perform processing more easily.

### (Program)

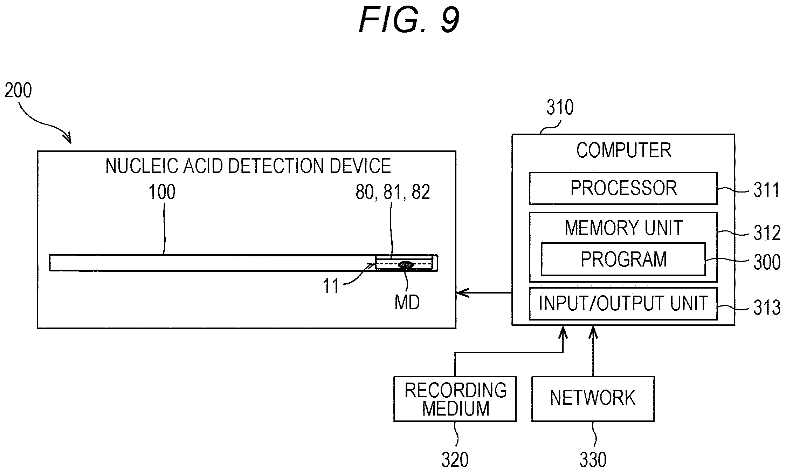

Next, a program 300 according to the first embodiment will be described with reference to Fig. 9.

Although illustration is omitted, the operation of each part of the nucleic acid detection device 200 shown in Fig. 1 and Fig. 3 is controlled by the control unit provided in the housing 201. The control unit is configured by a computer including, for example, a processor such as a CPU and a memory unit such as a memory. The control unit may be configured by a programmable device such as, for example, a field-programmable gate array (FPGA) constructed for the nucleic acid detection device 200.

On the other hand, as shown in Fig. 9, the nucleic acid detection device 200 may be controlled by an external computer 310 recording the program 300. The program 300 is a control program for causing the computer 310 to function as a control unit of the nucleic acid detection device 200.

The computer 310 includes, for example, a processor 311 such as a CPU, a memory unit 312 including volatile memory and non-volatile memory, and an input/output unit 313 connected by wire or wirelessly to an external equipment or network including the nucleic acid detection device 200. The program 300 is stored in the memory unit 312. The program 300 is executed by the processor 311, thereby causing the computer 310 to function as a control unit of the nucleic acid detection device 200.

The program 300 can be provided by being recorded on, for example, a computer readable non-transitory recording medium 320. The program 300 recorded on the recording medium 320 is stored in the memory unit 312 of the computer 310 by being read by a reading device (not shown) of the computer 310. The recording medium 320 does not include a transitory propagating signal for transmitting the program 300. The recording medium 320 includes, for example, a non-volatile semiconductor memory, an optical or magnetic recording medium, and the like. The program 300 can be provided from a network 330 connected to the computer 310, for example, by means of a transitory propagating signal for transmitting the program 300.

### (Cartridge)

Next, another configuration of the cartridge 100 will be described.

As described above, the cartridge 100 is, for example, a flat plate-shaped member in which a space is formed inside. The cartridge 100 may not have a flat plate shape, and may have a block shape, a spherical shape or the like. When the cartridge 100 is rotated, in order to stir the dispersoid and the dispersion medium 82 in the chamber 11 by inertial force at the time of changing the rotational speed, the cartridge 100 is preferably lightweight, and not eccentric so as not to cause vibration along with the rotation, so that acceleration and deceleration can be easily performed. Specifically, the cartridge 100 preferably has a disk shape. On the other hand, when the emulsion 83 is produced by the pressure source 226 or the vibratory apparatus 227, the planar shape of the cartridge 100 is not particularly limited, and may be a rectangular shape or the like other than the circular shape.

The shape of the chamber 11 formed in the cartridge 100 is not particularly limited. However, when the cartridge 100 is rotated, it is preferable that the chamber 11 has a shape extending along a circumferential direction around the rotation axis 222 or a tangential direction of a circle centered on the rotation axis 222. For example, when the dispersoid including the nucleic acid MD and the reagent 81 is a water-based and the dispersion medium 82 is an oil-based liquid, the dispersoid and the dispersion medium 82 are vertically separated in the chamber 11 as shown in Fig. 2. For this reason, when the dispersoid and the dispersion medium 82 are stirred by the change in the rotational speed, as the area of the interface where the dispersoid and the dispersion medium 82 are in contact with each other is larger, it is easy to apply a shear force between the dispersoid and the dispersion medium 82. Therefore, the chamber 11 preferably has a flat shape along the rotational direction. When the chamber 11 is along the radial direction centering on the rotation axis 222, the inertial force associated with the change in the rotational speed is likely to vary between the inner peripheral side and the outer peripheral side in the radial direction. Therefore, it is preferable that the chamber 11 extends along the circumferential direction around the rotation axis 222 or the tangential direction of the circle centered on the rotation axis 222.

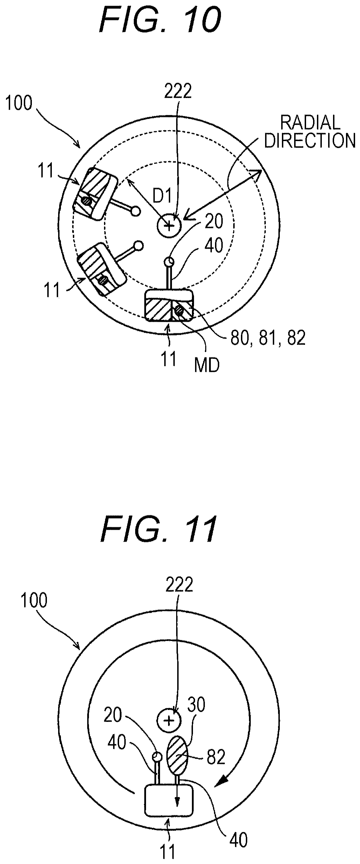

In an example shown in Fig. 10, the cartridge 100 includes a plurality of chambers 11. That is, the cartridge 100 is provided with a plurality of chambers 11 for producing an emulsion 83 in which the dispersoid containing the nucleic acid MD and the reagent 81 for amplifying the nucleic acid are dispersed in the dispersion medium 82. Thus, it is possible to collectively process a plurality of samples 80 collected from different living bodies and the same sample 80 in an amount exceeding the processing amount by one chamber 11 in the cartridge 100 to form an emulsion 83.

In the example of Fig. 10, the cartridge 100 includes a plurality of chambers 11 arranged in an arc shape having substantially equal radial distance from the rotation axis 222. In Fig. 10, the plurality of chambers 11 are provided at positions where rotational trajectories of the respective chambers 11 overlap with each other in plan view. The circumferential movement speed of the chambers 11 when the cartridge 100 rotates is proportional to radial distance D1 from the rotation axis 222 of each chamber 11. Therefore, when the distance from the rotation axis 222 of each chamber 11 is different, the magnitude of inertial force acting on each chamber 11 will be different, so that quality of emulsion 83 to be produced can vary among the chambers 11. On the other hand, in Fig. 10, the plurality of chambers 11 are arranged in an arc shape having substantially equal radial distance from the rotation axis 222, whereby the magnitude of inertial force acting on each chamber 11 will be substantially equal along with a rotation. Therefore, the quality of emulsion 83 to be produced can be made more uniform. For example, it is possible to suppress variations in the size of the droplets 84 contained in the emulsion 83 produced in each chamber 11 and the number of the droplets 84 by rotation.

As shown in Fig. 11, in addition to the sample introduction unit 20 and the chamber 11, the cartridge 100 can include a liquid storage unit 30 for storing liquid used for processing. The liquid storage unit 30 is a space formed to have a predetermined volume in the cartridge 100 as in the chamber 11. The liquid storage unit 30 is connected to the chamber 11 via the passage 40 formed in the cartridge 100.

In Fig. 11, as an example of the liquid storage unit 30, a liquid storage unit 30 in which the dispersion medium 82 is stored in advance is shown. That is, the cartridge 100 includes the liquid storage unit 30 in which the dispersion medium 82 is stored in advance, which is fluidly connected to the chamber 11. Then, the cartridge 100 is configured so that the dispersion medium 82 is transferred from the liquid storage unit 30 to the chamber 11 by being rotated around the rotation axis 222.

Specifically, the liquid storage unit 30 is provided at a position closer to the rotation axis 222 than the chamber 11 in the cartridge 100. The passage 40 is provided at a position on the outer peripheral side in the radial direction centering on the rotation axis 222 in the liquid storage unit 30. The passage 40 is connected to the chamber 11. For this reason, when the cartridge 100 is rotated around the rotation axis 222, a radially outward centrifugal force acts on the dispersion medium 82 stored in the liquid storage unit 30. As a result, the dispersion medium 82 is transferred from the liquid storage unit 30 into the chamber 11 by the centrifugal force generated by the rotation. When the liquid is transferred from the liquid storage unit 30 into the chamber 11, a centrifugal force of a magnitude necessary to move the liquid should be generated, so that the rotational speed of the cartridge 100 does not need to be repeatedly changed. When the liquid is transferred from the liquid storage unit 30 into the chamber 11 by pressure, the positional relationship between the chamber 11 and the liquid storage unit 30 is arbitrary.

### (Specific Configuration Example of Method for Detecting Nucleic Acid)

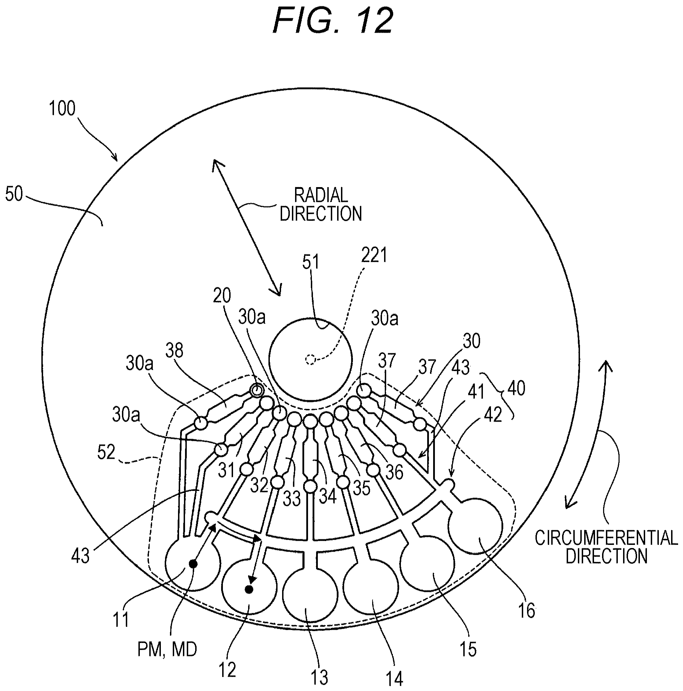

Next, a specific configuration example of the method for detecting nucleic acid according to the first embodiment will be described. Hereinafter, an example using the cartridge 100 shown in Fig. 12 will be described.

### <Cartridge>

In the example of Fig. 12, the cartridge 100 is a disc-type cartridge configured by a plate-like and disc-shaped substrate 50. Each part in the cartridge 100 is formed by laminating a through-hole part formed in the substrate 50 and a film (not shown) covering the entire surface including the through-hole part on both surfaces of the substrate 50. The substrate 50 has a thickness that facilitates the temperature regulation of the cartridge 100 by the temperature regulating unit 260 (see Fig. 1). For example, the thickness of the substrate 50 is several millimeters, specifically about 1.2 mm.

The substrate 50 is provided with a hole 51 and a sample processing region 52. In the sample processing region 52, a plurality of chambers 11 to 16, a sample introduction unit 20, a plurality of liquid storage units 30, and a plurality of passages 40 are provided.

The hole 51 penetrates the substrate 50 at the center of the substrate 50. The cartridge 100 is installed in the nucleic acid detection device 200 such that the center of the hole 51 coincides with the center of the rotation axis 222. Hereinafter, the radial direction and the circumferential direction of a circle centered on the hole 51 will be referred to as "radial direction" and "circumferential direction", respectively.

The sample introduction unit 20 is an opening for introducing a sample 80 collected from a living body into the cartridge 100. The sample 80 contains nucleic acid MD. In the example of Fig. 12, a mixed solution of the sample 80 and the reagent 81 for amplifying the nucleic acid is injected into the sample introduction unit 20. The mixed solution is prepared in advance by an operator and injected into the cartridge 100, but the separately injected sample 80 and reagent 81 may be mixed in the cartridge 100.

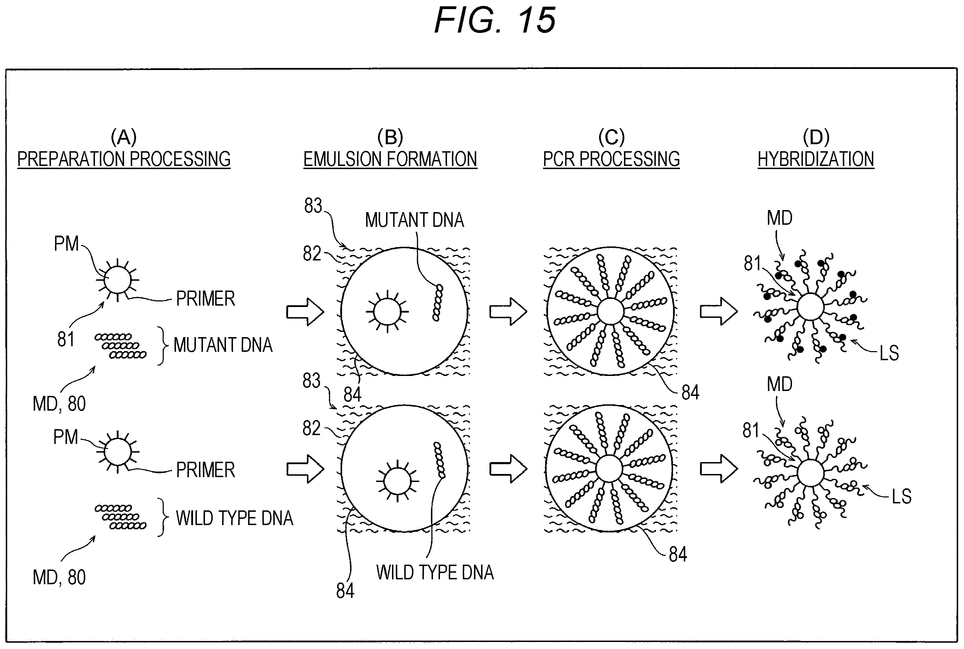

The reagent 81 is a nucleic acid amplification reagent that amplifies the nucleic acid in the sample 80. The nucleic acid amplification reagent contains substances necessary for PCR such as DNA polymerase. Furthermore, the reagent 81 contains a magnetic particle PM (see Fig. 15) that binds to the nucleic acid in the sample 80. The magnetic particle PM has a primer for nucleic acid amplification applied to the surface. The magnetic particle may be a particle that contains a material having magnetism as a base material and that is used for normal specimen measurement. For example, magnetic particle using Fe₂O₃ and/or Fe₃O₄, cobalt, nickel, phyllite, magnetite or the like as a base material can be used. A mixed solution in which the sample 80, the nucleic acid amplification reagent and the magnetic particle PM are mixed is introduced into the cartridge 100 from the sample introduction unit 20. In the mixed solution, the nucleic acid MD is bound to the magnetic particle PM.

The chambers 11 to 16 are chambers capable of storing liquid. The chambers 11 to 16 are arranged in the circumferential direction near the outer periphery of the substrate 50. Fig. 12 shows an example in which six chambers 11 to 16 are provided in the cartridge 100. As described later, in the example of Fig. 12, the chamber in which the emulsion 83 is produced is the chamber 11.

The liquid storage unit 30 is a storage space for storing the liquid to be transferred to the chamber. Each of the liquid storage units 30 is connected to any one of the chambers via the passage 40. Fig. 12 shows an example in which nine liquid storage units 30 are provided in the cartridge 100. The respective liquid storage units 30 are arranged in the circumferential direction near the center of the substrate 50. The liquid storage units 30 are respectively arranged at a position closer to the rotation axis 222 than the connected chambers. Therefore, it is possible to transfer the liquid stored in the liquid storage unit 30 from the liquid storage unit 30 to the chamber by centrifugal force by rotating the cartridge 100 around the rotation axis 222.

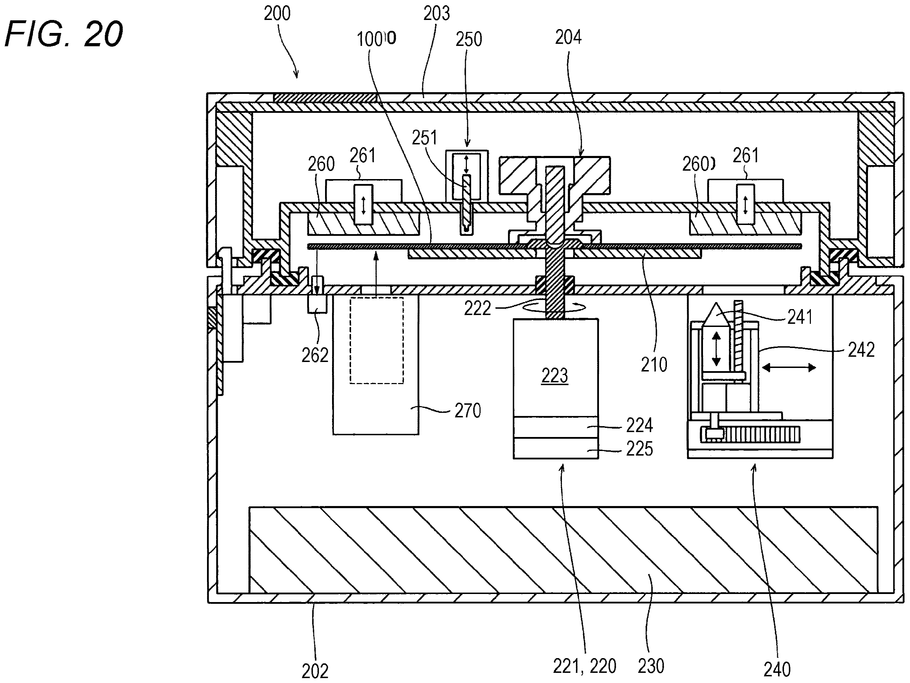

The liquid storage unit 30 includes a sealing body 30a for sealing a connection portion with the chamber 11. The liquid in the liquid storage unit 30 is each stored in advance in the liquid storage unit 30 when the cartridge 100 is manufactured. The sealing body 30a is a plug that closes the liquid storage unit 30 and the external space. The sealing body 30a is configured to be openable by being pressed from above by an opening mechanism 250 (see Fig. 20) of the nucleic acid detection device 200. Before the sealing body 30a is opened, the reagent in the liquid storage unit 30 does not flow into the passage 40, and when the sealing body 30a is opened, the reagent in the liquid storage unit 30 flows out.

The liquid storage unit 30 includes a first liquid storage unit 38 that stores the mixed solution injected from the sample introduction unit 20. By rotating the cartridge 100 around the rotation axis 222, the mixed solution of the sample 80 and the reagent 81 is transferred as a dispersoid from the first liquid storage unit 38 to the chamber 11.

All of the liquid storage units 30 for storing the reagent in advance store the reagent capable of performing one measurement. That is, the cartridge 100 includes the liquid storage unit 30 that stores the reagent capable of performing one measurement on nucleic acid. In the cartridge 100 configured as described above, different reagents are stored in each cartridge 100 for one time use.

In Fig. 12, six chambers 11, 12, 13, 14, 15 and 16 of substantially the same shape are arranged in the circumferential direction so as to be adjacent to each other, and six chambers 11, 12, 13, 14, 15 and 16 are connected via the passages 40 each extending in the circumferential direction. Between the six chambers 11 to 16, nucleic acids are sequentially transferred one by one via the passage 40 from one side (the side of the chamber 11) to the other side (the side of the chamber 16).

The passage 40 includes six radial areas 41 extending in the radial direction and an arc-shaped circumferential area 42 extending in the circumferential direction. The circumferential area 42 is connected to the six radial areas 41. The six radial areas 41 are connected to the chambers 11 to 16, respectively. Each liquid storage unit 30 is connected to the chamber or passage via a flow path 43 extending in the generally radial direction.

The nucleic acid MD bound to the magnetic particle PM is transferred to the passage 40 and the other chamber 11, by a combination of the rotation of the cartridge 100 and the action of magnetic force. Specifically, the nucleic acid detection device 200 further includes a transfer mechanism 240 (see Fig. 20) that applies a magnetic force to the cartridge 100. The transfer mechanism 240 can move a magnetic force act position, at least in the radial direction. Thus, the magnetic particle PM is moved in the radial direction by the magnetic force, between the inside of the chamber 11 and the arc-shaped circumferential area 42 of the passage 40. The cartridge 100 is rotated, thereby moving the magnetic particle PM in the circumferential direction in the arc-shaped circumferential area 42. When the magnetic particle PM move to a position of the next chamber, the magnetic particle PM are moved in the radial direction by the magnetic force, between the arc-shaped circumferential area 42 of the passage 40 and the inside of the next chamber. As described above, the magnetic particle PM bound to the nucleic acid MD are sequentially moved to the chambers 11 to 16, by a combination of the radial movement by the action of the magnetic force and the circumferential movement by the rotation.

The sample processing region 52 in the example of Fig. 12 is formed only in approximately one-third area of the substrate 50. However, the sample processing region 52 is not limited thereto, and two more sample processing regions 52 may be formed in the remaining two-third region of the substrate 50, and three sample processing regions 52 may be provided on the substrate 50. One sample processing region 52 may be formed over a region larger than one-third region of the substrate 50.

The number and shape of the chambers 11 and the passages 40 are not limited to those shown in Fig. 12. The configuration of each part of the sample processing region 52 is determined in accordance with the contents of the sample processing assay performed in the sample processing region 52.

### <Processing Flow of Biological Sample>

Next, an example of a specific assay using the cartridge 100 of Fig. 12 will be described.

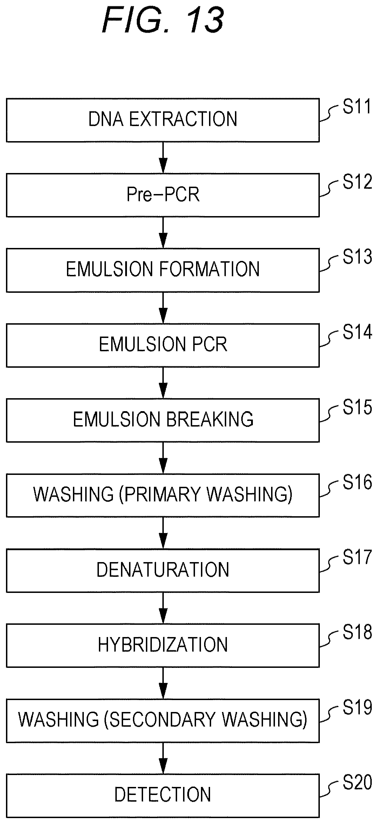

Fig. 13 shows an example of a flow of an emulsion PCR assay. Fig. 15 is a diagram for illustrating progress of the reaction in an emulsion PCR assay. The processing using the cartridge 100 shown in Fig. 12 includes steps S13 to S20.

In step S11, DNA is extracted from a sample such as blood by preprocessing (see Fig. 15A). The preprocessing is performed using a dedicated nucleic acid extraction apparatus.

In step S12, the extracted DNA is amplified by pre-PCR processing (see Fig. 15A). The pre-PCR processing is a processing of preliminarily amplifying the DNA contained in the extract liquid after the preprocessing to such an extent that the following emulsion formation processing becomes possible. In the pre-PCR processing, the extracted DNA is mixed with a reagent for PCR amplification containing a polymerase and a primer, and the DNA in a dispersoid are amplified by temperature control by a thermal cycler. The thermal cycler performs a thermal cycle processing of repeating one cycle of changing to a plurality of different temperatures on the dispersoid a plurality of times. The pre-PCR processing may be omitted, depending on the concentration of the nucleic acid to be extracted.

Step S 13 is an emulsion forming step of dispersing the dispersoid containing the nucleic acid MD (DNA), the nucleic acid amplification reagent, and the magnetic particle PM as a carrier of the nucleic acid in the dispersion medium 82. The reagent for amplification reaction of the nucleic acid contains substances necessary for PCR such as DNA polymerase. In step S13, an emulsion 83 containing a reagent containing magnetic particle, polymerase and the like and DNA is formed using the cartridge 100 (see Fig. 15B). That is, a droplet 84 composed of a dispersoid containing the nucleic acid amplification reagent containing the magnetic particle PM, a polymerase and the like and DNA is formed, and a large number of droplets 84 are dispersed in the dispersion medium 82. As a result, DNA as the nucleic acid MD is subjected to limiting dilution (dilution such that the target component is 1 or 0 in each microcompartment) and dispersed in the microcompartment. That is, individual droplets 84 in the emulsion 83 is a microcompartment, and the droplet 84 is formed such that about one magnetic particle and one target DNA molecule as the nucleic acid MD are contained in each droplet 84.

Step S 14 is an emulsion PCR step of amplifying the nucleic acid (DNA) in the droplet 84 formed in the emulsion forming step. In step S14, by temperature control by the thermal cycler, DNA is bound to the primer on the magnetic particle PM within each droplet 84 of the emulsion 83, and amplified (emulsion PCR) (see Fig. 15C). Thus, the target DNA molecule is amplified within individual droplets 84. That is, an amplification product of the nucleic acid is formed within each droplet 84. The amplified nucleic acid binds to the magnetic particle PM as the carrier via the primer within the droplet 84.

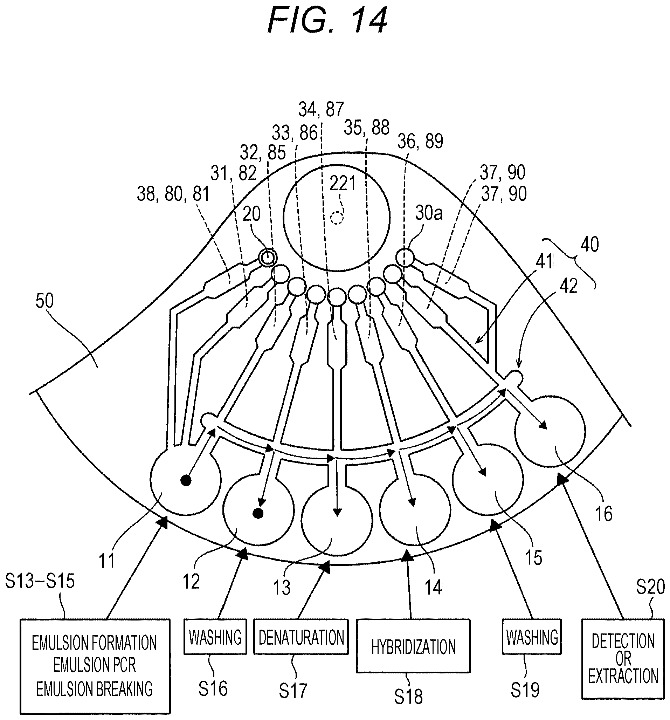

Step S15 is an emulsion breaking step of breaking down a droplet 84 containing magnetic particle PM carrying an amplification product of nucleic acid (DNA) in the emulsion PCR step. In other words, step S15 is a step of demulsifying the emulsion 83 after the emulsion PCR step. After amplifying the DNA on the magnetic particle in step S14, the emulsion 83 is broken in step S15, and the magnetic particle PM containing the amplified DNA is taken out from the droplet 84 (emulsion breaking). A reagent 85 (see Fig. 14) for demulsifying the emulsion is used to break the emulsion. The reagent 85 for demulsifying the emulsion contains an alcohol or a surfactant, and the like.

Step S 16 is a washing step of washing the magnetic particle PM taken out from the droplet 84 by demulsification in the emulsion breaking step. In step S16, the magnetic particle PM taken out from the droplet 84 is washed with a reagent 86 (see Fig. 14) for washing the nucleic acid (primary washing). The reagent 86 for washing the nucleic acid contains, for example, an alcohol. The alcohol removes an oil film on the magnetic particle.

Step S17 is a denaturation step of denaturing amplified one or more double-stranded DNAs binding to the magnetic particle PM into single strands. In step S17, the washed magnetic particle PM are denatured by a reagent 87 (see Fig. 14) for washing the nucleic acid. The reagent 87 for washing the nucleic acid contains, for example, an alcohol. The alcohol denatures the amplified one or more double-stranded DNAs into single strands.

Step S18 is a hybridization step in which an amplification product of DNA is reacted with a labeling substance. After the denaturation step, in step S18, the DNA denatured to single strands on the magnetic particle PM is hybridized with a labeling substance LS by a reagent 88 (see Fig. 14) containing the labeling substance LS that reacts with the amplification product of the nucleic acid (hybridization) (see Fig. 15D). The labeling substance LS includes, for example, a fluorescent substance. The labeling substance LS is designed to specifically bind to the DNA to be detected.

In step S19, the magnetic particle PM carrying the DNA bound to the labeling substance LS are washed with a reagent 89 (see Fig. 14) for washing the nucleic acid (secondary washing). In the secondary washing step, for example, PBS (phosphate buffered saline) is used as a washing liquid. PBS removes unreacted labeling substances (including labeling substances nonspecifically adsorbed to the magnetic particle) not bound to the DNA.

In step S20, the nucleic acid MD is detected via the hybridized labeling substance LS. The nucleic acid MD is captured, for example, by an imaging unit included in the detection unit 270. Based on the captured image, the number of magnetic particles emitting fluorescence is counted.

Next, details of processing using the cartridge 100 will be described with reference to Fig. 14

The cartridge 100 includes the chamber 11 in which the emulsion 83 is formed. As shown in Fig. 14, steps S13 to S15 are performed in the chamber 11.

The cartridge 100 for performing the emulsion PCR assay shown in Fig. 13 includes nine liquid storage units 30. By these liquid storage units 30, the cartridge 100 includes the first liquid storage unit 38 for storing the reagent 81 for amplifying the nucleic acid and the magnetic particle PM, a second liquid storage unit 31 for storing the dispersion medium 82, a third liquid storage unit 32 for storing the reagent 85 for demulsifying the emulsion 83, a fourth liquid storage unit 35 for storing a reagent containing the labeling substance LS, and a fifth liquid storage unit 33 for storing a reagent 86 for washing the nucleic acid.

### <Emulsion Formation>

In step S13, first, a dispersoid containing the sample 80 containing the nucleic acid MD, the reagent 81 for amplifying the nucleic acid, and magnetic particle PM as a carrier of the nucleic acid is injected from the sample introduction unit 20 into the first liquid storage unit 38. In addition, the sealing body 30a of the second liquid storage unit 31 is opened. The rotation mechanism 221 transfers the reagent 81 for amplifying the nucleic acid stored in the first liquid storage unit 38 included in the cartridge 100 to the chamber 11 by rotating the cartridge 100. The rotation mechanism 221 mixes the nucleic acid and the reagent 81 for amplifying the nucleic acid in the chamber 11 to form the dispersoid in the chamber 11 by rotating the cartridge 100. Thus, it is possible to prepare the dispersoid for forming the droplet 84 in the chamber 11 simply by rotating the cartridge 100.

Since the sealing body 30a of the second liquid storage unit 31 is opened, the dispersion medium 82 is transferred from the second liquid storage unit 31 to the chamber 11 by a centrifugal force accompanying the rotation. In the chamber 11, the introduced sample 80, the reagent 81 for amplifying the nucleic acid in the sample 80, the magnetic particle PM, and the dispersion medium 82 are stored. Then, the rotation mechanism 221 rotates the cartridge 100 to mix the dispersion medium 82 and the dispersoid in the chamber 11, thereby collectively forming a plurality of droplets 84 in the chamber 11. Thus, the emulsion 83 containing a plurality of droplets 84 can be formed in the chamber 11 by mixing simply by rotating the cartridge 100 using the rotation mechanism 221. Thus, the emulsion 83 can be efficiently produced in the chamber 11, as compared with the case where droplets are formed one by one at the merging portion of the fluid channel.

Specifically, the rotation mechanism 221 repeats an operation of changing rotational speed of the rotated cartridge 100 to mix the dispersoid and the dispersion medium 82 in the chamber 11, thereby collectively forming a plurality of the droplets 84 in the chamber 11. Thus, it is possible to change an inertial force acting on the liquid in the chamber 11 to efficiently mix the dispersoid and the dispersion medium 82 by repeating the operation of changing the rotational speed of the cartridge 100 rotated by the rotation mechanism 221. As a result, it is possible to collectively form a plurality of droplets 84 in the chamber 11 easily and efficiently by the rotation mechanism 221.

The operation of changing the rotational speed of the cartridge 100 is, for example, as shown in Fig. 4A, an operation of rotationally driving the cartridge 100 in a predetermined direction and then driving the cartridge 100 in a reverse direction. The operation of changing the rotational speed of the cartridge 100 is, for example, as shown in Fig. 4B, an operation of rotationally driving the cartridge 100 in a predetermined direction and then stopping the cartridge 100 in a reverse direction. As described above, the rotation mechanism 221 produces the emulsion 83 by repeating an operation of rotationally driving the cartridge 100 in a predetermined direction and then stopping or driving the cartridge 100 in a reverse direction. Thus, when the cartridge 100 is stopped or the direction of rotation is reversed, rapid speed change is easily generated, and a large inertial force can be applied, so that the emulsion 83 can be efficiently produced.

The sample processing unit 220 forms an emulsion 83 containing the droplets 84 of the dispersoid storing one molecule or one of the nucleic acid in the chamber 11. Thus, as shown in Fig. 15, it is possible to compartmentalize each one of the nucleic acid MD contained in the sample into the droplets 84 contained in the emulsion 83 to ensure processing. That is, it is possible to compartmentalize each one of the nucleic acid contained in the sample into the droplets 84 contained in the emulsion 83 to ensure processing. As a result, even nucleic acid which is contained in an extremely small amount in a sample can be amplified with high accuracy, so that it is possible to improve detection accuracy even when detecting the amplified nucleic acid.

### <Emulsion PCR>

In step S14, after the emulsion 83 is formed, the nucleic acid contained in the dispersoid is amplified by periodically changing the temperature of the cartridge 100 to a plurality of temperature ranges. Thus, it is possible to perform a thermal cycle processing in the droplets 84 (see Fig. 15) contained in the emulsion 83 to efficiently amplify the nucleic acid.

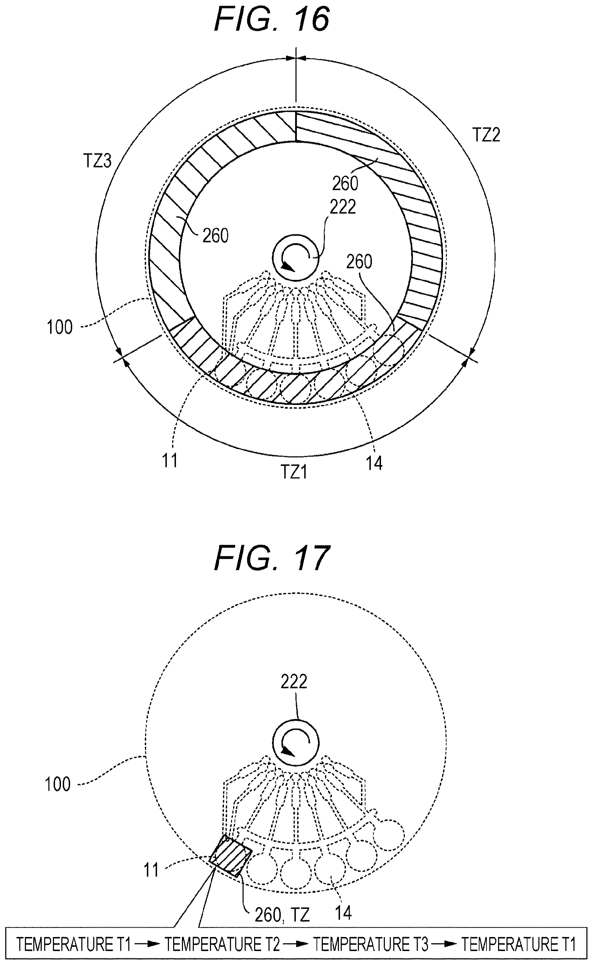

The thermal cycling is performed in the chamber 11 by the temperature regulating unit 260 (see Fig. 16). The temperature regulating unit 260 regulates the temperature of the chamber 11 by heating the cartridge 100 from outside of the cartridge 100. Thus, for example, unlike the case where the emulsion 83 is transferred from the cartridge 100 to a mechanism for performing a temperature regulation, or the case where a temperature regulation is performed by directly contacting the emulsion 83, it is possible to amplify the nucleic acid by performing a temperature regulation from outside of the cartridge 100 while storing the emulsion 83 in the cartridge 100. As a result, it is possible to further effectively suppress sample mix-up and contamination.

The temperature regulating unit 260 changes the temperature of the cartridge 100 by coming into contact with the cartridge 100. Heat can be efficiently transferred by bringing the temperature regulating unit 260 into contact with the cartridge 100, as compared with the case where the cartridge 100 is heated in a non-contact manner, so that the temperature of the cartridge 100 can be easily changed. The temperature regulating unit 260 performs the temperature regulation by coming into contact with the cartridge 100 from one or both of the upper surface and the lower surface of the disk-shaped cartridge 100.

For example, in Fig. 16, the temperature regulating unit 260 includes a plurality of temperature zones TZ having different temperatures, which are plurally arranged in the rotational direction on the cartridge 100 arranged in the installation unit 210. The temperature regulating unit 260 is formed in the shape along the circumferential direction centering on the rotation axis 222 in plan view. In the example of Fig. 16, the temperature regulating unit 260 is provided in a belt-like range overlapping a rotation track of the chamber 11 in plan view. A plurality of temperature regulating units 260 are provided in the circumferential direction. A plurality of temperature regulating units 260 form temperature zones TZ set to be different temperature from each other. In Fig. 16, three temperature regulating units 260 form three temperature zones TZ1 to TZ3 equally divided in the range of 120 degrees in the circumferential direction. The temperature zones TZ1 to TZ3 are set, for example, at three temperatures, low temperature, medium temperature, and high temperature, in the range of 30°C or more and 90°C or less.

The rotation mechanism 221 rotates the cartridge 100 to sequentially move the chamber 11 to the arrangement positions of the plurality of temperature zones TZ and periodically changes the temperature of the cartridge 100 into a plurality of temperature ranges, thereby amplifying the nucleic acid contained in the dispersoid in the chamber 11. The rotation mechanism 221 performs thermal cycling by rotating the cartridge 100 and arranging in order the chambers 11 in which the emulsions 83 are formed in the respective temperature zones TZ1 to TZ3. The number of temperature regulating units 260 (i.e., the number of temperature zones TZ) is not limited to this, and may be four or more. Thus, the step of amplifying nucleic acid can be performed simply by rotating the cartridge 100 and positioning the chamber 11 in each temperature zone TZ of the temperature regulating unit 260. In this case, a set temperature of each temperature zone TZ may be constant, and control for periodically changing the temperature of the temperature regulating unit 260 is unnecessary, so that temperature control can be facilitated.

For example, in Fig. 17, the thermal cycling is performed by one temperature regulating unit 260 that periodically changes the temperature in plan view. In the example of Fig. 17, the temperature regulating unit 260 is provided at a position overlapping the rotation track of the chamber 11 in plan view. The temperature regulating unit 260 can accommodate one chamber 11 in a temperature zone TZ to be formed. The temperature regulating unit 260 forms a temperature zone TZ that can be changed to an arbitrary temperature within a variable range by a Peltier element or the like. In Fig. 17, one temperature regulating unit 260 forms a temperature zone TZ in which the temperature changes in three stages. The temperature zone TZ is changed, for example, to three temperature stages T1 to T3 of low, medium and high temperatures, in the range of 30°C or more and 90°C or less.

The rotation mechanism 221 rotates the cartridge 100 to move the chamber 11 storing the emulsion 83 to the arrangement position of the temperature regulating unit 260. That is, the rotation mechanism 221 rotates the cartridge 100 to arrange the chamber 11 in which the emulsion 83 is formed at a position overlapping the temperature zone TZ. The temperature regulating unit 260 amplifies the nucleic acid contained in the dispersoid in the chamber 11 by periodically changing the temperature of the cartridge 100 to a plurality of temperature ranges. The number of temperature stages to be changed by the temperature regulating unit 260 is not limited to this, and may be four or more. Thus, the step of amplifying nucleic acid can be performed simply by rotating the cartridge 100 and positioning the chamber 11 at the arrangement position of the temperature regulating unit 260. In addition, since one chamber 11 can be processed by one temperature regulating unit 260, device configuration for performing a thermal cycle processing can be simplified. When a plurality of chambers 11 are provided, the thermal cycle processing can be performed simultaneously by also providing a plurality of temperature regulating units 260.

With such a configuration, PCR processing is performed in the emulsion 83. After thermal cycle processing, the temperature regulating unit 260 is separated from the cartridge 100. The cartridge 100 is returned to room temperature.

### <Emulsion Breaking>

Referring back to Fig. 14, in step S15, after amplifying the nucleic acid contained in the dispersoid, the reagent 85 for demulsifying the emulsion is transferred from the third liquid storage unit 32 to the chamber 11 in which the emulsion 83 is formed.

First, after amplifying the nucleic acid contained in the dispersoid, a sealing body 30a of the third liquid storage unit 32 is opened. Thus, the sealing body 30a can prevent the reagent 85 for demulsifying the emulsion from being unintentionally fed from the third liquid storage unit 32. Therefore, after forming the emulsion 83 and amplifying the nucleic acid, the emulsion 83 can be reliably demulsified.

Next, the rotation mechanism 221 rotates the cartridge 100 to transfer the reagent 85 for demulsifying the emulsion 83 from the third liquid storage unit 32 included in the cartridge 100 to the chamber 11 in which the emulsion 83 is formed. Thus, the nucleic acid amplified in the droplets 84 contained in the emulsion 83 can be taken out by the reagent 85 for demulsifying the emulsion. At this time, unlike a case of feeding liquid by air pressure or the like, the reagent 85 for demulsifying the emulsion can be fed to the chamber 11 simply by rotating the cartridge 100 by the rotation mechanism 221, so that the emulsion 83 can be easily demulsified.

When the reagent 85 for demulsifying the emulsion is transferred into the chamber 11, the emulsion 83 is broken, and the magnetic particle PM containing the amplified nucleic acid is taken out from the droplet 84. The inside of the chamber 11 may be stirred by rotating the cartridge 100 and changing the rotational speed. Thus, the reagent 85 for demulsifying the emulsion and the emulsion 83 are stirred, and demulsification can be efficiently performed.

### <Primary Washing>

The cartridge 100 includes a third chamber 12 connected to the chamber 11. The liquid storage unit 30 is connected to the third chamber 12. The liquid storage unit 30 includes a fifth liquid storage unit 33 for storing a reagent 86 for washing the nucleic acid. In step S16, the rotation mechanism 221 transfers the reagent 86 for washing the nucleic acid stored in the fifth liquid storage unit 33 included in the cartridge 100 by rotating the cartridge 100, to bring the nucleic acid captured on a carrier into contact with the reagent 86 for washing the nucleic acid in the cartridge 100. The carrier is a magnetic particle PM that binds to the nucleic acid. Thus, unnecessary components contained in the sample 80 can be removed by washing by bringing the nucleic acid into contact with the reagent 86 for washing the nucleic acid. The unnecessary component means all components unnecessary for detection of the nucleic acid to be detected.

The sealing body 30a of the fifth liquid storage unit 33 is opened, for example, at the same timing as the timing when the nucleic acid contained in the dispersoid is amplifies and then the sealing body 30a of the third liquid storage unit 32 is opened. Thus, the sealing body 30a can prevent the reagent 86 for washing the nucleic acid from being unintentionally fed from the fifth liquid storage unit 33. In addition, when the third liquid storage unit 32 and the fifth liquid storage unit 33 are opened at the same timing, only one operation of rotating the cartridge 100 for liquid feed is sufficient.

As mentioned above, the reagent 86 for washing the nucleic acid contains an alcohol. When amplifying the nucleic acid contained in the dispersoid in step S14, the temperature of the cartridge 100 is changed in the range of 30°C or more and 90°C or less. Therefore, when the reagent 86 for washing the nucleic acid is affected by temperature change, the alcohol may be vaporized. Therefore, by feeding the reagent 86 for washing the nucleic acid to the third chamber 12 after amplification of the nucleic acid, it is possible to prevent the reagent 86 for washing the nucleic acid from being affected by temperature change. This configuration is particularly effective in that vaporization of alcohol can be suppressed when the reagent 86 for washing the nucleic acid contains an alcohol.

In step S16, after the reagent 85 for demulsifying the emulsion is transferred to the chamber 11, a magnetic force is applied to the cartridge 100, thereby transferring the nucleic acid bound to the magnetic particle PM from the demulsified liquid, from the chamber 11 to the third chamber 12. Since the magnetic particle PM carrying the nucleic acid are taken out from the droplet 84 by the demulsification, the magnetic particle PM are moved to the third chamber 12 by a magnetic force by the transfer mechanism 240 and the rotation of the cartridge 100 by the rotation mechanism 221, as described above.

That is, the transfer mechanism 240 transfers the magnetic particle PM bound to the nucleic acid to the third chamber 12 by magnetic force, along with the rotation of the cartridge 100 by the rotation mechanism 221, thereby bringing the nucleic acid captured on the magnetic particle PM into contact with the reagent 86 for washing the nucleic acid. Thus, the nucleic acid can be transferred to the third chamber 12 and washed simply by applying a magnetic force to the cartridge 100 from outside by the transfer mechanism 240 without feeding liquid by air pressure or the like, by binding the nucleic acid MD to the magnetic particle PM. In the case of feeding liquid by air pressure or the like, the nucleic acid in the chamber is transferred together with the liquid, but in the transfer by magnetic force, only the nucleic acid bound to the magnetic particle PM can be transferred to the third chamber 12 leaving the liquid behind. As a result, a transfer of the unnecessary components to the third chamber 12 can be suppressed, and the washing of the nucleic acid in the third chamber 12 can be efficiently performed.

In the third chamber 12, the magnetic particle PM carrying the nucleic acid is washed with the reagent 86 for washing the nucleic acid.

### <Denaturation>

The cartridge 100 includes a chamber 13 connected to the third chamber 12. The cartridge 100 includes a liquid storage unit 34 for storing the reagent 87 for washing the nucleic acid. The liquid storage unit 34 is connected to the chamber 13.

In step S17, the rotation mechanism 221 amplifies at least nucleic acid contained in the dispersoid, and then the rotation mechanism 221 rotates the cartridge 100 around the rotation axis 222, thereby transferring the reagent 87 for washing the nucleic acid from the liquid storage unit 34 to the chamber 13. In the cartridge 100, the nucleic acid after subjected to processing for washing the nucleic acid in the chamber 13 is subjected to processing for washing the nucleic acid.

The sealing body 30a of the liquid storage unit 34 is opened, for example, at the same timing as the timing when the nucleic acid contained in the dispersoid is amplified and then each of the sealing bodies 30a of the third liquid storage unit 32 and the fifth liquid storage unit 33 is opened. When the third liquid storage unit 32, the fifth liquid storage unit 33, and the liquid storage unit 34 are opened at the same timing, only one operation of rotating the cartridge 100 for liquid feed is sufficient.

In step S17, after the rotation mechanism 221 transfers the reagent 87 for washing the nucleic acid to the chamber 11, the transfer mechanism 240 applies a magnetic force to the cartridge 100, thereby transferring the nucleic acid bound to the washed magnetic particle PM from the third chamber 12 to the chamber 13.

In the chamber 13, the reagent 87 for washing the nucleic acid denatures an amplified one or more double-stranded DNAs into single strands.

### <Hybridization>

The cartridge 100 includes a second chamber 14 connected to the chamber 13. The fourth liquid storage unit 35 for storing the reagent 88 containing the labeling substance LS is connected to the second chamber 14.

In step S18, the rotation mechanism 221 rotates the cartridge 100 to transfer the reagent containing the labeling substance LS that reacts with the amplification product of the nucleic acid from the fourth liquid storage unit 35 included in the cartridge 100 to the second chamber 14 included in the cartridge 100.

The sealing body 30a of the fourth liquid storage unit 35 is opened, for example, at the same timing as the timing when the nucleic acid contained in the dispersoid is amplified and then each of the sealing bodies 30a of the liquid storage unit 32 to the liquid storage unit 34 is opened. When the liquid storage unit 32 to the liquid storage unit 35 are opened at the same timing, only one operation of rotating the cartridge 100 for liquid feed is sufficient.

In step S18, after the rotation mechanism 221 transfers the reagent 88 containing the labeling substance LS to the second chamber 14, the transfer mechanism 240 applies a magnetic force to the cartridge 100, thereby transferring the nucleic acid bound to the denatured magnetic particle PM from the chamber 13 to the second chamber 14. As described above, the magnetic particle PM are moved to the second chamber 14 by the magnetic force and the rotation of the cartridge 100.

In step S18, the temperature regulating unit 260 adjusts the temperature of the second chamber 14 to react the amplification product of the nucleic acid taken from the droplet 84 by the demulsification with the labeling substance LS. Thus, the temperature regulating unit 260 performs hybridization processing by a temperature regulation, in addition to the thermal cycle processing of the PCR.

The temperature regulating unit 260 raises the temperature of the cartridge 100 at least by heating to react the nucleic acid in the second chamber 14 with the labeling substance LS. Thus, both processing for amplifying the nucleic acid and processing for labeling the nucleic acid can be performed in the cartridge 100 simply by heating at least the cartridge 100. In the second chamber 14, the labeling substance LS in the reagent 88 containing the labeling substance LS labels a target DNA by binding to the nucleic acid MD carried by the magnetic particle PM (see Fig. 15).

### <Secondary Washing>

The cartridge 100 includes a third chamber 15 connected to the second chamber 14. A fifth liquid storage unit 36 for storing the reagent 89 for washing the nucleic acid is connected to the third chamber 15. In step S19, the rotation mechanism 221 amplifies at least nucleic acid contained in the dispersoid, and then the rotation mechanism 221 rotates the cartridge 100 around the rotation axis 222, thereby transferring the reagent 89 for washing the nucleic acid from the fifth liquid storage unit 36 to the third chamber 15.

The sealing body 30a of the fifth liquid storage unit 36 is opened, for example, at the same timing as the timing when each of the sealing bodies 30a of the liquid storage unit 32 to the liquid storage unit 35 is opened. Thus, only one operation of rotating the cartridge 100 for liquid feed is sufficient.

In step S19, after the rotation mechanism 221 transfers the reagent 89 for washing the nucleic acid to the third chamber 15, the transfer mechanism 240 applies a magnetic force to the cartridge 100, thereby transferring the magnetic particle PM bound to the labeled nucleic acid from the second chamber 14 to the third chamber 15. As described above, the magnetic particle PM are moved to the third chamber 15 by the magnetic force and the rotation of the cartridge 100.

### <Detection>

The cartridge 100 includes a chamber 16 connected to the third chamber 15. The cartridge 100 includes a liquid storage unit 37 for storing a reagent 90 for dispersing nucleic acid, which is connected to the chamber 16. In the examples of Fig. 12 and Fig. 14, examples in which two liquid storage units 37 are provided are shown. As described above, a plurality of liquid storage units for storing the same reagent may be provided. Thereby, a required amount of liquid can be easily secured. The reagent 90 for dispersing nucleic acid is, for example, PBS (phosphate buffered saline).

In step S20, the rotation mechanism 221 amplifies at least nucleic acid contained in the dispersoid, and then the rotation mechanism 221 rotates the cartridge 100 around the rotation axis 222, thereby transferring the reagent 90 for dispersing nucleic acid from the liquid storage unit 37 to the chamber 16.

The sealing body 30a of the liquid storage unit 37 is opened, for example, at the same timing as the timing when each of the sealing bodies 30a of the liquid storage unit 32 to the liquid storage unit 36 is opened. Thus, only one operation of rotating the cartridge 100 for liquid feed is sufficient.

After the rotation mechanism 221 transfers the reagent 90 for dispersing nucleic acid to the chamber 16, the transfer mechanism 240 applies a magnetic force to the cartridge 100, thereby transferring the magnetic particle PM bound to the labeled nucleic acid from the third chamber 15 to the chamber 16. As described above, the magnetic particle PM are moved to the chamber 16 by the magnetic force by the transfer mechanism 240 and the rotation of the cartridge 100 by the rotation mechanism 221.

The detection unit 270 detects a signal based on the labeling substance LS bound to the nucleic acid MD taken from the droplets 84. Thus, an assay for detection of nucleic acid can be performed in the cartridge 100 by performing nucleic acid amplification in the droplet 84 contained in the emulsion 83, and then binding the labeling substance LS to the nucleic acid and the amplification product of the nucleic acid taken out from the droplet 84. As a result, it is possible to effectively suppress contamination in each step above.

The detection unit 270 images, for example, the chamber 16 of the cartridge 100, and the detection unit 270 counts the magnetic particle PM which are illuminated by the labeling substance LS by image processing. By filling the chamber 16 with a predetermined amount of PBS, the magnetic particle PM containing the labeled nucleic acid is sufficiently dispersed in the chamber 16.

With the configuration as described above, the nucleic acid detection device 200 according to the first embodiment performs the method for detecting nucleic acid shown in steps S13 to S20 of Fig. 13 using one cartridge 100.

### (Specific Configuration Example of Nucleic Acid Detection Device)

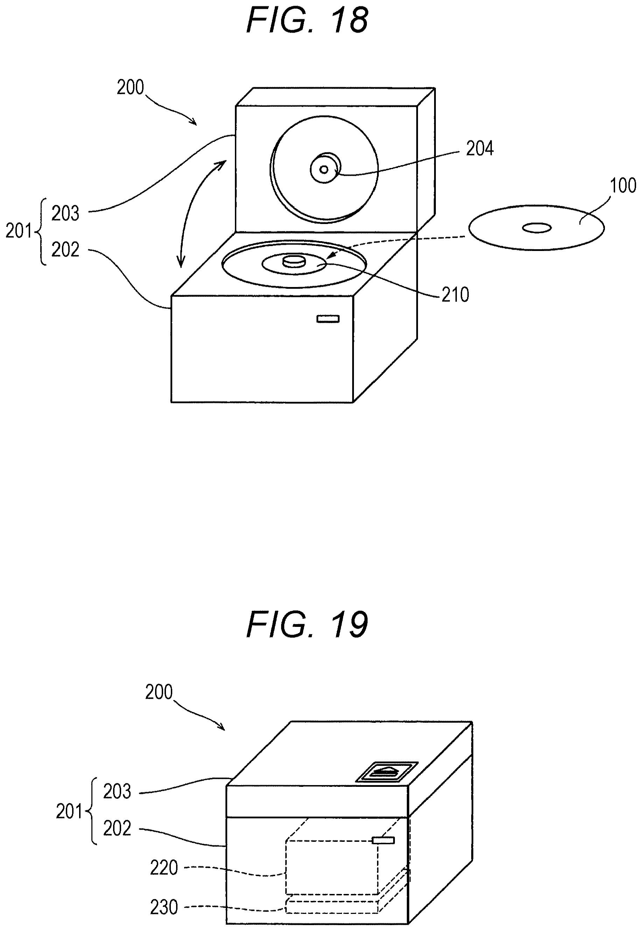

Next, with reference to Figs. 18 to 22, a specific configuration example of the nucleic acid detection device 200 for performing the above-described method for detecting nucleic acid using the cartridge 100 is shown. That is, the nucleic acid detection device 200 can perform processing of emulsion formation (step S13), emulsion PCR (step S14), emulsion breaking (step S15), primary washing (step S16), denaturation (step S17), hybridization (step S18), secondary washing (step S19), and detection processing (step S20) on the nucleic acid MD (DNA).

As shown in Figs. 18 and 19, the housing 201 of the nucleic acid detection device 200 includes a main body portion 202 and a lid portion 203. The lid portion 203 is provided to cover substantially the entire upper surface of the main body portion 202. In the upper surface of the main body portion 202, an installation unit 210 in which the cartridge 100 is arranged is provided. The lid portion 203 is pivoted up and down with respect to the main body portion 202. The lid portion 203 is openably and closably provided in a state where the installation unit 210 is opened as shown in Fig. 18 and a state where the installation unit 210 is covered as shown in Fig. 19. The sample processing unit 220 and a control unit 230 are stored in the main body portion 202.

### <Internal Structure of Nucleic Acid Detection Device>

As shown in Fig. 20, the installation unit 210 is configured as a support member that supports the cartridge 100 from below. The installation unit 210 is provided at the upper end part of the rotation axis 222 of the rotation mechanism 221. The installation unit 210 is, for example, a turntable. The installation unit 210 aligns the center of the cartridge 100 with the rotation axis 222 by fitting in the hole 51 (see Fig. 12) of the cartridge 100. The lid portion 203 is provided with a clamper 204. The clamper 204 rotatably supports a central portion of the upper surface of the cartridge 100 installed on the installation unit 210, with the lid portion 203 closed.

In the example of Fig. 20, the nucleic acid detection device 200 includes the sample processing unit 220 including the rotation mechanism 221, the transfer mechanism 240, and the opening mechanism 250.

The rotation mechanism 221 includes the rotation axis 222 and the drive unit 223 composed of an electric motor. The rotation mechanism 221 drives the drive unit 223 to rotate the cartridge 100 installed in the installation unit 210 around the rotation axis 222. The rotation mechanism 221 includes an encoder 224 for detecting the rotation angle of the drive unit 223, and an origin sensor 225 for detecting an origin position of the rotation angle. The cartridge 100 can be moved to an arbitrary rotational position by driving the drive unit 223 based on the detection angle of the encoder 224 with reference to the position detected by the origin sensor 225.

The rotation mechanism 221 is configured to perform at least a part of the processing of the biological sample by rotating the cartridge 100 around the rotation axis 222. That is, the rotation mechanism 221 performs processing such as formation of an emulsion, transfer of a sample, transfer of a reagent to each of the chambers 11 to 16 (see Fig. 14), stirring of the reagent and the sample, transfer of the magnetic particle PM in the circumferential direction between each of the chambers 11 to 16, and the like, inside the cartridge 100, by rotation.

The transfer mechanism 240 includes a magnet 241. The transfer mechanism 240 has a function of moving the magnetic particle PM inside the cartridge 100 in the radial direction by a magnet moving mechanism 242. The transfer mechanism 240 is arranged below the installation unit 210. The magnet moving mechanism 242 is configured by a combination of linear motion mechanisms. The magnet moving mechanism 242 is configured to move the magnet 241 in the radial direction. The magnet moving mechanism 242 is configured to move the magnet 241 in a direction toward or away from the cartridge 100. The magnetic particle PM in the cartridge 100 are collected by bringing the magnet 241 close, and collection of the magnetic particle PM is released by separating the magnet 241. The transfer mechanism 240 can move the magnetic particle PM in the cartridge 100 into and out of the chamber (see Fig. 14) by moving the magnet 241 in the radial direction in the state of magnetic collection. Thereby, the transfer mechanism 240 transfers the magnetic particle PM to the third chamber 12 connected to the chamber 11. Similarly, the transfer mechanism 240 transfers the magnetic particle PM to other chambers.

As described above, the reagent is enclosed in the cartridge 100 in advance, and the nucleic acid detection device 200 includes the opening mechanism 250 that opens a seal of the reagent in the cartridge 100. Thus, it is possible to enclose a reagent necessary for nucleic acid detection in advance in the cartridge 100, and use the reagent by opening the cartridge 100 by the opening mechanism 250. Therefore, for example, it is possible to effectively suppress contamination, as compared with a configuration in which a reagent to be used is externally injected into the cartridge 100 from outside.

The opening mechanism 250 includes a pin member 251 which can be advanced and retracted toward the cartridge 100 from above the cartridge 100 arranged in the installation unit 210. The opening mechanism 250 advances and retracts the pin member 251 by a driving source such as solenoid or motor. When the sealing body 30a (see Fig. 12) is arranged at a position immediately below the pin member 251 by the rotation mechanism 221, the opening mechanism 250 causes the pin member 251 to protrude and abut on the cartridge 100, and the opening mechanism 250 opens the sealing body 30a in the cartridge 100 by pressing the pin member 251. After opening, the opening mechanism 250 moves the pin member 251 away from the cartridge 100 to the retracted position not in contact.

The temperature regulating unit 260 is provided at a position immediately above the cartridge 100 arranged in the installation unit 210. The temperature regulating unit 260 is arranged on the inner surface of the lid portion 203 so as to face the cartridge 100. The planar shape of the temperature regulating unit 260 can adopt the configuration shown in Fig. 16 or 17. The temperature regulating unit 260 is configured by a heater or a Peltier element.

The temperature regulating unit 260 is configured to be movable relative to a position in contact with and a position away from the cartridge 100 arranged in the installation unit 210. In the example of Fig. 20, the temperature regulating unit 260 can be moved to a position in contact with the surface of the cartridge 100 and a position away from the surface of the cartridge 100 by the moving mechanism 261. The moving mechanism 261 includes a driving source such as, for example, solenoid or motor. The temperature regulating unit 260 changes the temperature of the cartridge 100 in contact with the cartridge 100. As a result, heat can be efficiently transferred by bringing the temperature regulating unit 260 into contact with the cartridge 100, as compared with the case where the cartridge 100 is heated in a non-contact manner, so that the temperature of the cartridge 100 can be easily changed.

The temperature regulating unit 260 may be fixedly provided, and the installation unit 210 may be configured to move the cartridge 100 in a direction toward or away from the temperature regulating unit 260. The nucleic acid detection device 200 includes a temperature sensor 262. The temperature sensor 262 detects the temperature of the cartridge 100, for example, by infrared light. Based on the detection result of the temperature sensor 262, the temperature regulating unit 260 is controlled.

The detection unit 270 includes an imaging unit for acquiring an image in the chamber 16 from outside of the cartridge 100. The detection unit 270 is arranged at a position facing the cartridge 100 arranged in the installation unit 210 via an opening formed in the upper surface of the main body portion 202. In Fig. 20, the detection unit 270 images the inside of the chamber 16 storing the nucleic acid MD, from below the cartridge 100. By performing image processing on a captured image, it is possible to identify individual magnetic particle PM in the chamber 16 and acquire fluorescent color caused by the labeling substance LS bound to the magnetic particle PM. The detection unit 270 is arranged at a position overlapping the rotation track of the chamber 16 that performs detection. That is, a radial distance from the rotation axis 222 of the chamber 16 that performs detection and a radial distance from the rotation axis 222 of the detection unit 270 substantially match, and the chamber 16 can be arranged in the imaging field of the detection unit 270 by the rotation of the cartridge 100. Thus, while the nucleic acid MD is stored in the cartridge 100, imaging can be performed from outside of the cartridge 100 to detect the nucleic acid MD. Therefore, since there is no need to transfer a liquid containing nucleic acid to outside of the cartridge 100 for detection, it is possible to further effectively suppress sample mix-up and contamination. Since production of the emulsion 83, amplification of nucleic acid, and detection of nucleic acid can be performed in the cartridge 100, processing efficiency can be improved as compared with the case of transferring the nucleic acid to another device.

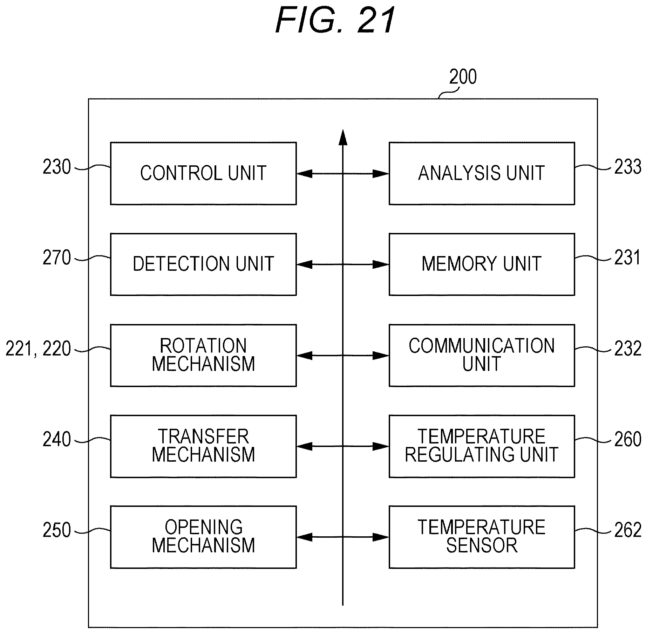

Fig. 21 shows a control configuration of the nucleic acid detection device 200.

The nucleic acid detection device 200 includes a control unit 230. The control unit 230 includes, for example, a processor and a memory. The processor is configured of, for example, a CPU, an MPU, an FPGA, and the like. The memory is configured of, for example, a ROM, a RAM, and the like. The control unit 230 receives a signal from each unit of the nucleic acid detection device 200. The control unit 230 controls each unit of the nucleic acid detection device 200.

The nucleic acid detection device 200 includes a memory unit 231. The memory unit 231 memorizes a program for causing the processor to function as the control unit 230 of the nucleic acid detection device 200. The memory unit 231 is configured of, for example, a flash memory, a hard disk, and the like.

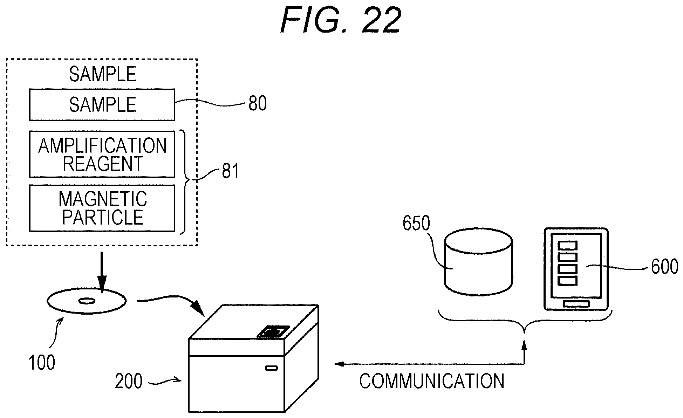

The nucleic acid detection device 200 includes a communication unit 232. The communication unit 232 can transmit information to external equipment. The communication unit 232 can receive information from the external equipment. The communication unit 232 includes, for example, a communication module, an interface for external connection, and the like. The communication unit 232 can communicate with a terminal 600 (see Fig. 22) capable of communicating with the nucleic acid detection device 200 by wired or wireless communication. The communication unit 232 can communicate with a server 650 (see Fig. 22) via a network. The nucleic acid detection device 200 is configured so as to perform various operations and display various pieces of information by using the external terminal 600. The terminal 600 includes, for example, a tablet type terminal, a portable information terminal such as a smartphone, and an information terminal such as a PC (personal computer).

The control unit 230 controls each unit of the nucleic acid detection device 200 to perform the method for detecting nucleic acid of the first embodiment.

The nucleic acid detection device 200 includes an analysis unit 233 for outputting whether or not the nucleic acid to be detected is present, based on captured image information of the emulsion 83 in the chamber 16. The analysis unit 233 identifies the magnetic particle PM in the chamber 16 one by one by performing image processing on the image captured by the detection unit 270. Then, the analysis unit 233 acquires fluorescent color caused by the labeling substance LS bound to the individual magnetic particle PM. For example, in Fig. 15, a mutant nucleic acid to be detected produces fluorescence of a first fluorescent color, and a wild type nucleic acid produces fluorescence of a second fluorescent color different from the first fluorescent color. Whether or not the nucleic acid MD bound to the individual magnetic particle PM is the nucleic acid to be detected (mutant nucleic acid) is determined by the fluorescent color. Thus, unlike the case where the presence or absence of nucleic acid to be detected is analyzed by an external analyzer, for example, the nucleic acid detection device alone can analyze the presence or absence of the nucleic acid to be detected and can output it to a user.

The analysis unit 233 is configured as a functional block constructed by the processor executing a program for analysis. In that case, the control unit 230 and the analysis unit 233 can be configured by the same hardware. The analysis unit 233 may be configured by a dedicated processor that is separate from the control unit 230.

The nucleic acid detection device 200 may not include the analysis unit 233. In that case, the nucleic acid detection device 200 transmits, for example, a captured image as a detection result of the detection unit 270 to the external terminal 600 or the server 650. Then, in the terminal 600 or the server 650, whether or not the nucleic acid to be detected is present may be analyzed, based on the captured image information of the emulsion 83 in the chamber 16.

### <Operation of Nucleic Acid Detection Device>

Next, a processing operation of the nucleic acid detection device 200 will be described with reference to Figs. 12 to 20. The processing operation of the nucleic acid detection device 200 is controlled by the control unit 230. As preparation work, dispersoids of the sample 80 and the reagent 81 after step S11 and step S12 of Fig. 13 are performed are injected into the cartridge 100 by the operator. Then, the cartridge 100 is set in the installation unit 210 (see Fig. 18) of the nucleic acid detection device 200, and the lid portion 203 is closed (see Fig. 19). In this state, the processing operation is started.

In step S13, the control unit 230 controls the rotation mechanism 221 so as to transfer the dispersoid stored in the first liquid storage unit 38 included in the cartridge 100 to the chamber 11 by rotation. The control unit 230 controls the opening mechanism 250 so as to open the sealing body 30a of the second liquid storage unit 31. After opening, the control unit 230 controls the rotation mechanism 221 so as to transfer the dispersion medium 82 stored in the second liquid storage unit 31 included in the cartridge 100 to the chamber 11 by rotation. The dispersoid and the dispersion medium 82 are transferred into the chamber 11 by centrifugal force.

The control unit 230 controls the rotation mechanism 221 so as to repeat the operation of changing rotational speed of the cartridge 100, thereby forming droplets 84 of the dispersoid containing the sample 80 and the reagent 81 in the dispersion medium 82 in the chamber 11 to form an emulsion 83.

For example, when forming the emulsion 83, the control unit 230 controls the rotation mechanism 221 so as to repeat an operation of reversing the direction of rotation of the cartridge 100 with a period of 165 milliseconds or more and 330 milliseconds or less. As a result, the emulsion 83 suitable for processing and detecting the nucleic acid MD can be produced only by repeating the operation of reversing the direction of rotation with a period of 165 milliseconds or more and 330 milliseconds or less, based on the results of experiments described later.

In step S14, the control unit 230 controls the temperature regulating unit 260 so as to change the temperature of the cartridge 100 in the range of 30°C or more and 90°C or less. Thereby, emulsion PCR processing is performed.

After forming the emulsion 83 in the chamber 11 and changing the temperature of the cartridge 100 to react the nucleic acid MD with the reagent 81, the control unit 230 controls the opening mechanism 250 so as to open the sealing body 30a of the third liquid storage unit 32. Similarly, the control unit 230 controls the opening mechanism 250 so as to open the sealing body 30a of each of the liquid storage unit 33 to the liquid storage unit 37.

In step S15, the control unit 230 controls the rotation mechanism 221 so as to transfer the reagent 85 for emulsifying the emulsion from the third liquid storage unit 32 included in the cartridge 100 to the chamber 11 in which the emulsion 83 is formed by rotation. The emulsion 83 in the chamber 11 is demulsified by the reagent 85 for demulsifying the emulsion.

At this time, when the sealing bodies 30a of the liquid storage unit 33 to the liquid storage unit 37 are also opened, each reagent stored in the liquid storage units 30 thereof can also be fed to the corresponding chamber in one liquid transfer operation.

In step S16, after the emulsion 83 is demulsified, the control unit 230 controls the transfer mechanism 240 so as to transfer the nucleic acid MD from the chamber 11 to the third chamber 12. That is, the control unit 230 moves the magnetic particle PM in the radial direction by the magnetic force of the transfer mechanism 240, and the control unit 230 also moves the cartridge 100 in the circumferential direction with respect to the magnetic particle PM by the rotation of the rotation mechanism 221. The combination of the magnetic force and the rotation of the cartridge 100 moves the magnetic particle PM bound to the nucleic acid MD to the third chamber 12 via the passage 40. The control unit 230 washes the magnetic particle PM with the reagent 86 for washing the nucleic acid.

In step S17, the control unit 230 controls the transfer mechanism 240 so as to transfer the nucleic acid MD stored in the third chamber 12 to the chamber 13. In the chamber 13, the reagent 87 for washing the nucleic acid is transferred from the liquid storage unit 34 by rotation. As a result, in the chamber 13, the nucleic acid (one or more double-stranded DNAs) bound to the magnetic particle PM are denatured into single strands.

In step S18, the control unit 230 controls the transfer mechanism 240 so as to transfer the nucleic acid MD stored in the chamber 13 to the second chamber 14. The reagent 88 containing the labeling substance LS that reacts with the amplification product of the nucleic acid is transferred to the second chamber 14 from the fourth liquid storage unit 35 by the rotation of the cartridge 100.

The control unit 230 controls the temperature regulating unit 260 to raise the temperature of the cartridge 100 to react the nucleic acid in the second chamber 14 with the labeling substance LS. As a result, the control unit 230 performs labeling processing on the amplification product of the nucleic acid in the second chamber 14.

In step S19, the control unit 230 controls the transfer mechanism 240 so as to transfer the nucleic acid MD stored in the second chamber 14 to the third chamber 15. The reagent 89 for washing the nucleic acid is transferred to the third chamber 15 from the fifth liquid storage unit 36 by the rotation of the cartridge 100. As a result, the control unit 230 washes the magnetic particle PM after the labeling processing in the third chamber 15.

In step S20, the control unit 230 controls the transfer mechanism 240 so as to transfer the nucleic acid MD stored in the third chamber 15 to the chamber 16. The reagent 90 (PBS) for dispersing nucleic acid is transferred from the liquid storage unit 37 to the chamber 16 by the rotation of the cartridge 100. As a result, the control unit 230 disperses the magnetic particle PM after the labeling processing in the reagent in the chamber 16.

The control unit 230 controls the rotation mechanism 221 to arrange the chamber 16 in the imaging field of the detection unit 270. The control unit 230 causes the detection unit 270 to acquire an image in the chamber 16. The analysis unit 233 counts the number of the individual magnetic particle PM based on the acquired image. The analysis unit 233 acquires fluorescent color and fluorescence intensity of each of the magnetic particle PM. The analysis unit 233 creates a scattergram of a normal (wild type) nucleic acid and a mutant nucleic acid, for example, from the acquired fluorescent color and fluorescence intensity of the magnetic particle PM.

### EXAMPLES

Next, experiments (examples) conducted by the inventors of the present application for emulsion production according to the first embodiment will be described. The inventors of the present invention conducted experiments under various conditions with respect to a period of changing rotational speed of the cartridge 100, an amount of liquid (the dispersoid and the dispersion medium 82) stored in the chamber 11, a rotational direction, and a rotational speed. Then, the obtained experimental result was compared with that of comparative example. Each of the experiments will be described below.

As a sample, a nucleic acid solution in which 1% of mutant DNA was mixed with wild type DNA was prepared, which was commonly used in examples and comparative example.

### (Comparative Example)

In the comparative example, the inventors of the present application measured samples subjected to processing of steps S13 to S19 in Fig. 13 by hand methods, using a flow cytometer. An emulsion of a dispersoid containing a sample (nucleic acid solution) and a nucleic acid amplification reagent and a dispersion medium was produced, and PCR was performed. A labeling substance that specifically binds to each of the wild type and the mutant type was hybridized to each DNA extracted by emulsion breaking treatment, and then detected by a flow cytometer.

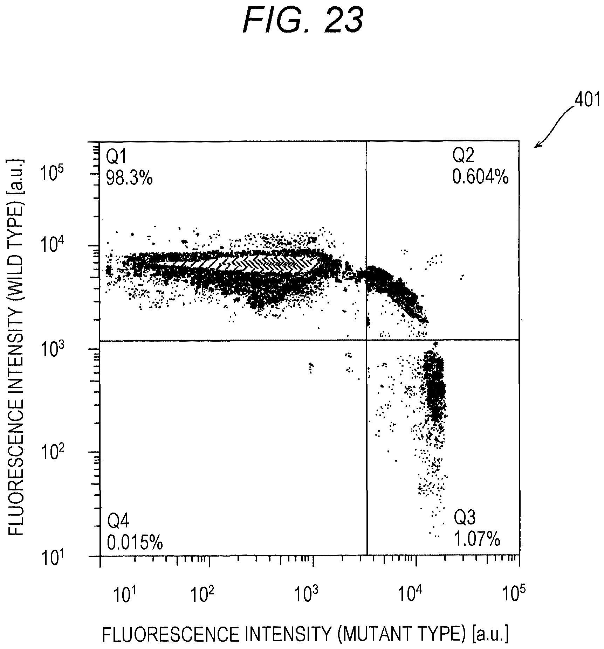

Fig. 23 shows a scattergram 401 according to the comparative example. In the scattergram, a vertical axis represents fluorescence intensity indicating wild type DNA, and a horizontal axis represents fluorescence intensity indicating mutant DNA. A plot included in area Q1 divided by two straight lines respectively parallel to the vertical axis and the horizontal axis represents wild type DNA, and a plot contained in area Q3 represents mutant DNA. The wild type DNA was 98.3% and the mutant DNA was 1.07% with respect to the total number of plots included in areas Q1 to Q4.

### (Example 1: Change Period of Rotational Speed)

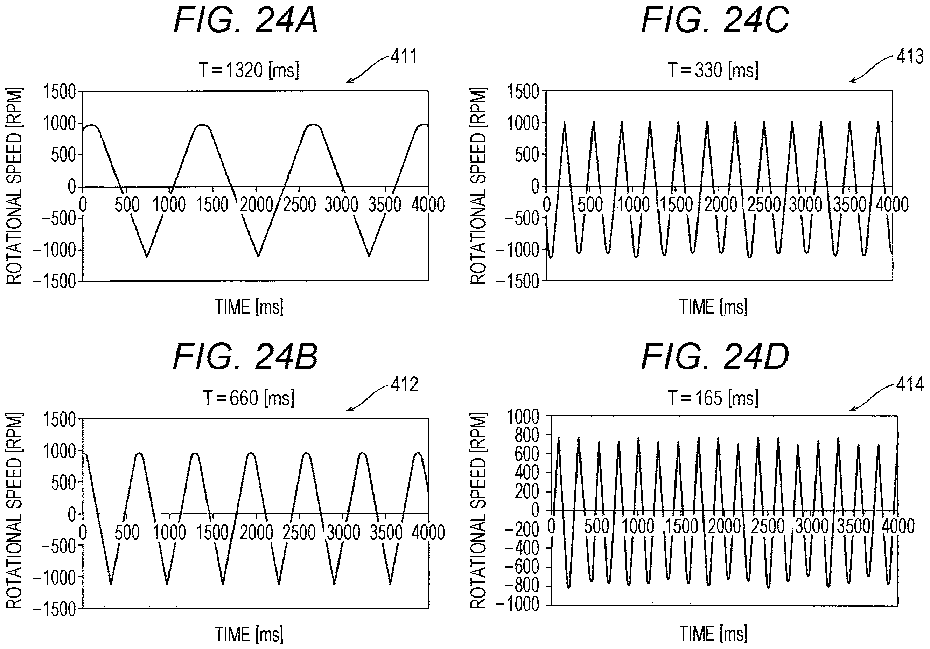

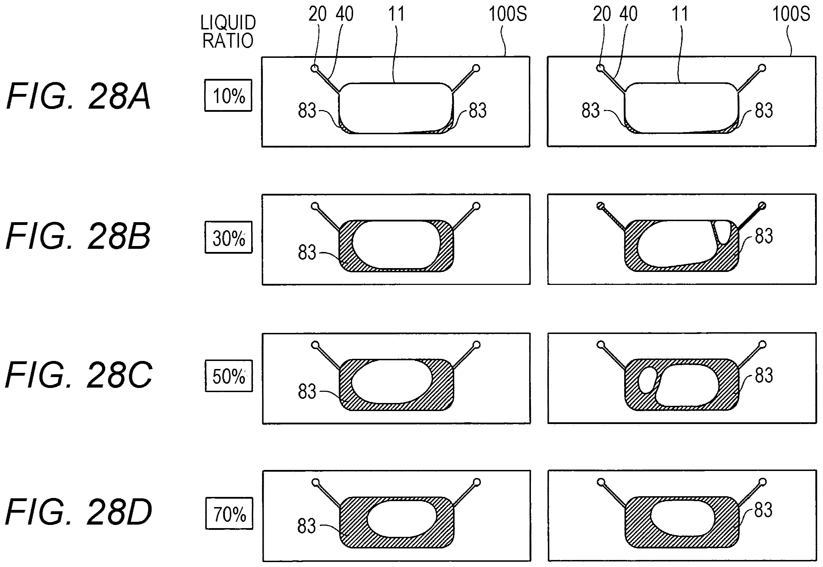

A test cartridge 100S shown in Fig. 28 was produced, and emulsions were produced under four conditions in which reversing period was differentiated, under a condition that an operation of reversing a direction of rotation of the cartridge 100S was repeated. Change period T of the rotational speed was set to four conditions of T = 1320 milliseconds, 660 milliseconds, 330 milliseconds, and 165 milliseconds. Change graphs 411 to 414 of the rotational speed in each period T are shown in Fig. 24.

Volume of the chamber 11 used is 284.62 µL. 90 µL of the dispersoid containing a sample (nucleic acid solution) and a nucleic acid amplification reagent and the dispersion medium were injected into the chamber 11. Ratio of the total volume of a sample 80, a reagent 81 and a dispersion medium 82 in the chamber 11 is about 30%. An operation of changing rotational speed of the cartridge 100S at each change period T was repeated for one minute.

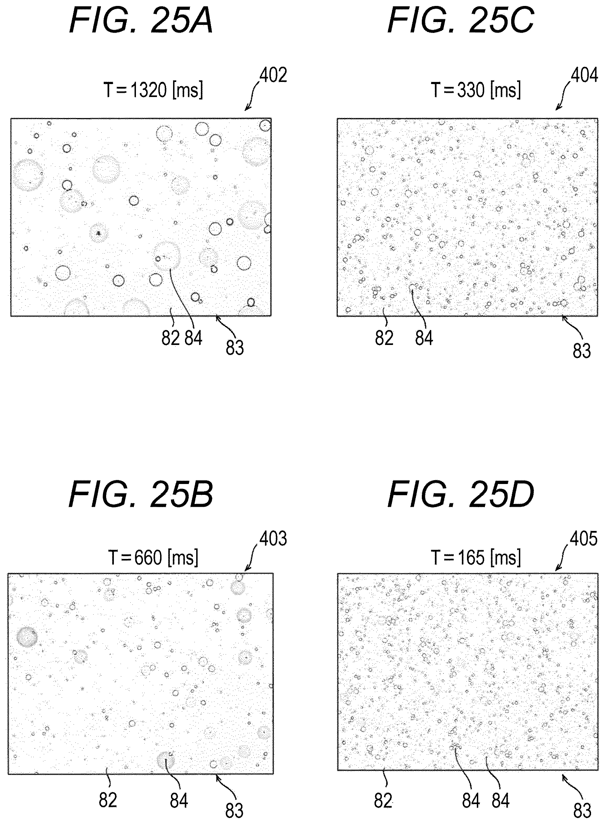

Fig. 25 shows microscope images 402 to 405 of the emulsion 83 produced in each period T of Example 1. From the microscope images, it was confirmed that the emulsion 83 in which the droplets 84 of the dispersoid were dispersed was produced in the dispersion medium 82 in any period T. As can be seen from the images, it can be seen that the smaller the period T, the smaller the droplet size (diameter in the images) tends to be.

Twenty-five microscope images 402 to 405 were acquired for each period T, and droplets 84 included in the 25 microscope images were counted by image processing. The total number of droplets in each period T is as follows.

| | |
|---|---|
| T = 1320 milliseconds | Number of droplets: 410 |
| T = 660 milliseconds | Number of droplets: 766 |
| T = 330 milliseconds | Number of droplets: 2440 |
| T = 165 milliseconds | Number of droplets: 2726 |

From the above results, in Example 1, it was first confirmed that the emulsion 83 can be formed even when the period T is changed. Moreover, it was confirmed that the size and number of droplets 84 contained in the emulsion 83 can be controlled by changing the period T. In order to form an emulsion 83 containing droplets 84 of a dispersoid storing one molecule or one target DNA, the number of droplets 84 so that the majority of the droplets 84 contains zero or one DNA is statistically determined, with respect to the number of DNA contained in the sample 80. In other words, when it is possible to produce an emulsion 83 containing a statistically determined number of droplets 84 with respect to the number of DNA contained in the sample 80, digital processing of the target DNA that has been subjected to limiting dilution for individual droplets 84 (i.e., PCR processing for each DNA contained in the sample) becomes possible. According to Example 1, it was suggested that digital processing of the target DNA according to the concentration of DNA contained in the sample becomes possible, by appropriately setting the change period T of the rotational speed of the cartridge 100S.

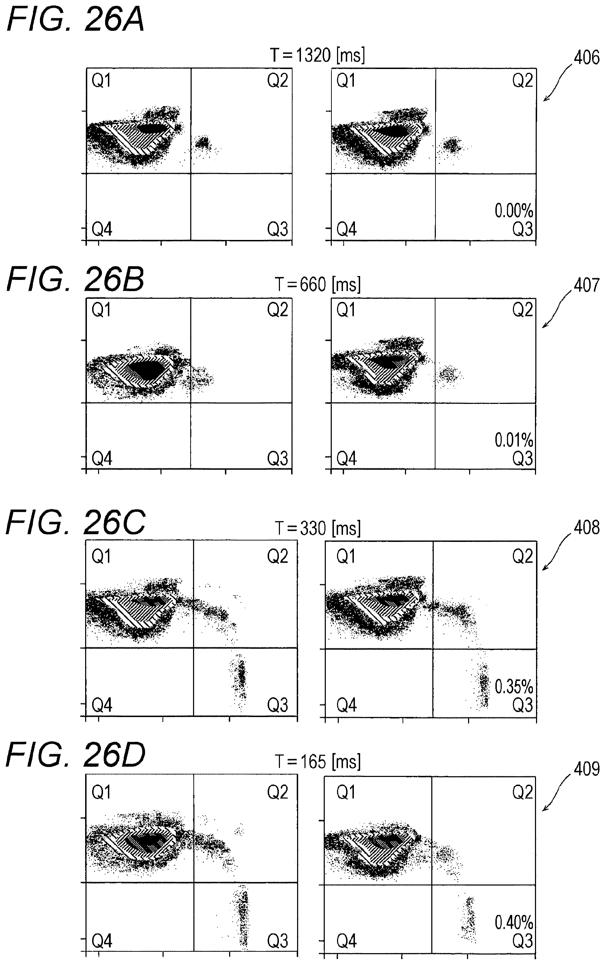

Next, PCR processing was performed on the emulsion 83 produced in each period T, and a labeling substance that specifically binds to each of the wild type and the mutant type was hybridized to each DNA extracted by the emulsion breaking treatment, and then detected by a flow cytometer. Then, scattergrams similar to the comparative example (see Fig. 23) were created. Fig. 26 shows scattergrams 406 to 409 when processed under the conditions of each period T. The experiment was performed a plurality of times, and two each of scattergrams 406 to 409 are shown.

Assuming that the number of mutant DNAs detected in the comparative example was Mr and the number of mutant DNAs detected in Example 1 was Mt, a deviation rate from the comparative example was determined as (Mr - Mt)/Mr. The deviation rate was as follows. Focusing on the deviation rate of the number of detected mutant DNAs, it was confirmed that the condition of period T = 330 milliseconds (deviation rate: 16%) is most preferable, and the condition of period T = 165 milliseconds (deviation rate: 28%) is secondly preferable, for the DNA concentration of the sample used in this experiment. As a result, it was confirmed that an emulsion more suitable for DNA detection can be produced by appropriately setting the period T for changing the rotational speed of the cartridge 100S with respect to the DNA concentration of the sample. In particular, in the period of 165 milliseconds or more and 330 milliseconds or less, the number of droplets 84 formed increases, and it is possible to increase the DNA concentration of the sample accordingly. As a result, it is preferable because more DNAs can be efficiently processed and detected in one processing.

### (Example 2)

Emulsions were produced under four conditions in which ratio Rv of the total volume of the sample 80, the reagent 81 and the dispersion medium 82 in the chamber 11 of the cartridge 100S was differentiated. The volume of the chamber 11 is 284.62 µL. The ratio Rv of the total volume was set to four conditions of 10%, 30%, 50%, and 70%. Under any conditions, the volume ratio of the dispersoid containing a sample (nucleic acid solution) and a nucleic acid amplification reagent to the dispersion medium was maintained at dispersoid : dispersion medium = 2:7, and the liquid amount was differentiated. In the emulsion production, the change period T of the rotational speed was set to 330 milliseconds under the condition that the operation of reversing the direction of rotation of the cartridge 100S was repeated. The operation of changing the rotational speed of the cartridge 100S at period T was repeated for one minute.

Fig. 28 shows schematic diagrams of conditions in the chamber 11 after the emulsion production. The experiment was performed a plurality of times, and two each of schematic diagrams under each condition are shown. A portion not hatched in the chamber 11 is a region occupied by air. It was confirmed that the emulsion 83 indicated by hatching was formed in any ratio Rv of the total volume.

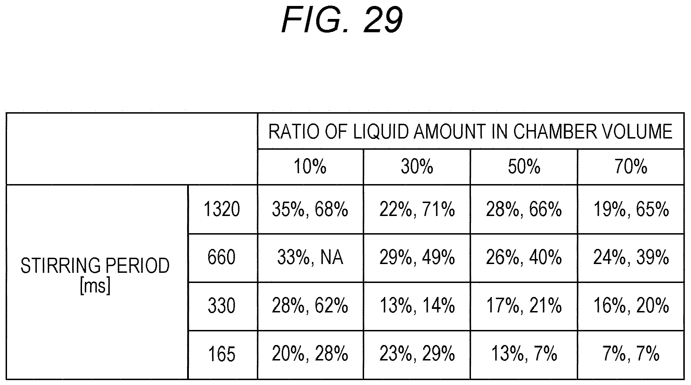

Next, the ratio Rv of the total volume was set to four conditions of 10%, 30%, 50%, and 70%, and for each ratio Rv, a scattergram of DNA detected by performing the same processing as in Example 1 was created when the emulsions 83 were produced under four conditions of the change period T of the rotational speed of T = 1320 milliseconds, 660 milliseconds, 330 milliseconds, and 165 milliseconds. Then, deviation rates of the number of detected wild type DNAs with respect to the comparative example and deviation rates of the number of detected mutant DNAs with respect to the comparative example, in each of the obtained scattergrams, were respectively calculated. The calculation results of the deviation rates are shown in Fig. 29.

From the results shown in Fig. 29, it was confirmed that a detection accuracy equivalent to that of the comparative example can be obtained by an appropriate combination of ratio Rv and period T. That is, with ratio Rv of the total volume = 30% and change period T of the rotational speed = 330 milliseconds, the deviation rates were about 13% in all cases, and a result equivalent to the hand method was obtained. With ratio Rv of the total volume = 50% or 70% and change period T of the rotational speed = 165 milliseconds, the deviation rates were about 7% in all cases, and a result equivalent to the hand method was obtained. Variation in the result for each operation in the hand method is about 10% to 20%, and the result under the above preferable conditions may be considered to be equivalent to the result according to the hand method.

As the results of Example 1 and Example 2 above, the operation of changing the rotational speed of the cartridge 100S preferably includes an operation of reversing the direction of rotation of the cartridge 100S in a period of 165 milliseconds or more and 330 milliseconds or less. As a result, the emulsion 83 suitable for processing and detecting the nucleic acid MD can be produced only by repeating the operation of reversing the direction of rotation with a period of 165 milliseconds or more and 330 milliseconds or less.

Ratio of the total volume of the sample 80, the reagent 81 and the dispersion medium 82 in the chamber 11 is preferably 30% or more and 70% or less. Thereby, the emulsion 83 suitable for processing and detecting the nucleic acid MD can be produced.

It is more preferable that the operation of changing the rotational speed of the cartridge 100S includes the operation of reversing the direction of rotation of the cartridge 100S in a period of 330 milliseconds, and the ratio of the total volume of the sample 80, the reagent 81 and the dispersion medium 82 in the chamber 11 is 30%. Thereby, the emulsion 83 suitable for processing and detecting the nucleic acid MD can be further produced.

Similarly, it is preferable that the operation of changing the rotational speed of the cartridge 100S includes the operation of reversing the direction of rotation of the cartridge 100S in a period of 165 milliseconds, and the ratio of the total volume of the sample 80, the reagent 81 and the dispersion medium 82 in the chamber 11 is 50% or more and 70% or less. Thereby, the emulsion 83 suitable for processing and detecting the nucleic acid MD can be further produced.

### (Example 3)

In Example 3, it was verified whether or not the production of the emulsion 83 and the detection of DNA were possible when the operation of accelerating and decelerating in the same direction was repeated, as the operation of changing the rotational speed of the cartridge 100S.

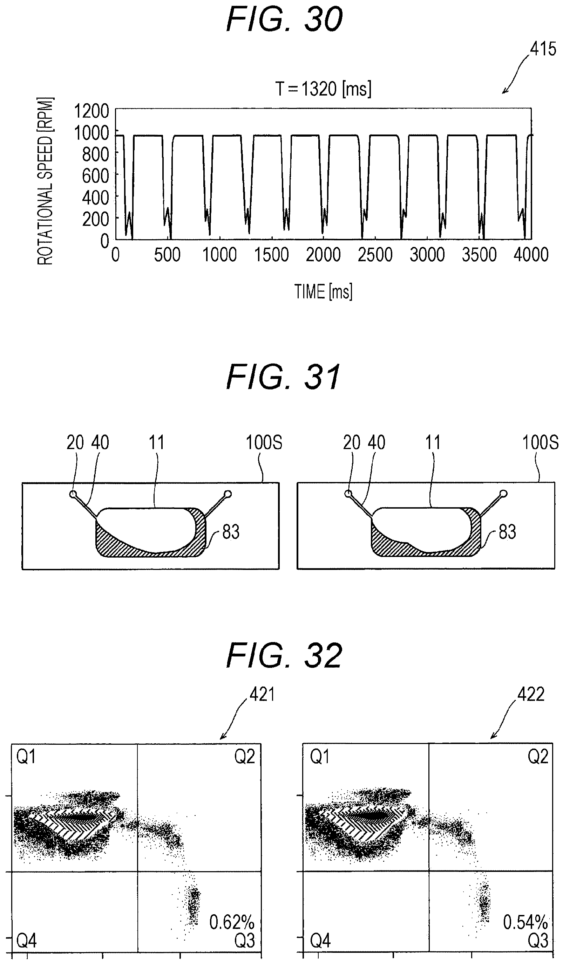

That is, as shown in Fig. 4B, the emulsion was produced under the condition that the operation of accelerating and decelerating the cartridge 100S in the same direction was repeated. The change period T of the rotational speed was set to T = 380 milliseconds. Change graph 415 of the rotational speed in Example 3 is shown in Fig. 30. Other experimental conditions are the same as those in Example 1 above.

Fig. 31 shows schematic diagrams of conditions in the chamber 11 after the emulsion production. The experiment was performed a plurality of times, and two schematic diagrams under each condition are shown. It was confirmed from Fig. 31 that the emulsion 83 indicated by hatching was formed. In Example 3, it was confirmed that the emulsion 83 can be produced even when the operation of accelerating and decelerating the cartridge 100S in the same direction is repeated.

Next, Fig. 32 shows scattergrams 421 and 422 when processed under the conditions of Example 3. In each scattergram, in area Q3 representing mutant DNA, the number of detected mutant DNAs was 0.62% (first time) and 0.54% (second time) with respect to the total number of plots included in areas Q1 to Q4. Thus, it was confirmed that nucleic acid can be detected using the emulsion 83 produced under the condition that the operation of accelerating and decelerating the cartridge 100S in the same direction was repeated.

### (Example 4: Rotational Speed)

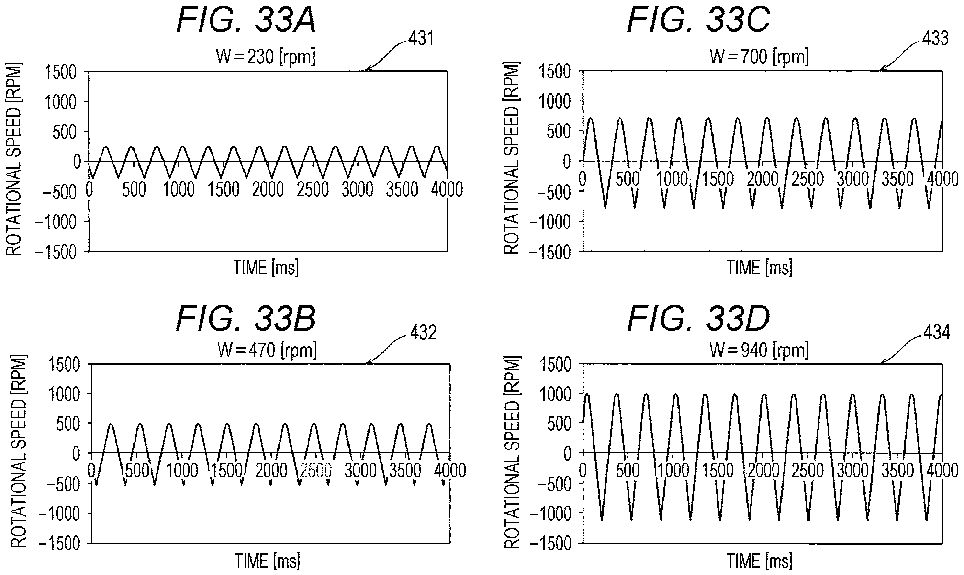

Emulsions were produced under four conditions in which the maximum value of the rotational speed was differentiated, under the condition that the operation of reversing the direction of rotation of the cartridge 100S was repeated. The rotational speed W was set to four conditions of W = 230 rpm, 470 rpm, 700 rpm, and 940 rpm. The change period T of the rotational speed was set to T = 330 milliseconds. Change graphs 431 to 434 of the rotational speed at each speed condition are shown in Fig. 33. The ratio Rv of the total volume of the sample 80, the reagent 81 and the dispersion medium 82 in the chamber 11 was set to 30%, and the operation of changing the rotational speed of the cartridge 100S at each speed condition was repeated for 1 minute. It was confirmed that an emulsion was formed at any speed W.

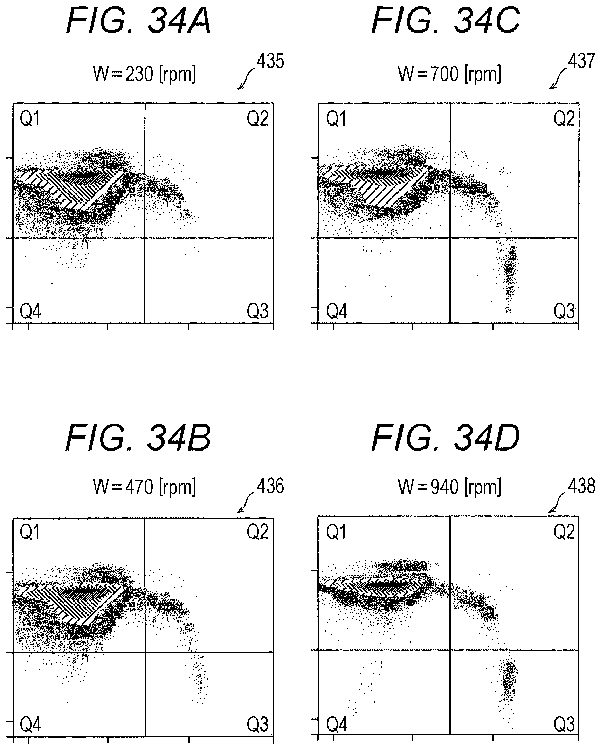

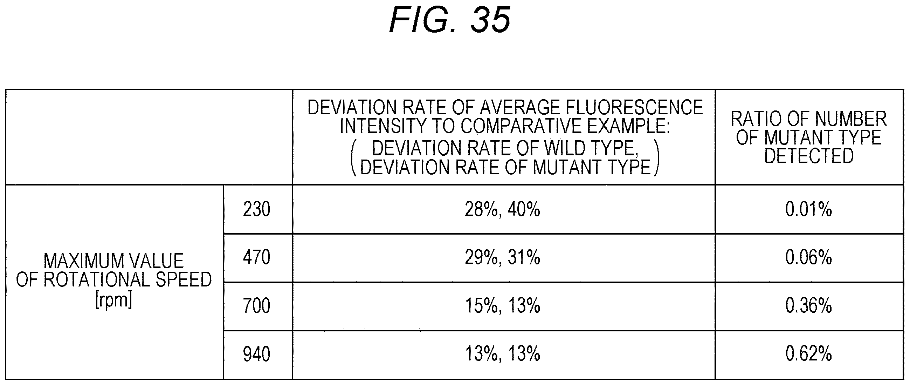

Next, PCR processing was performed on the emulsion 83 produced at each speed W, and a labeling substance that specifically binds to each of the wild type and the mutant type was hybridized to each DNA extracted by the emulsion breaking treatment, and then detected by a flow cytometer. Then, as shown in Fig. 34, scattergrams 435 to 438 were created. Ratios of the number of detected mutant DNAs in each of scattergrams 435 to 438 and deviation rates with respect to the comparative example are shown in Fig. 35.

From the results of Fig. 35, it was confirmed that the condition of speed W = 940 rpm is most preferable and the condition of speed W = 700 rpm is secondly preferable, for the DNA concentration of the sample used in this experiment. It was confirmed that an emulsion more suitable for DNA detection can be produced by appropriately setting the rotational speed W of the cartridge 100S with respect to the DNA concentration of the sample.

### [Second Embodiment]

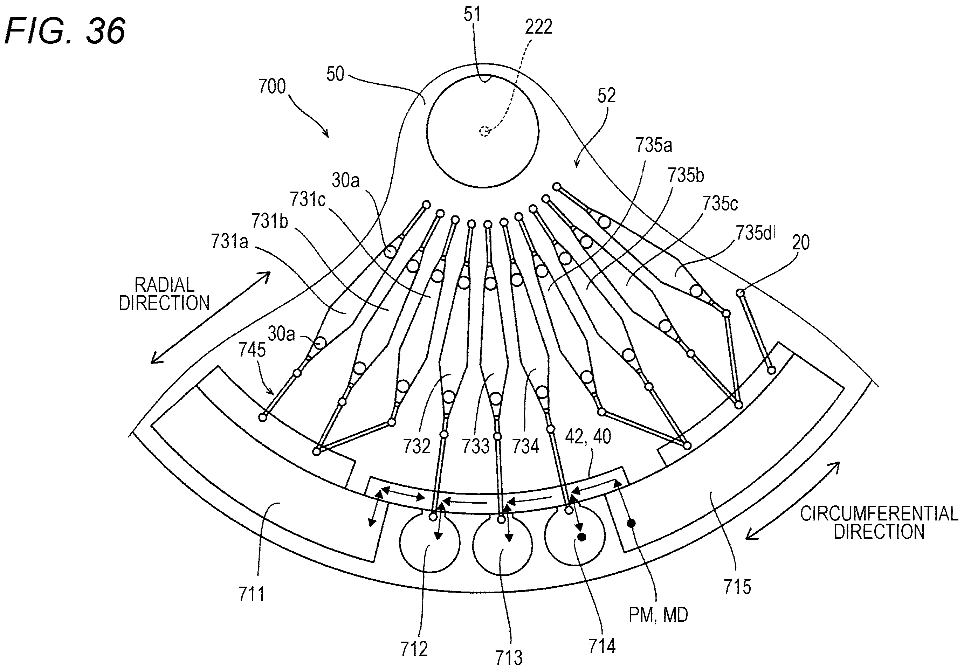

Fig. 36 shows a cartridge 700 according to the second embodiment. The cartridge 700 includes five chambers 711 to 715 and ten liquid storage units.

The chambers 711 to 715 are arranged in the circumferential direction. Three liquid storage units 731a, 731b and 731c are connected to the chamber 711, respectively. One liquid storage unit 732 is connected to the chamber 712. One liquid storage unit 733 is connected to the chamber 713. One liquid storage unit 734 is connected to the chamber 714. Four liquid storage units 735a, 735b, 735c, and 735d are connected to the chamber 715, respectively. A sample introduction unit 20 is connected to the chamber 715. The five chambers 711 to 715 are connected via the circumferential area 42 of the passage 40. The cartridge 700 includes the chamber 711 for producing the emulsion 83, the third chambers 712 to 714, and the fourth chamber 715. The chamber 711 is also a second chamber to which the reagent 88 containing the labeling substance LS is transferred.

All ten liquid storage units 731a to 731c, 732 to 734, and 735a to 735d communicate with outside of the cartridge 700 on a radially inner side. All ten liquid storage units 731a to 731c, 732 to 734, and 735a to 735d are connected to a flow path 745 communicating with a corresponding chamber on a radially outer side. The sealing bodies 30a are provided at connection parts with outside of each liquid storage unit and connection parts with each flow path 745, which can be opened by the opening mechanism 250. Other configuration of the cartridge 700 is the same as that of the cartridge 100, so the description will be omitted.

The nucleic acid detection device 200 using the cartridge 700 is equivalent to the device configuration of the first embodiment shown in Fig. 20. On the other hand, while the extract from which the nucleic acid MD was extracted was introduced as the sample 80 into the cartridge 100 in the first embodiment, a biological sample before the nucleic acid MD is extracted is introduced as a sample 80 into the cartridge 700 of the second embodiment. Then, the nucleic acid detection device 200 performs an extraction processing of the nucleic acid MD from the sample 80 introduced into the cartridge 700 inside the cartridge 700.

That is, in the second embodiment, the sample processing unit 220 extracts nucleic acid from the sample 80 in the cartridge 700, and the sample processing unit 220 forms the emulsion 83 by a dispersoid containing the extracted nucleic acid. Thereby, a step of extracting nucleic acid can be performed in the cartridge 700 as well as the step of producing the emulsion 83 and the nucleic acid amplification step. Thus, it is possible to effectively suppress sample mix-up and contamination, as compared with the case where the step of extracting nucleic acid is performed outside the cartridge 700.

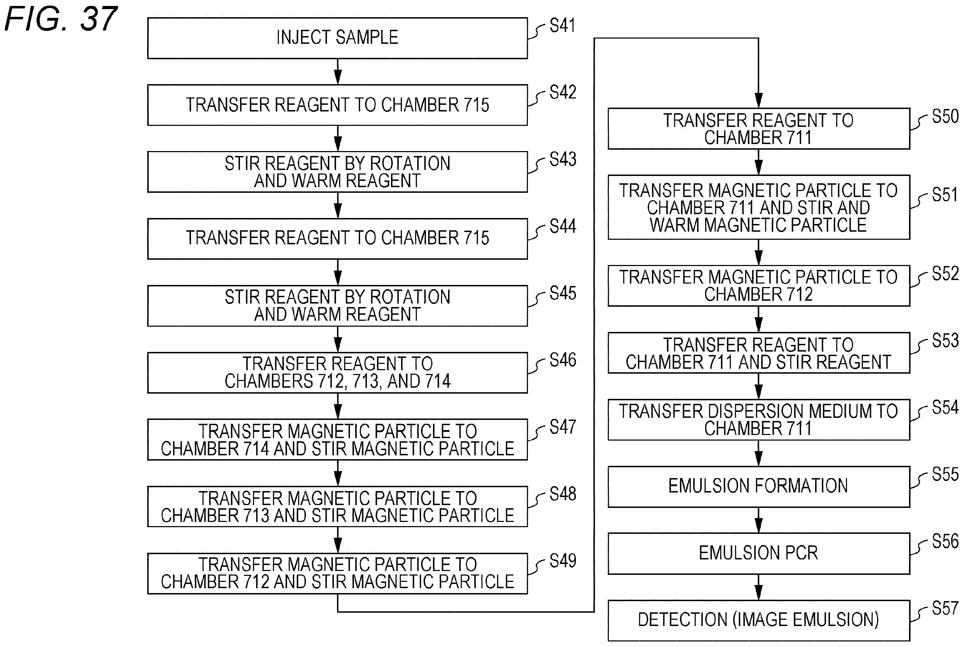

Fig. 37 shows a flow of processing using the cartridge 700. The processing using the cartridge 700 includes steps S41 to S57.

In step S41, the sample 80 containing the nucleic acid MD is injected from the sample introduction unit 20. The sample 80 is, for example, a blood sample such as whole blood, plasma or serum. Then, a rotation mechanism 221 transfers the sample 80 to the fourth chamber 715 by rotating the cartridge 700.

In step S42, the reagent is transferred to the fourth chamber 715. First, a reagent for extracting nucleic acid is stored in advance in the seventh liquid storage units 735a to 735d. Specifically, an enzyme reagent is stored in the seventh liquid storage unit 735a. A solubilizing reagent is stored in the seventh liquid storage unit 735b. A nucleic acid extraction reagent is stored in the seventh liquid storage unit 735c. A magnetic particle reagent is stored in the seventh liquid storage unit 735d.

The enzyme reagent contains a proteolytic enzyme that degrades a protein contained in the sample 80. The proteolytic enzyme allows efficient extraction of nucleic acid from cells in the sample 80. The proteolytic enzyme is, for example, proteinase K. The solubilizing reagent contains a chaotropic compound such as a guanidine compound, which acts to lyse cells and bind nucleic acid to a carrier. The nucleic acid extraction reagent mainly contains an alcohol component, which acts to make the nucleic acid preferentially adsorbed to the magnetic particle PM by making the nucleic acid solution from which the nucleic acid was extracted into a hydrophobic environment. The magnetic particle reagent is a liquid containing magnetic particle PM coated with a carrier that adsorbs nucleic acid, and the carrier is, for example, silica. The nucleic acid is preferentially adsorbed to a silica carrier of the magnetic particle PM in the presence environment of the solubilizing reagent and the nucleic acid extraction reagent.

In step S42, the sealing bodies 30a of the two seventh liquid storage units 735a and 735b storing the enzyme reagent and the solubilizing reagent among the reagents are opened by the opening mechanism 250. The rotation mechanism 221 transfers the enzyme reagent and the solubilizing reagent from the seventh liquid storage units 735a and 735b to the fourth chamber 715 by rotating the cartridge 700.

In step S43, the dispersoid containing the sample 80 and the reagent is stirred and warmed to a predetermined temperature. The rotation mechanism 221 stirs a liquid in the fourth chamber 715 by repeating the operation of changing the rotational speed of the cartridge 700. Then, the temperature of the dispersoid in the fourth chamber 715 is adjusted to, for example, 75°C, for a predetermined reaction time, by a temperature regulating unit 260.

In step S44, the reagent is transferred to the fourth chamber 715. First, the sealing bodies 30a of the two seventh liquid storage units 735c and 735d storing the nucleic acid extraction reagent and the magnetic particle reagent are opened by the opening mechanism 250. The rotation mechanism 221 transfers the nucleic acid extraction reagent and the magnetic particle reagent from the seventh liquid storage units 735c and 735d to the fourth chamber 715 by rotating the cartridge 700.

In step S45, stirring by the rotation mechanism 221 and warming by the temperature regulating unit 260 are performed as in step S43. The temperature during the warming is, for example, 75°C.

As a result, in the fourth chamber 715, the cells and the like in the sample 80 are lysed by action of the enzyme reagent and the solubilizing reagent. The solution containing the nucleic acid MD is mixed with the extraction reagent and the magnetic particle PM in the fourth chamber 715, thereby being captured on the surface of the magnetic particle PM as the carrier. As a result, the extraction processing of the nucleic acid MD is completed.

Thus, the rotation mechanism 221 rotates the cartridge 700 to mix the sample 80 introduced into the sample introduction unit 20 included in the cartridge 700, and the reagent for extracting nucleic acid stored in the seventh liquid storage units 735a to 735d included in the cartridge 700, in the cartridge 700, thereby performing processing for extracting the nucleic acid MD from the sample 80. Thereby, a step of extracting the nucleic acid MD can be performed in the cartridge 700 as well as the step of producing the emulsion 83 and the amplification step of the nucleic acid MD. Thus, it is possible to effectively suppress sample mix-up and contamination, as compared with the case where the step of extracting the nucleic acid MD is performed outside the cartridge 700. Then, the step of extracting the nucleic acid MD can be performed by rotation of the cartridge 700 using the rotation mechanism 221 in the same manner as the step of producing an emulsion 83, so that device configuration can be simplified.

The processing for extracting the nucleic acid MD is performed in the fourth chamber 715 different from the chamber 711 which performs the processing for producing the emulsion 83. The rotation mechanism 221 rotates the cartridge 700 to transfer the sample 80 containing the nucleic acid MD and the reagent for extracting nucleic acid to the fourth chamber 715 included in the cartridge 700, and the rotation mechanism 221 rotates the cartridge 700 to mix the sample 80 containing the nucleic acid MD and the reagent for extracting nucleic acid in the fourth chamber 715, thereby performing processing for extracting the nucleic acid MD. Thereby, the step of extracting the nucleic acid MD can be performed in the fourth chamber 715 different from the chamber 711 for producing the emulsion 83. Therefore, it is possible to suppress adhesion of unnecessary components other than the nucleic acid generated in the step of extracting the nucleic acid MD to the inside of the chamber 711 for producing the emulsion 83.

In step S46, the reagent 86 for washing the nucleic acid is transferred into the third chambers 712, 713, and 714, respectively. The liquid storage units 732, 733, and 734 are the fifth liquid storage units each storing the reagent 86 for washing the nucleic acid. The reagent 86 for washing the nucleic acid contains, for example, an alcohol such as ethanol. By making the nucleic acid solution into a hydrophobic environment, the alcohol removes unnecessary components adhered to the magnetic particle PM other than the nucleic acid MD while suppressing desorption of the nucleic acid MD from the carrier of the magnetic particle PM. The reagents stored in each of the fifth liquid storage units 732, 733, and 734 may have the same or different alcohol content ratio. For example, the alcohol content ratio in the stored reagent is in the relation of liquid storage units 732 > 733 > 734. Then, in order to improve the washing effect, the content ratio of washing components such as surfactant in the stored reagent is in the relation of liquid storage units 732 < 733 < 734.

First, the sealing bodies 30a of each of the fifth liquid storage units 732 to 734 are opened by the opening mechanism 250. The rotation mechanism 221 transfers the reagent 86 for washing the nucleic acid from the fifth liquid storage units 732 to 734 to the corresponding third chambers 712 to 714, respectively by rotating the cartridge 700.

In step S47, the magnetic particle PM is transferred from the fourth chamber 715 to the third chamber 714. The transfer mechanism 240 transfers the magnetic particle PM bound to the nucleic acid MD to the third chamber 12 by magnetic force, along with the rotation of the cartridge 700 by the rotation mechanism 221, thereby bringing the nucleic acid MD captured on the magnetic particle PM into contact with the reagent 86 for washing the nucleic acid. The magnetic particle PM moved into the third chamber 714 are stirred in the reagent 86 for washing the nucleic acid in the third chamber 714, by repeating an operation of approaching and separating a magnet 241 or by repeating the operation of changing the rotational speed of the cartridge 700. Thereby, the magnetic particle PM is washed.

In step S48, the magnetic particle PM is transferred from the third chamber 714 to the third chamber 713. Then, in the same manner as step S47, the magnetic particle PM are stirred in the reagent 86 for washing the nucleic acid in the third chamber 713. Thereby, the magnetic particle PM is washed.

In step S49, the magnetic particle PM is transferred from the third chamber 713 to the third chamber 712. Then, in the same manner as step S47, the magnetic particle PM are stirred in the reagent 86 for washing the nucleic acid in the third chamber 712. Thereby, the magnetic particle PM is washed.

In step S50, the reagent is transferred to the chamber 711. In the cartridge 700, an elution reagent is stored in advance in the sixth liquid storage unit 731a, the reagent 81 is stored in advance in the first liquid storage unit 731b, and a dispersion medium 82 is stored in advance in the second liquid storage unit 731c.

By making the nucleic acid solution into a hydrophilic environment, the elution reagent allows the nucleic acid MD adsorbed to the magnetic particle PM to be desorbed from the magnetic particle PM and eluted in the solution. The elution reagent contains a buffer such as, for example, TE (Tris/EDTA) buffer. The reagent 81 is a nucleic acid amplification reagent that amplifies the nucleic acid in the sample 80. In the example of Fig. 36, the reagent 81 contains a substance necessary for PCR such as DNA polymerase, and further contains a labeling substance LS. In the example of Fig. 36, the reagent 81 does not contain the magnetic particle PM. The dispersion medium 82 contains an oil that is immiscible with the sample 80 and the reagent 81.

In step S50, the sealing body 30a of the sixth liquid storage unit 731a storing the elution reagent among the reagents is opened by the opening mechanism 250. The rotation mechanism 221 transfers the elution reagent from the sixth liquid storage unit 731a to the chamber 711 by rotating the cartridge 700.

In step S51, the magnetic particle PM is transferred from the third chamber 712 to the chamber 711 by cooperation of the rotation mechanism 221 and the transfer mechanism 240. Then, the magnetic particle PM are stirred and warmed in the elution reagent in the chamber 711, by repeating the operation of changing the rotational speed of the cartridge 700 by the rotation mechanism 221. Thereby, the nucleic acid MD adsorbed to the carrier of the magnetic particle PM is desorbed from the magnetic particle PM and eluted in the elution reagent.

In step S52, the magnetic particle PM is transferred from the chamber 711 to the third chamber 712. In step S52, since the nucleic acid MD is eluted in the solution in the chamber 711, the magnetic particle PM not adsorbing the nucleic acid MD are transferred to the third chamber 712. The nucleic acid MD is left in the solution in the chamber 711.

By the operation of steps S50 to S52, the rotation mechanism 221 rotates the cartridge 700 to transfer the reagent for eluting the nucleic acid stored in the sixth liquid storage unit 731a included in the cartridge 700, and the rotation mechanism 221 bring the reagent into contact with the nucleic acid MD captured on the carrier, thereby storing the nucleic acid MD desorbed from the carrier in the chamber 711, and separating the carrier from the chamber 711. After the nucleic acid MD is washed, the nucleic acid MD can be desorbed from the carrier by the reagent for eluting the nucleic acid to separate the carrier. As a result, unnecessary components and the like nonspecifically adsorbed on the carrier can be separated from the nucleic acid, so that nucleic acid detection with higher accuracy becomes possible.

In step S53, the reagent is transferred to the chamber 711. The sealing body 30a of the first liquid storage unit 731b storing the reagent 81 for amplifying the nucleic acid is opened by the opening mechanism 250. The rotation mechanism 221 transfers the reagent 81 from the first liquid storage unit 731b to the chamber 711 by rotating the cartridge 700. Then, the rotation mechanism 221 repeats the operation of changing the rotational speed of the cartridge 700, whereby the nucleic acid amplification reagent and the nucleic acid solution in the chamber 711 are stirred.

In step S54, the reagent is transferred to the chamber 711. The sealing body 30a of the second liquid storage unit 731c storing the oil as the dispersion medium 82 is opened by the opening mechanism 250. The rotation mechanism 221 transfers the dispersion medium 82 from the second liquid storage unit 731 c to the chamber 711 by rotating the cartridge 700.

In step S55, an emulsion forming step is performed in the chamber 711. The chamber 711 stores the nucleic acid MD in the sample 80, the reagent 81 for amplifying the nucleic acid, the labeling substance LS, and the oil as the dispersion medium 82. Then, the rotation mechanism 221 forms the emulsion 83 in which the dispersoid containing the sample 80 and the reagent 81 is dispersed in the dispersion medium 82, in the chamber 711, by repeating the operation of changing the rotational speed when the cartridge 700 is rotated around the rotation axis 222. In the emulsion forming step, the operation of changing the rotational speed faster than the stirring operation in each chamber is repeated.

In step S56, an emulsion PCR step of amplifying the nucleic acid MD in the droplet 84 formed in the emulsion forming step is performed. After the emulsion 83 is formed, a thermal cycle processing of periodically changing the temperature of the chamber 711 to a plurality of temperature ranges is performed by the rotation mechanism 221 and the temperature regulating unit 260, and the nucleic acid MD contained in the dispersoid is amplified. Thus, the nucleic acid MD is efficiently amplified in the droplets 84 (see Fig. 15) contained in the emulsion 83. The nucleic acid amplification product in the droplet 84 reacts with the labeling substance LS contained in the nucleic acid amplification reagent to emit fluorescence.

In step S57, a detection step of the nucleic acid MD is performed. The rotation mechanism 221 rotates the cartridge 700 to move the chamber 711 to the position detected by the detection unit 270. The chamber 711 of the cartridge 700 is imaged by the detection unit 270 configured by the imaging unit. Image analysis is performed on the captured image by the analysis unit 233, and the nucleic acid MD is detected. That is, the analysis unit 233 outputs whether or not the nucleic acid MD is present, based on the captured image of the emulsion 83 in the chamber 711 captured by the detection unit 270. In the example of Figs. 36 and 37, the emulsion breaking step is not performed. Therefore, with the droplet 84 formed so as to contain one molecule or one nucleic acid MD as a unit of detection, whether or not the nucleic acid MD is present is counted from the captured image. The captured image may be transmitted from the nucleic acid detection device 200 to the server 650 (see Fig. 22), and the image analysis may be performed in the server 650. In that case, the server 650 outputs whether or not the nucleic acid MD is present, based on the captured image of the emulsion 83 in the chamber 711, via the nucleic acid detection device 200 or directly to the external terminal 600 (see Fig. 22).

### [Third Embodiment]

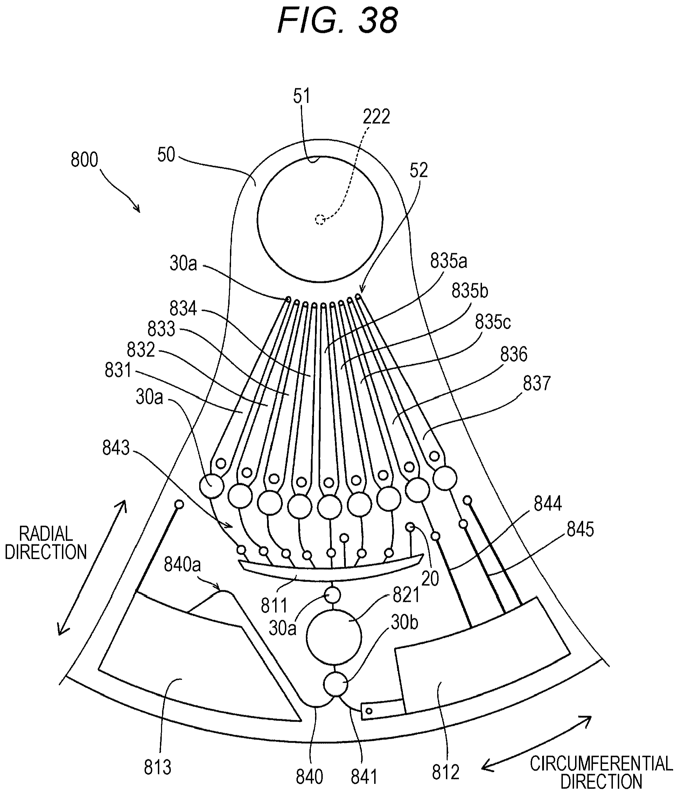

Fig. 38 shows a cartridge 800 according to the third embodiment. The cartridge 800 includes three chambers 811 to 813, nine liquid storage units, and a filter unit 821 for nucleic acid extraction. The cartridge 800 includes the filter unit 821 for capturing the nucleic acid MD, as a carrier for capturing the nucleic acid MD.

The chamber 811 extends circumferentially in an arc shape on a position radially inner side of the chambers 812 and 813. The chambers 812 and 813 are circumferentially arranged in the vicinity of the radially outer edge of the cartridge 800. Seven liquid storage units of liquid storage units 831, 832, 833, 834, 835a, 835b, and 835c are connected to the chamber 811, respectively. A sample introduction unit 20 is connected to the chamber 811. Two liquid storage units 836 and 837 are connected to the chamber 812. The cartridge 800 includes the chamber 812 for producing the emulsion 83 and the fourth chamber 811 for extracting the nucleic acid MD.

The chamber 811 and the chambers 812 and 813 are connected via the filter unit 821. Specifically, the chamber 811 communicates with the filter unit 821 via the sealing body 30a. The filter unit 821 communicates with each of the chambers 812 and 813 via a sealing body 30b on the radially outer side. The filter unit 821 communicates with the chamber 812 via a flow path 841 extending from the sealing body 30b. The filter unit 821 communicates with the chamber 813 via a flow path 840 extending from the sealing body 30b. The sealing body 30b is provided so as to communicate the filter unit 821 with the flow path 840 and seal the flow path 841. When the sealing body 30b is opened, the filter unit 821 communicates with the chamber 812 via the flow path 841. The chambers 811 to 813 each communicate with outside of the cartridge 800 on the radially inner side.

The seven liquid storage units 831 to 834 and 835a to 835c all communicate with outside of the cartridge 800 on the radially inner side. The seven liquid storage units 831 to 834 and 835a to 835c are connected to flow paths 843 communicating with the chamber 811 on a radially outer side. The sealing bodies 30a are each provided in the flow paths 843, a flow path 844 connecting the liquid storage unit 836 with the chamber 812, and a flow path 845 connecting the liquid storage unit 837 with the chamber 812, which can be opened by the opening mechanism 250.

In the third embodiment, a washing processing is performed by transferring the reagent 86 for washing the nucleic acid to the filter unit 821 in which the nucleic acid MD is captured. That is, the rotation mechanism 221 transfers the solution containing the nucleic acid MD to the filter unit 821 included in the cartridge 800 by rotating the cartridge 800. The rotation mechanism 221 rotates the cartridge 800 to transfer the reagent 86 for washing the nucleic acid from the fifth liquid storage units 835a to 835c to the filter unit 821, thereby bringing the nucleic acid MD into contact with the reagent 86 for washing the nucleic acid. With this configuration, with a simple configuration in which the cartridge 800 is only rotated by the rotation mechanism 221 without adding a configuration such as applying a magnetic force to the cartridge 800 from outside, the nucleic acid MD can be washed by bringing the nucleic acid MD captured in the filter unit 821 into contact with the reagent 86 for washing the nucleic acid.

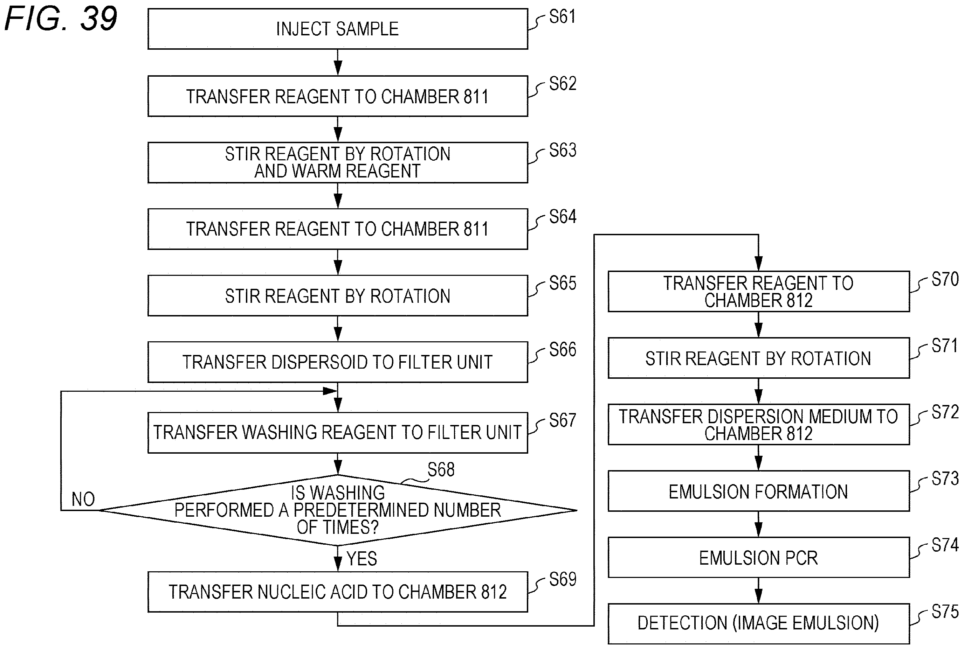

Fig. 39 shows a flow of processing using the cartridge 800. The processing using the cartridge 800 includes steps S61 to S75.

In step S61, the sample 80 containing an antigen as the nucleic acid MD is injected from the sample introduction unit 20. The sample 80 is the same as that of the second embodiment. Then, the rotation mechanism 221 transfers the sample 80 to the fourth chamber 811 by rotating the cartridge 800.

In step S62, the reagent is transferred to the fourth chamber 811. First, an enzyme reagent, a solubilizing reagent, a nucleic acid extraction reagent, and an elution reagent are stored in advance in the liquid storage units 831 to 834, respectively. The enzyme reagent, the solubilizing reagent, and the nucleic acid extraction reagent are stored in the seventh liquid storage units 831 to 833, respectively. The elution reagent is stored in the sixth liquid storage unit 834. The sealing bodies 30a of the two seventh liquid storage units 831 and 832 storing the enzyme reagent and the solubilizing reagent are opened by the opening mechanism 250. The rotation mechanism 221 transfers the enzyme reagent and the solubilizing reagent from the seventh liquid storage units 831 and 832 to the fourth chamber 811 by rotating the cartridge 800.

In step S63, the dispersoid containing the sample 80 and the reagent is stirred and warmed to a predetermined temperature. The rotation mechanism 221 stirs a liquid in the fourth chamber 811 by repeating the operation of changing the rotational speed of the cartridge 800. Then, the temperature of the dispersoid in the fourth chamber 811 is adjusted to, for example, 75°C, for a predetermined reaction time, by a temperature regulating unit 260.

In step S64, the reagent is transferred to the fourth chamber 811. First, the sealing body 30a of the seventh liquid storage unit 833 storing the nucleic acid extraction reagent is opened by the opening mechanism 250. The rotation mechanism 221 transfers the nucleic acid extraction reagent from the seventh liquid storage unit 833 to the fourth chamber 811 by rotating the cartridge 800.

In step S65, stirring is performed as in step S63.

In step S66, the dispersoid in the fourth chamber 811 is transferred to the filter unit 821. First, the sealing body 30a between the fourth chamber 811 and the filter unit 821 is opened by the opening mechanism 250. The dispersoid in the fourth chamber 811 is a nucleic acid solution containing the nucleic acid MD. The rotation mechanism 221 transfers the nucleic acid solution from the fourth chamber 811 to the filter unit 821 by rotating the cartridge 800.

The filter unit 821 includes a filter having a carrier that adsorbs the nucleic acid MD, and the filter is, for example, a silica membrane filter. The filter unit 821 adsorbs and captures the nucleic acid MD in the presence environment of the solubilizing reagent and the nucleic acid extraction reagent, and the filter unit 821 passes components and liquids other than nucleic acid. The nucleic acid MD in the nucleic acid solution is adsorbed by the filter unit 821 to be fixed.

In step S67, the washing processing is performed with the reagent 86 for washing the nucleic acid. Specifically, the reagent 86 for washing the nucleic acid is stored in advance in each of the fifth liquid storage units 835a to 835c. The reagent 86 for washing the nucleic acid is the same as that in the second embodiment. For example, the sealing body 30a of the first fifth liquid storage unit 835a among the fifth liquid storage units 835a to 835c is opened by the opening mechanism 250. The rotation mechanism 221 transfers the reagent 86 for washing the nucleic acid from the fifth liquid storage unit 835a to the filter unit 821 via the fourth chamber 811 by rotating the cartridge 800.

In the opening mechanism 250, the cartridge 800 is further rotated, with the reagent 86 for washing the nucleic acid transferred to the filter unit 821. Thus, the reagent 86 for washing the nucleic acid is transferred from the filter unit 821 to the chamber 813 via the sealing body 30b and the flow path 840. As a result, unnecessary components present in the filter unit 821 are discharged together with the reagent. The unnecessary component is a component other than nucleic acid MD, which is unnecessary for detection of nucleic acid MD. The reagent does not flow into the chamber 812 since the flow path 841 is sealed by the sealing body 30b.

In step S68, a control unit 230 determines whether the washing processing has been performed a predetermined number of times. In the example of Fig. 38, since the reagent 86 for washing the nucleic acid is stored in each of the three fifth liquid storage units 835a to 835c, the predetermined number of times is three. When the washing processing has not been performed a predetermined number of times, the process returns to step S67, the sealing body 30a of the next fifth liquid storage unit 835b is opened, and the reagent 86 for washing the nucleic acid is transferred to the chamber 813 via the filter unit 821 by the rotation of the cartridge 800. The same applies to the fifth liquid storage unit 835c. When the washing processing is performed a predetermined number of times, the process proceeds to step S69.

In step S69, the nucleic acid MD captured by the filter unit 821 is transferred to the chamber 812. Specifically, first, the sealing body 30a of the sixth liquid storage unit 834 storing the elution reagent is opened by the opening mechanism 250. The sealing body 30b for sealing the flow path 841 connected to the chamber 812 is opened by the opening mechanism 250. The rotation mechanism 221 transfers the elution reagent from the sixth liquid storage unit 834 to the filter unit 821 via the fourth chamber 811 by rotating the cartridge 800. As a result, the nucleic acid MD adsorbed to the filter unit 821 is desorbed from the filter unit 821 and dissolved in the elution reagent. The rotation mechanism 221 transfers the nucleic acid solution in the filter unit 821 to the chamber 812 via the sealing body 30a and the flow path 841, by further continuing the rotation of the cartridge 800.

The flow path 840 connected to the chamber 813 extends to a flow path portion 840a located radially inner side of the chamber 812 and then connects to the chamber 813. Therefore, when the liquid is transferred by centrifugal force, with the sealing body 30b opened, the liquid does not flow into the chamber 813 beyond the flow path portion 840a unless the chamber 812 is filled with the liquid. Thus, substantially all amount of the nucleic acid solution flowing out of the filter unit 821 is transferred to the chamber 812 without flowing into the chamber 813.

In step S70, the reagent 81 for amplifying the nucleic acid is transferred to the chamber 812. First, the reagent 81 for amplifying the nucleic acid is stored in advance in the first liquid storage unit 836. The dispersion medium 82 is stored in advance in the second liquid storage unit 837. The reagent 81 and the dispersion medium 82 are the same as those in the second embodiment. Among these, the sealing body 30a of the first liquid storage unit 836 storing the reagent 81 is opened by the opening mechanism 250. The rotation mechanism 221 transfers reagent 81 from the first liquid storage unit 836 to the chamber 812 via the flow path 844 by rotating the cartridge 800.

In step S71, the nucleic acid solution in the chamber 812 and the reagent 81 are stirred, by repeating the operation of changing the rotational speed of the cartridge 800 by the rotation mechanism 221.

In step S72, the reagent is transferred to the chamber 812. The sealing body 30a of the second liquid storage unit 837 storing the oil as the dispersion medium 82 is opened by the opening mechanism 250. The rotation mechanism 221 transfers the dispersion medium 82 from the second liquid storage unit 837 to the chamber 812 via the flow path 845 by rotating the cartridge 800.

In step S73, an emulsion forming step is performed in the chamber 812. The chamber 812 stores the nucleic acid MD in the sample 80, the reagent 81 for amplifying the nucleic acid, the labeling substance LS, and the oil as the dispersion medium 82. Then, the rotation mechanism 221 forms the emulsion 83 in which the dispersoid containing the sample 80 and the reagent 81 is dispersed in the dispersion medium 82, in the chamber 812, by repeating the operation of changing the rotational speed when the cartridge 800 is rotated around the rotation axis 222. In the examples of Figs. 38 and 39, the nucleic acid MD is not carried by the magnetic particle PM. Therefore, the magnetic particle reagent containing the magnetic particle PM is not stored in the cartridge 800. In the example of Fig. 38, since the filter unit 821 is provided instead of the magnetic particle PM as described above, the transfer of the nucleic acid MD to each chamber is performed only by the centrifugal force accompanying the rotation of the cartridge 800. Thus, when the magnetic particle PM is not used, the transfer mechanism 240 need not be provided.

In step S74, an emulsion PCR step of amplifying the nucleic acid (DNA) in the droplet 84 formed in the emulsion forming step is performed. After the emulsion 83 is formed, a thermal cycle processing of periodically changing the temperature of the cartridge 800 to a plurality of temperature ranges is performed by the temperature regulating unit 260, and the nucleic acid MD contained in the dispersoid is amplified. Thus, the nucleic acid MD is efficiently amplified in the droplets 84 (see Fig. 15) contained in the emulsion 83. The nucleic acid amplification product in the droplet 84 reacts with the labeling substance LS contained in the nucleic acid amplification reagent to emit fluorescence.

In the example of Fig. 38, the emulsion breaking step is not performed, as in the second embodiment. In step S75, a detection step of the nucleic acid MD is performed. The chamber 812 of the cartridge 800 is imaged by the detection unit 270 configured by the imaging unit. Image analysis is performed on the captured image by the analysis unit 233, and the nucleic acid MD is detected. That is, the analysis unit 233 outputs whether or not the nucleic acid MD is present, based on the captured image of the emulsion 83 in the chamber 812 captured by the detection unit 270. Image analysis may be performed in the server 650.

### [Modification Example of Nucleic Acid Detection Device]

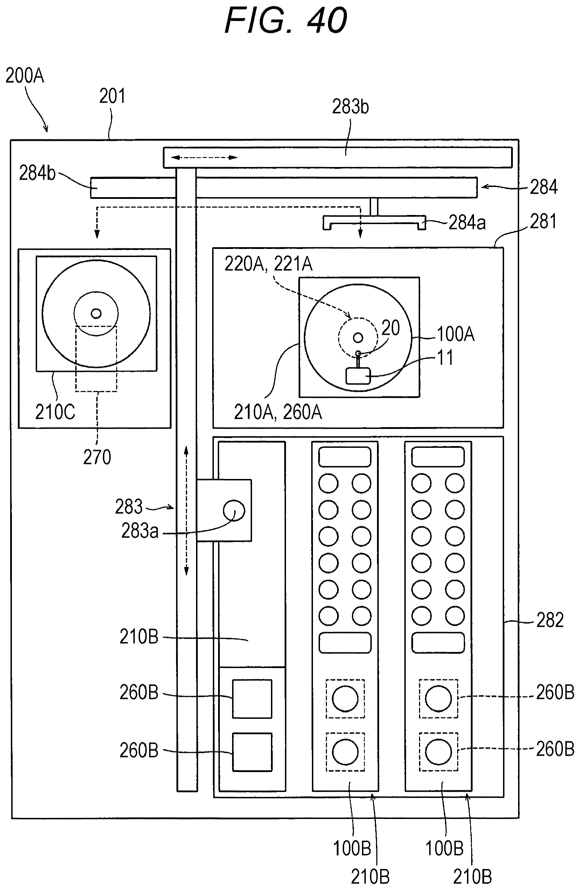

Fig. 40 shows a modification example of the nucleic acid detection device according to the first embodiment. A nucleic acid detection device 200A according to the modification example is configured to perform processing using a plurality of cartridges.

In the example of Fig. 40, the nucleic acid detection device 200A includes a sample processing unit 281 that processes a sample using a first cartridge 100A that performs a step of producing the emulsion 83 and a nucleic acid amplification step, and a sample extraction unit 282 that processes a sample using a second cartridge 100B that performs a step of extracting nucleic acid.

The nucleic acid detection device 200A includes a sample dispensing unit 283 for transferring a sample between the cartridge 100B and the cartridge 100A, a detection unit 270, and a cartridge transfer mechanism 284 for transferring the cartridge 100A.

The sample extraction unit 282 includes three installation units 210B in which the rectangular cartridge 100B is installed, and a temperature regulating unit 260B provided in each installation unit 210B. The sample 80 containing the nucleic acid MD is introduced into the cartridge 100B installed in the sample extraction unit 282. The introduction of the sample 80 may be performed by the user or may be performed by the sample dispensing unit 283.

The nucleic acid detection device 200A performs the step of extracting the nucleic acid MD from at least the sample 80, using the cartridge 100B arranged in the sample extraction unit 282. In the cartridge 100B, for example, an enzyme reagent, a solubilizing reagent, a nucleic acid extraction reagent, and an elution reagent can be enclosed in advance, as reagents for extracting nucleic acid.

The nucleic acid detection device 200A may perform a step of washing the nucleic acid extracted with the reagent 86 for washing the nucleic acid, using the cartridge 100B arranged in the sample extraction unit 282. In that case, the reagent 86 for washing the nucleic acid can be enclosed in advance in the cartridge 100B.

Transfer of the sample 80 and the reagent in the cartridge 100B is performed by transferring a liquid between wells formed in the cartridge 100B by the sample dispensing unit 283. The sample dispensing unit 283 may apply a pressure to the opening formed in the cartridge 100B to transfer the liquid by pressure in the cartridge 100B.

The sample dispensing unit 283 includes, for example, a suction unit 283a that sucks and discharges liquid, and a suction unit moving mechanism 283b. The suction unit 283a is configured to be movable to an installation position of the cartridge 100B in the sample extraction unit 282 and an installation position of the cartridge 100A in the sample processing unit 281 by the suction unit moving mechanism 283b. The suction unit 283a is configured so that, for example, a disposal pipette tip can be detachably attached. The suction unit 283a exchanges the pipette tip when suctioning and dispensing the liquid containing the nucleic acid MD. The sample dispensing unit 283 sucks the liquid containing the nucleic acid MD extracted using the cartridge 100B from the cartridge 100B by the suction unit 283a The sample dispensing unit 283 injects the liquid into the sample introduction unit 20 of the cartridge 100A.

The sample processing unit 281 is provided with an installation unit 210A on which the disk-shaped cartridge 100A is installed, and a temperature regulating unit 260A. In the example of Fig. 40, the sample processing unit 281 includes a rotation mechanism 221 A.

The nucleic acid detection device 200A performs at least the step of producing the emulsion 83 and the nucleic acid amplification step, using the cartridge 100A installed in the sample processing unit 281. In the cartridge 100A, for example, a reagent 81 for amplifying the nucleic acid and a labeling substance LS and an oil as a dispersion medium 82 are enclosed in advance.

The nucleic acid detection device 200A may perform a step of washing the nucleic acid extracted in the cartridge 100B with the reagent 86 for washing the nucleic acid, using the cartridge 100A arranged in the sample processing unit 281. In that case, the reagent 86 for washing the nucleic acid can be enclosed in advance in the cartridge 100A.

Specifically, the configuration of the first embodiment shown in Fig. 40 can be adopted for the cartridge 100A. In that case, the nucleic acid detection device 200A includes the transfer mechanism 240 that allows the cartridge 100A to exert a magnetic force. The cartridge 100A can adopt a configuration in which a structure related to the extraction of the nucleic acid MD is omitted, from the configuration of the second embodiment shown in Fig. 36 and the configuration of the third embodiment shown in Fig. 38.

In the sample processing unit 281, the emulsion 83 is formed in the chamber 11 of the cartridge 100A by the rotation mechanism 221A, and amplification of the nucleic acid MD (i.e., emulsion PCR) in the droplet 84 is performed in the chamber 11 by the temperature regulating unit 260A. In the sample processing unit 281, the amplification product of the nucleic acid MD reacts with the labeling substance LS.

The cartridge transfer mechanism 284 includes, for example, a gripping unit 284a that grips the cartridge 100A, and a gripping unit moving mechanism 284b. The gripping unit 284a holds the cartridge 100A by means such as clamping, adsorption, and engagement. The gripping unit 284a is configured to be movable to an installation position of the cartridge 100A in the sample processing unit 281 and an installation unit 210C of the cartridge 100A in the detection unit 270 by the gripping unit moving mechanism 284b. After the processing in the sample processing unit 281, the cartridge transfer mechanism 284 transports the cartridge 100A storing the nucleic acid MD bound to the labeling substance LS from the sample processing unit 281 to the installation unit 210C of the detection unit 270.

The detection unit 270 detects a signal based on the nucleic acid MD amplified in the cartridge 100A. The detection unit 270 includes, for example, an imaging unit. The detection unit 270 may be arranged in the sample processing unit 281 as in the configuration shown in Fig. 20. In that case, the detection unit 270 images the chamber 11 from below or above the cartridge 100A arranged in the installation unit 210A of the sample processing unit 281. In this case, the cartridge transfer mechanism 284 can be omitted.

With such a configuration, the nucleic acid detection device 200A according to the modification example performs nucleic acid detection by transferring a sample between the cartridges using a plurality of the cartridges.

It should be considered that the embodiment disclosed herein is an example in all respects and is not restrictive. The scope of the present invention is indicated not by the description of the above embodiment but by the scope of claims, and further includes meanings equivalent to the scope of claims and all changes within the scope.

## Claims

1. A nucleic acid detection device comprising:
an installation unit on which a cartridge is installed, the cartridge including a chamber configured to store nucleic acid, a reagent for amplifying the nucleic acid and a dispersion medium for dispersing the nucleic acid and the reagent;
a sample processing unit configured to produce, within the chamber, an emulsion in which droplets containing the nucleic acid and the reagent are dispersed in the dispersion medium;
a temperature regulating unit configured to regulate a temperature of the cartridge to amplify the nucleic acid contained in the droplet; and
a detection unit configured to detect a signal based on the nucleic acid amplified by temperature regulation by the temperature regulating unit.

2. The nucleic acid detection device according to claim 1, wherein the sample processing unit forms the droplets in the chamber by stirring the nucleic acid, the reagent and the dispersion medium in the chamber.

3. The nucleic acid detection device according to claim 1 or 2, wherein
the sample processing unit includes a rotation mechanism that rotates the cartridge installed on the installation unit, and
the rotation mechanism rotationally moves the cartridge to a detection position where the detection unit detects the signal based on the nucleic acid.

4. The nucleic acid detection device according to any one of claims 1 to 3, wherein
the sample processing unit includes a rotation mechanism that rotates the cartridge installed on the installation unit, and
the rotation mechanism transfers, within the cartridge, each of a solution containing the nucleic acid and the reagent to the chamber by rotating the cartridge.

5. The nucleic acid detection device according to any one of claims 1 to 4, wherein the sample processing unit produces, within the chamber, the emulsion including the droplets each containing one molecule or one nucleic acid.

6. The nucleic acid detection device according to any one of claims 1 to 5, wherein the sample processing unit extracts the nucleic acid from a sample and produces the emulsion from the extracted nucleic acid within the cartridge.

7. The nucleic acid detection device according to any one of claims 1 to 6, wherein the temperature regulating unit regulates a temperature of the chamber by heating the cartridge from outside of the cartridge.

8. The nucleic acid detection device according to any one of claims 1 to 7, wherein
the reagent is enclosed in the cartridge in advance, and
the nucleic acid detection device further comprises an opening mechanism that opens seal of the reagent in the cartridge.

9. The nucleic acid detection device according to any one of claims 1 to 8, wherein the detection unit includes an imaging unit configured to capture, from outside of the cartridge, an image in the chamber storing the nucleic acid.

10. The nucleic acid detection device according to claim 9, further comprising an analysis unit configured to output whether or not the nucleic acid to be detected is present, based on the captured image.

11. The nucleic acid detection device according to claim 4, wherein the rotation mechanism transfers the reagent stored in a first liquid storage unit included in the cartridge to the chamber by rotating the cartridge, and mixes the nucleic acid and the reagent in the chamber by rotating the cartridge.

12. The nucleic acid detection device according to claim 11, wherein the rotation mechanism transfers the dispersion medium stored in a second liquid storage unit included in the cartridge to the chamber by rotating the cartridge, and repeats an operation of changing a rotational speed of the cartridge to mix the nucleic acid, the reagent and the dispersion medium in the chamber.

13. The nucleic acid detection device according to claim 12, wherein the rotation mechanism repeats an operation of rotationally driving the cartridge in a predetermined direction and then stopping or driving the cartridge in a reverse direction to produce the emulsion.

14. The nucleic acid detection device according to claim 12 or 13, wherein the rotation mechanism rotates the cartridge to transfer a reagent for demulsifying the emulsion from a third liquid storage unit included in the cartridge to the chamber.

15. A method for detecting nucleic acid using a cartridge, the method comprising:
storing nucleic acid, a reagent for amplifying the nucleic acid and a dispersion medium for dispersing the nucleic acid and the reagent within a chamber of the cartridge;
producing, within the chamber, an emulsion in which droplets containing the nucleic acid and the reagent are dispersed in the dispersion medium;
regulating a temperature of the cartridge for amplifying the nucleic acid contained in the droplet; and
detecting a signal based on the nucleic acid amplified by temperature regulation.
